# EUROPEAN PATENT APPLICATION

(11) **EP 4 101 851 A1**
(43) Date of publication of application: **14.12.2022**
(21) Application number: 21750440.6
(22) Date of filing: 04.02.2021
(51) Int. Cl.: C07D 403/10, C07D 403/14, H01L 51/50

(54) **COMPOUND, MATERIAL FOR ORGANIC ELECTROLUMINESCENT ELEMENTS, ORGANIC ELECTROLUMINESCENT ELEMENT, AND ELECTRONIC DEVICE**

(30) Priority: 05.02.2020 JP 2020018225
(71) Applicant: Idemitsu Kosan Co., Ltd, Tokyo 100-8321 (JP)
(72) Inventor: YOSHIDA, Kei, Sodegaura-shi, Chiba 299-0293 (JP); SAITO, Masatoshi, Sodegaura-shi, Chiba 299-0293 (JP); NAKAMURA, Masato, Sodegaura-shi, Chiba 299-0293 (JP); MASUDA, Tetsuya, Sodegaura-shi, Chiba 299-0293 (JP)
(74) Representative: Vossius & Partner Patentanwälte Rechtsanwälte mbB
(86) International application number: PCT/JP2021/004019
(87) International publication number: WO 2021/157635

(57) **Abstract**

The present invention provides a compound that further improves the performance of an organic EL device, an organic electroluminescent device which has further improved device performance, and an electronic device which includes the organic electroluminescent device. A compound represented by the following formula (1) and having a molecular weight of 650 or more: 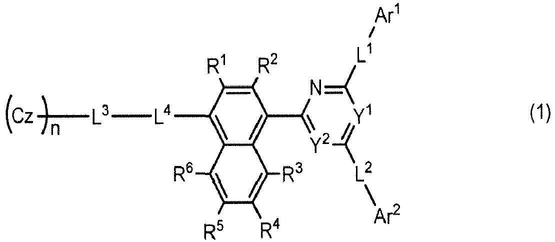 wherein Cz, n, Y¹, Y², Ar¹, Ar², L¹, L², L³, L⁴, and R¹ to R⁶ are defined in the description; an organic electroluminescent device which contains the compound; and an electronic device which includes the organic electroluminescent device.

## Description

### Technical Field

The present invention relates to a compound, a material for organic electroluminescent devices, an organic electroluminescent device, and an electronic device including the organic electroluminescent device.

### Background Art

In general, an organic electroluminescent device (which may be hereinafter referred to as an "organic EL device") is constituted by an anode, a cathode, and an organic layer intervening between the anode and the cathode. In application of a voltage between both the electrodes, electrons from the cathode side and holes from the anode side are injected into a light emitting region, and the injected electrons and holes are recombined in the light emitting region to generate an excited state, which then returns to a ground state to emit light. Accordingly, development of a material that efficiently transports electrons or holes into the light emitting region, and promotes recombination of the electrons and holes is important for obtaining a high-performance organic EL device.

PTLs 1 to 9 describe compounds used as materials for organic electroluminescent devices.

### Citation List

### Patent Literature

PTL 1: WO 2005/112519 A1
PTL 2: KR 2014-0006708 A
PTL 3: WO 2017/200210 A1
PTL 4: WO 2019/139419 A1
PTL 5: US 2014/361268 A1
PTL 6: WO 2018/105888 A1
PTL 7: CN 107880031 A
PTL 8: WO 2018/056645 A1
PTL 9: WO 2012/108881 A1

### Summary of Invention

### Technical Problem

Conventionally, various compounds for organic EL devices have been reported. However, a compound that further enhances the performance of an organic EL device has been still demanded.

The present invention has been made for solving the aforementioned problem, and an object thereof is to provide a compound that further improves the performance of an organic EL device, an organic EL device which has further improved device performance, and an electronic device which includes the organic EL device.

### Solution to Problem

As a result of the continued investigations by the present inventors on the performance of organic EL devices containing the compounds described in PTLs 1 to 9, it has been found that a naphthylpyrmidine compound having a molecular weight of 650 or more and having a structure in which a specific carbazole skeleton is bonded to a pyrimidine skeleton via a single bond or an arylene group and further via a naphthylene group, can provide an organic EL device having further improved device performance.

In one embodiment, the present invention provides a compound represented by the following formula (1) and having a molecular weight of 650 or more, 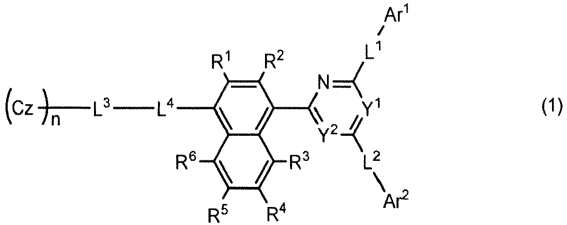 wherein
one of Y¹ and Y² is a nitrogen atom and the other one is CR,
R is selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a fluorine atom, and a cyano group;
Ar¹ and Ar² each are independently selected from a substituted or unsubstituted aryl group having 6 to 50 ring carbon atoms and a substituted or unsubstituted heterocyclic group having 5 to 50 ring atoms;
L¹ and L² each are independently a single bond or a substituted or unsubstituted arylene group having 6 to 50 ring carbon atoms;
R¹ to R⁶ each are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a fluorine atom, and a cyano group, and adjacent two selected from R¹ to R⁶ are not bonded to each other to form a ring;
Cz is represented by the following formula (1-a) or formula (1-b): 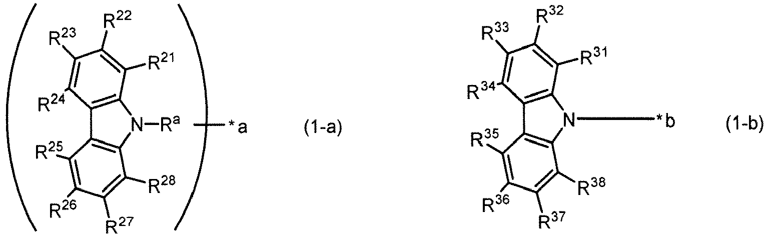 wherein
   R²¹ to R²⁸ and R³¹ to R³⁸ each are independently a hydrogen atom or a substituent, and the substituent is selected from a substituted or unsubstituted aryl group having 6 to 50 ring carbon atoms, a substituted or unsubstituted heterocyclic group having 5 to 50 ring atoms but not having a 5-membered nitrogen-containing ring and a 7-membered nitrogen-containing ring, a substituted or unsubstituted alkyl group having 1 to 50 carbon atoms, a substituted or unsubstituted cycloalkyl group having 3 to 50 ring carbon atoms, a fluorine atom, and a cyano group;
   provided that adjacent two selected from R²¹ to R²⁸, adjacent two selected from R³¹ to R³⁸, R²⁴ and R²⁵, and R³⁴ and R³⁵ are not bonded to each other to form a ring;
   Ra is selected from a substituted or unsubstituted aryl group having 6 to 50 ring carbon atoms and a substituted or unsubstituted alkyl group having 1 to 50 carbon atoms;
   one selected from R²¹ to R²⁸ is a single bond bonded to L³ via *a; *b represents a position bonded to L³;
   n is an integer of 1 to 3, and when n is an integer of 2 or 3, Cz's, which are 2 or 3, are the same or different from each other;
   L³ and L⁴ each are independently a single bond or a substituted or unsubstituted arylene group having 6 to 50 ring carbon atoms; provided that when n is an integer of 2 or 3, L⁴ is a substituted or unsubstituted arylene group having 6 to 50 ring carbon atoms.

In another embodiment, the present invention provides a material for an organic EL device that contains the compound represented by the formula (1) and having a molecular weight of 650 or more.

In still another embodiment, the present invention provides an organic electroluminescent device including an anode, a cathode, and organic layers intervening between the anode and the cathode, the organic layers including a light emitting layer, at least one layer of the organic layers containing the compound represented by the formula (1), the compound having a molecular weight of 650 or more.

In further another embodiment, the present invention provides an electronic device including the organic electroluminescent device.

### Advantageous Effects of Invention

An organic EL device containing the compound represented by the formula (1) with a molecular weight of 650 or more shows improved device performance.

### Brief Description of Drawings

Fig. 1 is a schematic view showing an example of a layer structure of an organic EL device according to an embodiment of the present invention.
Fig. 2 is a schematic view showing another example of the layer structure of the organic EL device according to an embodiment of the present invention.

### Description of Embodiments

### [Definitions]

In the description herein, the hydrogen atom encompasses isotopes thereof having different numbers of neutrons, i.e., a light hydrogen atom (protium), a heavy hydrogen atom (deuterium), and tritium.

In the description herein, the bonding site where the symbol, such as "R", or "D" representing a deuterium atom is not shown is assumed to have a hydrogen atom, i.e., a protium atom, a deuterium atom, or a tritium atom, bonded thereto.

In the description herein, the number of ring carbon atoms shows the number of carbon atoms among the atoms constituting the ring itself of a compound having a structure including atoms bonded to form a ring (such as a monocyclic compound, a condensed ring compound, a bridged compound, a carbocyclic compound, and a heterocyclic compound). In the case where the ring is substituted by a substituent, the carbon atom contained in the substituent is not included in the number of ring carbon atoms. The same definition is applied to the "number of ring carbon atoms" described hereinafter unless otherwise indicated. For example, a benzene ring has 6 ring carbon atoms, a naphthalene ring has 10 ring carbon atoms, a pyridine ring has 5 ring carbon atoms, and a furan ring has 4 ring carbon atoms. For example, 9,9-diphenylfluorenyl group has 13 ring carbon atoms, and 9,9'-spirobifluorenyl group has 25 ring carbon atoms.

In the case where a benzene ring has, for example, an alkyl group substituted thereon as a substituent, the number of carbon atoms of the alkyl group is not included in the number of ring carbon atoms of the benzene ring. Accordingly, a benzene ring having an alkyl group substituted thereon has 6 ring carbon atoms. In the case where a naphthalene ring has, for example, an alkyl group substituted thereon as a substituent, the number of carbon atoms of the alkyl group is not included in the number of ring carbon atoms of the naphthalene ring. Accordingly, a naphthalene ring having an alkyl group substituted thereon has 10 ring carbon atoms.

In the description herein, the number of ring atoms shows the number of atoms constituting the ring itself of a compound having a structure including atoms bonded to form a ring (such as a monocyclic ring, a condensed ring, and a set of rings) (such as a monocyclic compound, a condensed ring compound, a bridged compound, a carbocyclic compound, and a heterocyclic compound). The atom that does not constitute the ring (such as a hydrogen atom terminating the bond of the atom constituting the ring) and, in the case where the ring is substituted by a substituent, the atom contained in the substituent are not included in the number of ring atoms. The same definition is applied to the "number of ring atoms" described hereinafter unless otherwise indicated. For example, a pyridine ring has 6 ring atoms, a quinazoline ring has 10 ring atoms, and a furan ring has 5 ring atoms. For example, the number of hydrogen atoms bonded to a pyridine ring or atoms constituting a substituent is not included in the number of ring atoms of the pyridine ring. Accordingly, a pyridine ring having a hydrogen atom or a substituent bonded thereto has 6 ring atoms. For example, the number of hydrogen atoms bonded to carbon atoms of a quinazoline ring or atoms constituting a substituent is not included in the number of ring atoms of the quinazoline ring. Accordingly, a quinazoline ring having a hydrogen atom or a substituent bonded thereto has 10 ring atoms.

In the description herein, the expression "having XX to YY carbon atoms" in the expression "substituted or unsubstituted ZZ group having XX to YY carbon atoms" means the number of carbon atoms of the unsubstituted ZZ group, and, in the case where the ZZ group is substituted, the number of carbon atoms of the substituent is not included. Herein, "YY" is larger than "XX", "XX" represents an integer of 1 or more, and "YY" represents an integer of 2 or more.

In the description herein, the expression "having XX to YY atoms" in the expression "substituted or unsubstituted ZZ group having XX to YY atoms" means the number of atoms of the unsubstituted ZZ group, and, in the case where the ZZ group is substituted, the number of atoms of the substituent is not included. Herein, "YY" is larger than "XX", "XX" represents an integer of 1 or more, and "YY" represents an integer of 2 or more.

In the description herein, an unsubstituted ZZ group means the case where the "substituted or unsubstituted ZZ group" is an "unsubstituted ZZ group", and a substituted ZZ group means the case where the "substituted or unsubstituted ZZ group" is a "substituted ZZ group".

In the description herein, the expression "unsubstituted" in the expression "substituted or unsubstituted ZZ group" means that hydrogen atoms in the ZZ group are not substituted by a substituent. The hydrogen atoms in the "unsubstituted ZZ group" each are a protium atom, a deuterium atom, or a tritium atom.

In the description herein, the expression "substituted" in the expression "substituted or unsubstituted ZZ group" means that one or more hydrogen atom in the ZZ group is substituted by a substituent. The expression "substituted" in the expression "BB group substituted by an AA group" similarly means that one or more hydrogen atom in the BB group is substituted by the AA group.

### Substituents in Description

The substituents described in the description herein will be explained.

In the description herein, the number of ring carbon atoms of the "unsubstituted aryl group" is 6 to 50, preferably 6 to 30, and more preferably 6 to 18, unless otherwise indicated in the description.

In the description herein, the number of ring atoms of the "unsubstituted heterocyclic group" is 5 to 50, preferably 5 to 30, and more preferably 5 to 18, unless otherwise indicated in the description.

In the description herein, the number of carbon atoms of the "unsubstituted alkyl group" is 1 to 50, preferably 1 to 20, and more preferably 1 to 6, unless otherwise indicated in the description.

In the description herein, the number of carbon atoms of the "unsubstituted alkenyl group" is 2 to 50, preferably 2 to 20, and more preferably 2 to 6, unless otherwise indicated in the description.

In the description herein, the number of carbon atoms of the "unsubstituted alkynyl group" is 2 to 50, preferably 2 to 20, and more preferably 2 to 6, unless otherwise indicated in the description.

In the description herein, the number of ring carbon atoms of the "unsubstituted cycloalkyl group" is 3 to 50, preferably 3 to 20, and more preferably 3 to 6, unless otherwise indicated in the description.

In the description herein, the number of ring carbon atoms of the "unsubstituted arylene group" is 6 to 50, preferably 6 to 30, and more preferably 6 to 18, unless otherwise indicated in the description.

In the description herein, the number of ring atoms of the "unsubstituted divalent heterocyclic group" is 5 to 50, preferably 5 to 30, and more preferably 5 to 18, unless otherwise indicated in the description.

In the description herein, the number of carbon atoms of the "unsubstituted alkylene group" is 1 to 50, preferably 1 to 20, and more preferably 1 to 6, unless otherwise indicated in the description.

### Substituted or Unsubstituted Aryl Group

In the description herein, specific examples (set of specific examples G1) of the "substituted or unsubstituted aryl group" include the unsubstituted aryl groups (set of specific examples G1A) and the substituted aryl groups (set of specific examples G1B) shown below. (Herein, the unsubstituted aryl group means the case where the "substituted or unsubstituted aryl group" is an "unsubstituted aryl group", and the substituted aryl group means the case where the "substituted or unsubstituted aryl group" is a "substituted aryl group".) In the description herein, the simple expression "aryl group" encompasses both the "unsubstituted aryl group" and the "substituted aryl group".

The "substituted aryl group" means a group formed by substituting one or more hydrogen atom of the "unsubstituted aryl group" by a substituent. Examples of the "substituted aryl group" include groups formed by one or more hydrogen atom of each of the "unsubstituted aryl groups" in the set of specific examples G1A by a substituent, and the examples of the substituted aryl groups in the set of specific examples G1B. The examples of the "unsubstituted aryl group" and the examples of the "substituted aryl group" enumerated herein are mere examples, and the "substituted aryl group" in the description herein encompasses groups formed by substituting a hydrogen atom bonded to the carbon atom of the aryl group itself of each of the "substituted aryl groups" in the set of specific examples G1B by a substituent, and groups formed by substituting a hydrogen atom of the substituent of each of the "substituted aryl groups" in the set of specific examples G1B by a substituent.

Unsubstituted Aryl Group (Set of Specific Examples G1A):
a phenyl group,
a p-biphenyl group,
a m-biphenyl group,
an o-biphenyl group,
a p-terphenyl-4-yl group,
a p-terphenyl-3-yl group,
a p-terphenyl-2-yl group,
a m-terphenyl-4-yl group,
a m-terphenyl-3-yl group,
a m-terphenyl-2-yl group,
an o-terphenyl-4-yl group,
an o-terphenyl-3-yl group,
an o-terphenyl-2-yl group,
a 1-naphthyl group,
a 2-naphthyl group,
an anthryl group,
a benzanthryl group,
a phenanthryl group,
a benzophenanthryl group,
a phenarenyl group,
a pyrenyl group,
a chrysenyl group,
a benzochrysenyl group,
a triphenylenyl group,
a benzotriphenylenyl group,
a tetracenyl group,
a pentacenyl group,
a fluorenyl group,
a 9,9'-spirobifluorenyl group,
a benzofluorenyl group,
a dibenzofluorenyl group,
a fluoranthenyl group,
a benzofluoranthenyl group,
a perylenyl group, and
monovalent aryl groups derived by removing one hydrogen atom from each of the ring structures represented by the following general formulae (TEMP-1) to (TEMP-15):

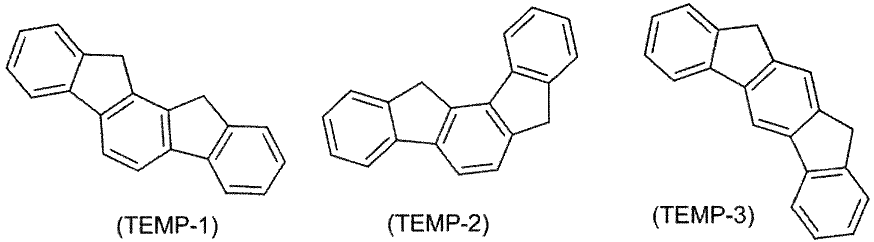

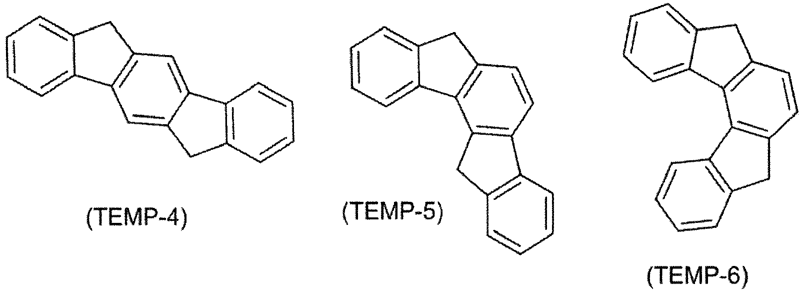

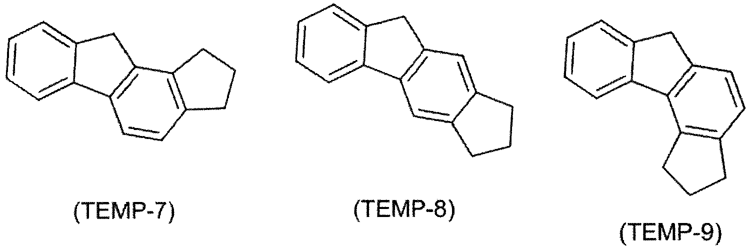

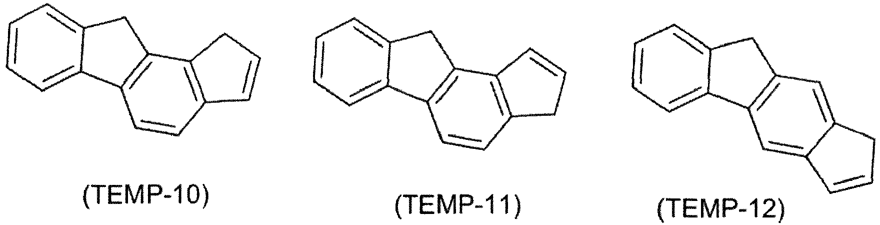

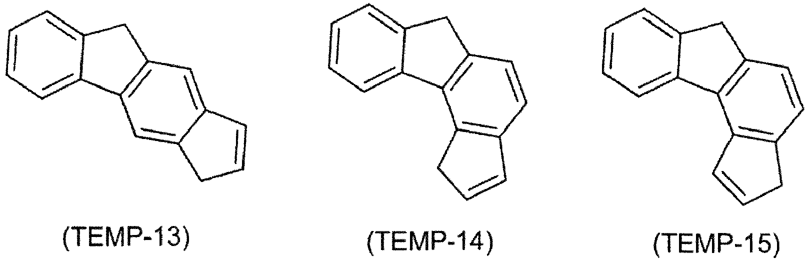

Substituted Aryl Group (Set of Specific Examples G1B):
an o-tolyl group,
a m-tolyl group,
a p-tolyl group,
a p-xylyl group,
a m-xylyl group,
an o-xylyl group,
a p-isopropylphenyl group,
a m-isopropylphenyl group,
an o-isopropylphenyl group,
a p-t-butylphenyl group,
a m-t-butylphenyl group,
a o-t-butylphenyl group,
a 3,4,5-trimethylphenyl group,
a 9,9-dimethylfluorenyl group,
a 9,9-diphenylfluorenyl group,
a 9,9-bis(4-methylphenyl)fluorenyl group,
a 9,9-bis(4-isopropylphenyl)fluorenyl group,
a 9,9-bis(4-t-butylphenyl)fluorenyl group,
a cyanophenyl group,
a triphenylsilylphenyl group,
a trimethylsilylphenyl group,
a phenylnaphthyl group,
a naphthylphenyl group, and
groups formed by substituting one or more hydrogen atom of each of monovalent aryl groups derived from the ring structures represented by the general formulae (TEMP-1) to (TEMP-15) by a substituent.

### Substituted or Unsubstituted Heterocyclic Group

In the description herein, the "heterocyclic group" means a cyclic group containing at least one hetero atom in the ring atoms. Specific examples of the hetero atom include a nitrogen atom, an oxygen atom, a sulfur atom, a silicon atom, a phosphorus atom, and a boron atom.

In the description herein, the "heterocyclic group" is a monocyclic group or a condensed ring group.

In the description herein, the "heterocyclic group" is an aromatic heterocyclic group or a non-aromatic heterocyclic group.

In the description herein, specific examples (set of specific examples G2) of the "substituted or unsubstituted heterocyclic group" include the unsubstituted heterocyclic groups (set of specific examples G2A) and the substituted heterocyclic groups (set of specific examples G2B) shown below. (Herein, the unsubstituted heterocyclic group means the case where the "substituted or unsubstituted heterocyclic group" is an "unsubstituted heterocyclic group", and the substituted heterocyclic group means the case where the "substituted or unsubstituted heterocyclic group" is a "substituted heterocyclic group".) In the description herein, the simple expression "heterocyclic group" encompasses both the "unsubstituted heterocyclic group" and the "substituted heterocyclic group".

The "substituted heterocyclic group" means a group formed by substituting one or more hydrogen atom of the "unsubstituted heterocyclic group" by a substituent. Specific examples of the "substituted heterocyclic group" include groups formed by substituting a hydrogen atom of each of the "unsubstituted heterocyclic groups" in the set of specific examples G2A by a substituent, and the examples of the substituted heterocyclic groups in the set of specific examples G2B. The examples of the "unsubstituted heterocyclic group" and the examples of the "substituted heterocyclic group" enumerated herein are mere examples, and the "substituted heterocyclic group" in the description herein encompasses groups formed by substituting a hydrogen atom bonded to the ring atom of the heterocyclic group itself of each of the "substituted heterocyclic groups" in the set of specific examples G2B by a substituent, and groups formed by substituting a hydrogen atom of the substituent of each of the "substituted heterocyclic groups" in the set of specific examples G2B by a substituent.

The set of specific examples G2A includes, for example, the unsubstituted heterocyclic group containing a nitrogen atom (set of specific examples G2A1), the unsubstituted heterocyclic group containing an oxygen atom (set of specific examples G2A2), the unsubstituted heterocyclic group containing a sulfur atom (set of specific examples G2A3), and monovalent heterocyclic groups derived by removing one hydrogen atom from each of the ring structures represented by the following general formulae (TEMP-16) to (TEMP-33) (set of specific examples G2A4).

The set of specific examples G2B includes, for example, the substituted heterocyclic groups containing a nitrogen atom (set of specific examples G2B1), the substituted heterocyclic groups containing an oxygen atom (set of specific examples G2B2), the substituted heterocyclic groups containing a sulfur atom (set of specific examples G2B3), and groups formed by substituting one or more hydrogen atom of each of monovalent heterocyclic groups derived from the ring structures represented by the following general formulae (TEMP-16) to (TEMP-33) by a substituent (set of specific examples G2B4).

Unsubstituted Heterocyclic Group containing Nitrogen Atom (Set of Specific Examples G2A1):
a pyrrolyl group,
an imidazolyl group,
a pyrazolyl group,
a triazolyl group,
a tetrazolyl group,
an oxazolyl group,
an isoxazolyl group,
an oxadiazolyl group,
a thiazolyl group,
an isothiazolyl group,
a thiadiazolyl group,
a pyridyl group,
a pyridazinyl group,
a pyrimidinyl group,
a pyrazinyl group,
a triazinyl group,
an indolyl group,
an isoindolyl group,
an indolizinyl group,
a quinolizinyl group,
a quinolyl group,
an isoquinolyl group,
a cinnolinyl group,
a phthalazinyl group,
a quinazolinyl group,
a quinoxalinyl group,
a benzimidazolyl group,
an indazolyl group,
a phenanthrolinyl group,
a phenanthridinyl group,
an acridinyl group,
a phenazinyl group,
a carbazolyl group,
a benzocarbazolyl group,
a morpholino group,
a phenoxazinyl group,
a phenothiazinyl group,
an azacarbazolyl group, and
a diazacarbazolyl group.

Unsubstituted Heterocyclic Group containing Oxygen Atom (Set of Specific Examples G2A2):
a furyl group,
an oxazolyl group,
an isoxazolyl group,
an oxadiazolyl group,
a xanthenyl group,
a benzofuranyl group,
an isobenzofuranyl group,
a dibenzofuranyl group,
a naphthobenzofuranyl group,
a benzoxazolyl group,
a benzisoxazolyl group,
a phenoxazinyl group,
a morpholino group,
a dinaphthofuranyl group,
an azadibenzofuranyl group,
a diazadibenzofuranyl group,
an azanaphthobenzofuranyl group, and
a diazanaphthobenzofuranyl group.

Unsubstituted Heterocyclic Group containing Sulfur Atom (Set of Specific Examples G2A3):
a thienyl group,
a thiazolyl group,
an isothiazolyl group,
a thiadiazolyl group,
a benzothiophenyl group (benzothienyl group),
an isobenzothiophenyl group (isobenzothienyl group),
a dibenzothiophenyl group (dibenzothienyl group),
a naphthobenzothiophenyl group (naphthobenzothienyl group),
a benzothiazolyl group,
a benzisothiazolyl group,
a phenothiazinyl group,
a dinaphthothiophenyl group (dinaphthothienyl group),
an azadibenzothiophenyl group (azadibenzothienyl group),
a diazadibenzothiophenyl group (diazadibenzothienyl group),
an azanaphthobenzothiophenyl group (azanaphthobenzothienyl group), and
a diazanaphthobenzothiophenyl group (diazanaphthobenzothienyl group).

Monovalent Heterocyclic Group derived by removing One Hydrogen Atom from Ring Structures represented by General Formulae (TEMP-16) to (TEMP-33) (Set of Specific Examples G2A4)

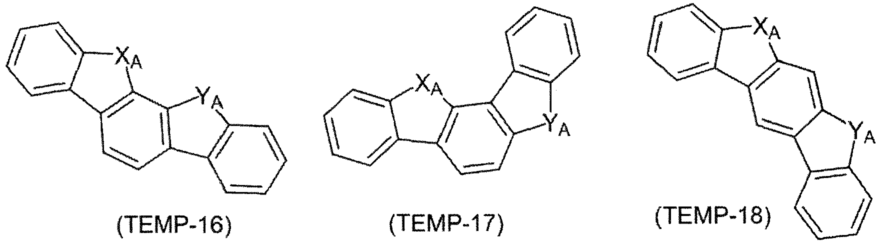

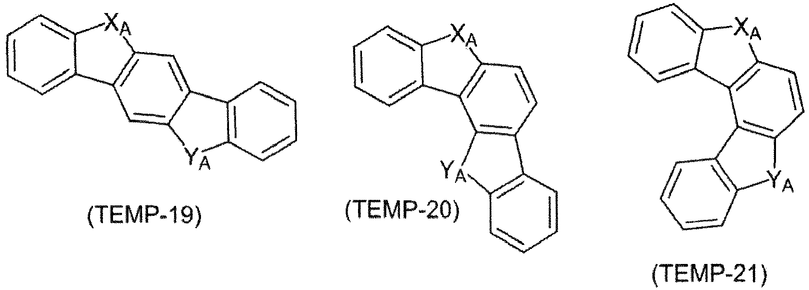

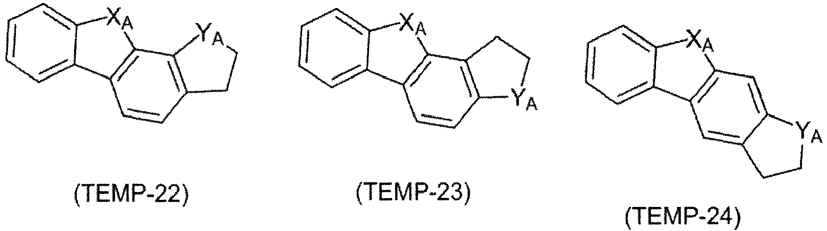

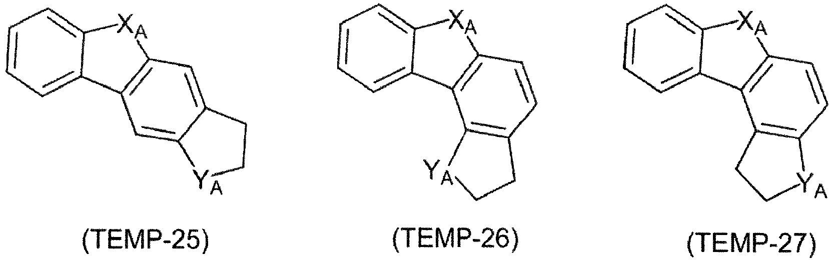

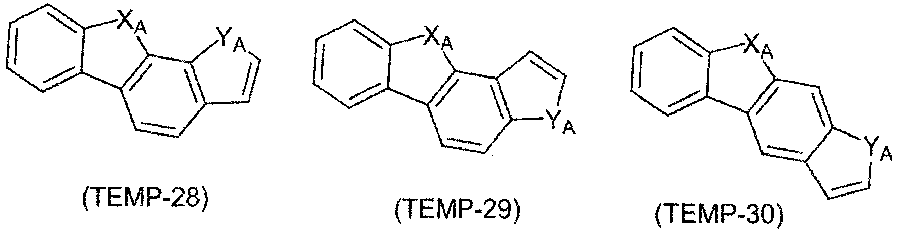

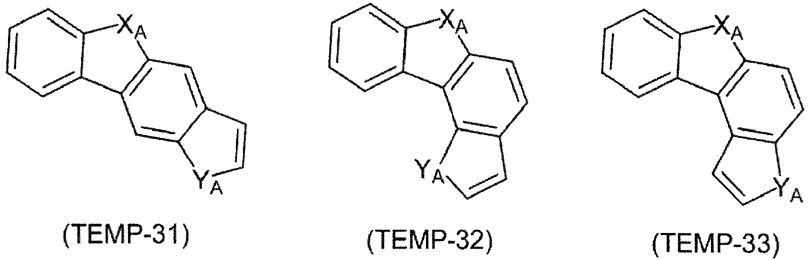

In the general formulae (TEMP-16) to (TEMP-33), X_{A} and Y_{A} each independently represent an oxygen atom, a sulfur atom, NH, or CH₂, provided that at least one of X_{A} and Y_{A} represents an oxygen atom, a sulfur atom, or NH.

In the general formulae (TEMP-16) to (TEMP-33), in the case where at least one of X_{A} and Y_{A} represents NH or CH₂, the monovalent heterocyclic groups derived from the ring structures represented by the general formulae (TEMP-16) to (TEMP-33) include monovalent groups formed by removing one hydrogen atom from the NH or CH₂.

Substituted Heterocyclic Group containing Nitrogen Atom (Set of Specific Examples G2B1):
a (9-phenyl)carbazolyl group,
a (9-biphenylyl)carbazolyl group,
a (9-phenyl)phenylcarbazolyl group,
a (9-naphthyl)carbazolyl group,
a diphenylcarbazol-9-yl group,
a phenylcarbazol-9-yl group,
a methylbenzimidazolyl group,
an ethylbenzimidazolyl group,
a phenyltriazinyl group,
a biphenyltriazinyl group,
a diphenyltriazinyl group,
a phenylquinazolinyl group, and
a biphenylquinazolinyl group.

Substituted Heterocyclic Group containing Oxygen Atom (Set of Specific Examples G2B2):
a phenyldibenzofuranyl group,
a methyldibenzofuranyl group,
a t-butyldibenzofuranyl group, and
a monovalent residual group of spiro[9H-xanthene-9,9'-[9H]fluorene].

Substituted Heterocyclic Group containing Sulfur Atom (Set of Specific Examples G2B3):
a phenyldibenzothiophenyl group,
a methyldibenzothiophenyl group,
a t-butyldibenzothiophenyl group, and
a monovalent residual group of spiro[9H-thioxanthene-9,9'-[9H]fluorene].

Group formed by substituting one or more Hydrogen Atom of Monovalent Heterocyclic Group derived from Ring Structures represented by General Formulae (TEMP-16) to (TEMP-33) by Substituent (Set of Specific Examples G2B4)

The "one or more hydrogen atom of the monovalent heterocyclic group" means one or more hydrogen atom selected from the hydrogen atom bonded to the ring carbon atom of the monovalent heterocyclic group, the hydrogen atom bonded to the nitrogen atom in the case where at least one of X_{A} and Y_{A} represents NH, and the hydrogen atom of the methylene group in the case where one of X_{A} and Y_{A} represents CH₂.

### Substituted or Unsubstituted Alkyl Group

In the description herein, specific examples (set of specific examples G3) of the "substituted or unsubstituted alkyl group" include the unsubstituted alkyl groups (set of specific examples G3A) and the substituted alkyl groups (set of specific examples G3B) shown below. (Herein, the unsubstituted alkyl group means the case where the "substituted or unsubstituted alkyl group" is an "unsubstituted alkyl group", and the substituted alkyl group means the case where the "substituted or unsubstituted alkyl group" is a "substituted alkyl group".) In the description herein, the simple expression "alkyl group" encompasses both the "unsubstituted alkyl group" and the "substituted alkyl group".

The "substituted alkyl group" means a group formed by substituting one or more hydrogen atom of the "unsubstituted alkyl group" by a substituent. Specific examples of the "substituted alkyl group" include groups formed by substituting one or more hydrogen atom of each of the "unsubstituted alkyl groups" (set of specific examples G3A) by a substituent, and the examples of the substituted alkyl groups (set of specific examples G3B). In the description herein, the alkyl group in the "unsubstituted alkyl group" means a chain-like alkyl group. Accordingly, the "unsubstituted alkyl group" encompasses an "unsubstituted linear alkyl group" and an "unsubstituted branched alkyl group". The examples of the "unsubstituted alkyl group" and the examples of the "substituted alkyl group" enumerated herein are mere examples, and the "substituted alkyl group" in the description herein encompasses groups formed by substituting a hydrogen atom of the alkyl group itself of each of the "substituted alkyl groups" in the set of specific examples G3B by a substituent, and groups formed by substituting a hydrogen atom of the substituent of each of the "substituted alkyl groups" in the set of specific examples G3B by a substituent.

Unsubstituted Alkyl Group (Set of Specific Examples G3A):
a methyl group,
an ethyl group,
a n-propyl group,
an isopropyl group,
a n-butyl group,
an isobutyl group,
a s-butyl group, and
a t-butyl group.

Substituted Alkyl Group (Set of Specific Examples G3B):
a heptafluoropropyl group (including isomers),
a pentafluoroethyl group,
a 2,2,2-trifluoroethyl group, and
a trifluoromethyl group.

### Substituted or Unsubstituted Alkenyl Group

In the description herein, specific examples (set of specific examples G4) of the "substituted or unsubstituted alkenyl group" include the unsubstituted alkenyl groups (set of specific examples G4A) and the substituted alkenyl groups (set of specific examples G4B) shown below. (Herein, the unsubstituted alkenyl group means the case where the "substituted or unsubstituted alkenyl group" is an "unsubstituted alkenyl group", and the substituted alkenyl group means the case where the "substituted or unsubstituted alkenyl group" is a "substituted alkenyl group".) In the description herein, the simple expression "alkenyl group" encompasses both the "unsubstituted alkenyl group" and the "substituted alkenyl group".

The "substituted alkenyl group" means a group formed by substituting one or more hydrogen atom of the "unsubstituted alkenyl group" by a substituent. Specific examples of the "substituted alkenyl group" include the "unsubstituted alkenyl groups" (set of specific examples G4A) that each have a substituent, and the examples of the substituted alkenyl groups (set of specific examples G4B). The examples of the "unsubstituted alkenyl group" and the examples of the "substituted alkenyl group" enumerated herein are mere examples, and the "substituted alkenyl group" in the description herein encompasses groups formed by substituting a hydrogen atom of the alkenyl group itself of each of the "substituted alkenyl groups" in the set of specific examples G4B by a substituent, and groups formed by substituting a hydrogen atom of the substituent of each of the "substituted alkenyl groups" in the set of specific examples G4B by a substituent.

Unsubstituted Alkenyl Group (Set of Specific Examples G4A):
a vinyl group,
an allyl group,
a 1-butenyl group,
a 2-butenyl group, and
a 3-butenyl group.

Substituted Alkenyl Group (Set of Specific Examples G4B):
a 1,3-butanedienyl group,
a 1-methylvinyl group,
a 1-methylallyl group,
a 1,1-dimethylallyl group,
a 2-methylallyl group, and
a 1,2-dimethylallyl group.

### Substituted or Unsubstituted Alkynyl Group

In the description herein, specific examples (set of specific examples G5) of the "substituted or unsubstituted alkynyl group" include the unsubstituted alkynyl group (set of specific examples G5A) shown below. (Herein, the unsubstituted alkynyl group means the case where the "substituted or unsubstituted alkynyl group" is an "unsubstituted alkynyl group".) In the description herein, the simple expression "alkynyl group" encompasses both the "unsubstituted alkynyl group" and the "substituted alkynyl group".

The "substituted alkynyl group" means a group formed by substituting one or more hydrogen atom of the "unsubstituted alkynyl group" by a substituent. Specific examples of the "substituted alkenyl group" include groups formed by substituting one or more hydrogen atom of the "unsubstituted alkynyl group" (set of specific examples G5A) by a substituent.

Unsubstituted Alkynyl Group (Set of Specific Examples G5A):
an ethynyl group.

### Substituted or Unsubstituted Cycloalkyl Group

In the description herein, specific examples (set of specific examples G6) of the "substituted or unsubstituted cycloalkyl group" include the unsubstituted cycloalkyl groups (set of specific examples G6A) and the substituted cycloalkyl group (set of specific examples G6B) shown below. (Herein, the unsubstituted cycloalkyl group means the case where the "substituted or unsubstituted cycloalkyl group" is an "unsubstituted cycloalkyl group", and the substituted cycloalkyl group means the case where the "substituted or unsubstituted cycloalkyl group" is a "substituted cycloalkyl group".) In the description herein, the simple expression "cycloalkyl group" encompasses both the "unsubstituted cycloalkyl group" and the "substituted cycloalkyl group".

The "substituted cycloalkyl group" means a group formed by substituting one or more hydrogen atom of the "unsubstituted cycloalkyl group" by a substituent. Specific examples of the "substituted cycloalkyl group" include groups formed by substituting one or more hydrogen atom of each of the "unsubstituted cycloalkyl groups" (set of specific examples G6A) by a substituent, and the example of the substituted cycloalkyl group (set of specific examples G6B). The examples of the "unsubstituted cycloalkyl group" and the examples of the "substituted cycloalkyl group" enumerated herein are mere examples, and the "substituted cycloalkyl group" in the description herein encompasses groups formed by substituting one or more hydrogen atom bonded to the carbon atoms of the cycloalkyl group itself of the "substituted cycloalkyl group" in the set of specific examples G6B by a substituent, and groups formed by substituting a hydrogen atom of the substituent of the "substituted cycloalkyl group" in the set of specific examples G6B by a substituent.

Unsubstituted Cycloalkyl Group (Set of Specific Examples G6A):
a cyclopropyl group,
a cyclobutyl group,
a cyclopentyl group,
a cyclohexyl group,
a 1-adamantyl group,
a 2-adamantyl group,
a 1-norbornyl group, and
a 2-norbornyl group.

Substituted Cycloalkyl Group (Set of Specific Examples G6B):
a 4-methylcyclohexyl group.

### Group represented by -Si(R₉₀₁)(R₉₀₂)(R₉₀₃)

In the description herein, specific examples (set of specific examples G7) of the group represented by -Si(R₉₀₁)(R₉₀₂)(R₉₀₃) include:

-Si(G1)(G1)(G1),

-Si(G1)(G2)(G2),

-Si(G1)(G1)(G2),

-Si(G2)(G2)(G2),

-Si(G3)(G3)(G3), and

-Si(G6)(G6)(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.
Plural groups represented by G1 in -Si(G1)(G1)(G1) are the same as or different from each other.
Plural groups represented by G2 in -Si(G1)(G2)(G2) are the same as or different from each other.
Plural groups represented by G1 in -Si(G1)(G1)(G2) are the same as or different from each other.
Plural groups represented by G2 in -Si(G2)(G2)(G2) are the same as or different from each other.
Plural groups represented by G3 in -Si(G3)(G3)(G3) are the same as or different from each other.
Plural groups represented by G6 in -Si(G6)(G6)(G6) are the same as or different from each other.

### Group represented by -O-(R₉₀₄)

In the description herein, specific examples (set of specific examples G8) of the group represented by -O-(R₉₀₄) include:

-O(G1),

-O(G2),

-O(G3), and

-O(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

### Group represented by -S-(R₉₀₅)

In the description herein, specific examples (set of specific examples G9) of the group represented by -S-(R₉₀₅) include:

-S(G1),

-S(G2),

-S(G3), and

-S(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

### Group represented by -N(R₉₀₆)(R₉₀₇)

In the description herein, specific examples (set of specific examples G10) of the group represented by -N(R₉₀₆)(R₉₀₇) include:

-N(G1)(G1),

-N(G2)(G2),

-N(G1)(G2),

-N(G3)(G3),

and

-N(G6)(G6).

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Plural groups represented by G1 in -N(G1)(G1) are the same as or different from each other.

Plural groups represented by G2 in -N(G2)(G2) are the same as or different from each other.

Plural groups represented by G3 in -N(G3)(G3) are the same as or different from each other.

Plural groups represented by G6 in -N(G6)(G6) are the same as or different from each other.

### Halogen Atom

In the description herein, specific examples (set of specific examples G11) of the "halogen atom" include a fluorine atom, a chlorine atom, a bromine atom, and an iodine atom.

### Substituted or Unsubstituted Fluoroalkyl Group

In the description herein, the "substituted or unsubstituted fluoroalkyl group" means a group formed by substituting at least one hydrogen atom bonded to the carbon atom constituting the alkyl group in the "substituted or unsubstituted alkyl group" by a fluorine atom, and encompasses a group formed by substituting all the hydrogen atoms bonded to the carbon atoms constituting the alkyl group in the "substituted or unsubstituted alkyl group" by fluorine atoms (i.e., a perfluoroalkyl group). The number of carbon atoms of the "unsubstituted fluoroalkyl group" is 1 to 50, preferably 1 to 30, and more preferably 1 to 18, unless otherwise indicated in the description. The "substituted fluoroalkyl group" means a group formed by substituting one or more hydrogen atom of the "fluoroalkyl group" by a substituent. In the description herein, the "substituted fluoroalkyl group" encompasses a group formed by substituting one or more hydrogen atom bonded to the carbon atom of the alkyl chain in the "substituted fluoroalkyl group" by a substituent, and a group formed by substituting one or more hydrogen atom of the substituent in the "substituted fluoroalkyl group" by a substituent. Specific examples of the "unsubstituted fluoroalkyl group" include examples of groups formed by substituting one or more hydrogen atom in each of the "alkyl group" (set of specific examples G3) by a fluorine atom.

### Substituted or Unsubstituted Haloalkyl Group

In the description herein, the "substituted or unsubstituted haloalkyl group" means a group formed by substituting at least one hydrogen atom bonded to the carbon atom constituting the alkyl group in the "substituted or unsubstituted alkyl group" by a halogen atom, and encompasses a group formed by substituting all the hydrogen atoms bonded to the carbon atoms constituting the alkyl group in the "substituted or unsubstituted alkyl group" by halogen atoms. The number of carbon atoms of the "unsubstituted haloalkyl group" is 1 to 50, preferably 1 to 30, and more preferably 1 to 18, unless otherwise indicated in the description. The "substituted haloalkyl group" means a group formed by substituting one or more hydrogen atom of the "haloalkyl group" by a substituent. In the description herein, the "substituted haloalkyl group" encompasses a group formed by substituting one or more hydrogen atom bonded to the carbon atom of the alkyl chain in the "substituted haloalkyl group" by a substituent, and a group formed by substituting one or more hydrogen atom of the substituent in the "substituted haloalkyl group" by a substituent. Specific examples of the "unsubstituted haloalkyl group" include examples of groups formed by substituting one or more hydrogen atom in each of the "alkyl group" (set of specific examples G3) by a halogen atom. A haloalkyl group may be referred to as a halogenated alkyl group in some cases.

### Substituted or Unsubstituted Alkoxy Group

In the description herein, specific examples of the "substituted or unsubstituted alkoxy group" include a group represented by -O(G3), wherein G3 represents the "substituted or unsubstituted alkyl group" described in the set of specific examples G3. The number of carbon atoms of the "unsubstituted alkoxy group" is 1 to 50, preferably 1 to 30, and more preferably 1 to 18, unless otherwise indicated in the description.

### Substituted or Unsubstituted Alkylthio Group

In the description herein, specific examples of the "substituted or unsubstituted alkylthio group" include a group represented by -S(G3), wherein G3 represents the "substituted or unsubstituted alkyl group" described in the set of specific examples G3. The number of carbon atoms of the "unsubstituted alkylthio group" is 1 to 50, preferably 1 to 30, and more preferably 1 to 18, unless otherwise indicated in the description.

### Substituted or Unsubstituted Aryloxy Group

In the description herein, specific examples of the "substituted or unsubstituted aryloxy group" include a group represented by -O(G1), wherein G1 represents the "substituted or unsubstituted aryl group" described in the set of specific examples G1. The number of ring carbon atoms of the "unsubstituted aryloxy group" is 6 to 50, preferably 6 to 30, and more preferably 6 to 18, unless otherwise indicated in the description.

### Substituted or Unsubstituted Arylthio Group

In the description herein, specific examples of the "substituted or unsubstituted arylthio group" include a group represented by -S(G1), wherein G1 represents the "substituted or unsubstituted aryl group" described in the set of specific examples G1. The number of ring carbon atoms of the "unsubstituted arylthio group" is 6 to 50, preferably 6 to 30, and more preferably 6 to 18, unless otherwise indicated in the description.

### Substituted or Unsubstituted Trialkylsilyl Group

In the description herein, specific examples of the "trialkylsilyl group" include a group represented by -Si(G3)(G3)(G3), wherein G3 represents the "substituted or unsubstituted alkyl group" described in the set of specific examples G3. Plural groups represented by G3 in -Si(G3)(G3)(G3) are the same as or different from each other. The number of carbon atoms of each of alkyl groups of the "substituted or unsubstituted trialkylsilyl group" is 1 to 50, preferably 1 to 20, and more preferably 1 to 6, unless otherwise indicated in the description.

### Substituted or Unsubstituted Aralkyl Group

In the description herein, specific examples of the "substituted or unsubstituted aralkyl group" include a group represented by -(G3)-(G1), wherein G3 represents the "substituted or unsubstituted alkyl group" described in the set of specific examples G3, and G1 represents the "substituted or unsubstituted aryl group" described in the set of specific examples G1. Accordingly, the "aralkyl group" is a group formed by substituting a hydrogen atom of an "alkyl group" by an "aryl group" as a substituent, and is one embodiment of the "substituted alkyl group". The "unsubstituted aralkyl group" is an "unsubstituted alkyl group" that is substituted by an "unsubstituted aryl group", and the number of carbon atoms of the "unsubstituted aralkyl group" is 7 to 50, preferably 7 to 30, and more preferably 7 to 18, unless otherwise indicated in the description.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, and a 2-β-naphthylisopropyl group.

In the description herein, the substituted or unsubstituted aryl group is preferably a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group, a 9,9-diphenylfluorenyl group, and the like, unless otherwise indicated in the description.

In the description herein, the substituted or unsubstituted heterocyclic group is preferably a pyridyl group, a pyrimidinyl group, a triazinyl group, a quinolyl group, an isoquinolyl group, a quinazolinyl group, a benzimidazolyl group, a phenanthrolinyl group, a carbazolyl group (e.g., a 1-carbazolyl, group, a 2-carbazolyl, group, a 3-carbazolyl, group, a 4-carbazolyl, group, or a 9-carbazolyl, group), a benzocarbazolyl group, an azacarbazolyl group, a diazacarbazolyl group, a dibenzofuranyl group, a naphthobenzofuranly group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (e.g., a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like, unless otherwise indicated in the description.

In the description herein, the carbazolyl group is specifically any one of the following groups unless otherwise indicated in the description.

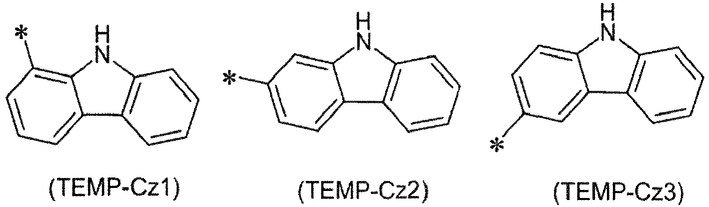

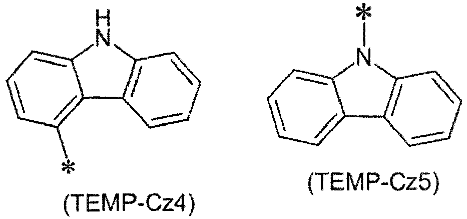

In the description herein, the (9-phenyl)carbazolyl group is specifically any one of the following groups unless otherwise indicated in the description.

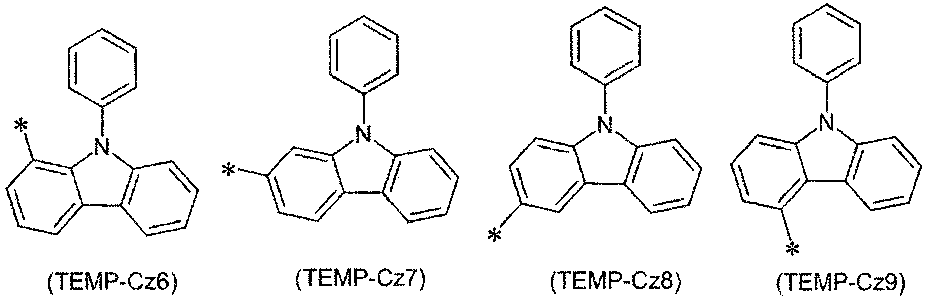

In the general formulae (TEMP-Czl) to (TEMP-Cz9), * represents a bonding site.

In the description herein, the dibenzofuranyl group and the dibenzothiophenyl group are specifically any one of the following groups unless otherwise indicated in the description.

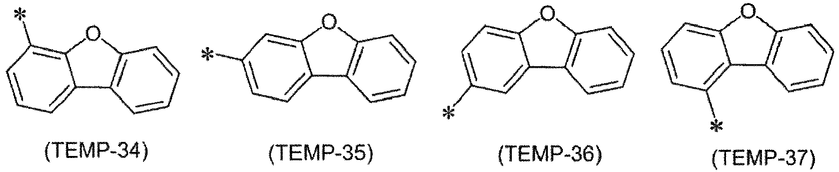

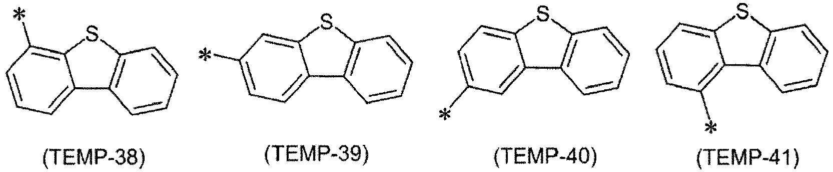

In the general formulae (TEMP-34) to (TEMP-41), * represents a bonding site.

In the description herein, the substituted or unsubstituted alkyl group is preferably a methyl group, an ethyl group, a propyl group, an isopropyl group, a n-butyl group, an isobutyl group, a t-butyl group, or the like unless otherwise indicated in the description.

### Substituted or Unsubstituted Arylene Group

In the description herein, the "substituted or unsubstituted arylene group" is a divalent group derived by removing one hydrogen atom on the aryl ring from the "substituted or unsubstituted aryl group" described above unless otherwise indicated in the description. Specific examples (set of specific examples G12) of the "substituted or unsubstituted arylene group" include divalent groups derived by removing one hydrogen atom on the aryl ring from the "substituted or unsubstituted aryl groups" described in the set of specific examples G1.

### Substituted or Unsubstituted Divalent Heterocyclic Group

In the description herein, the "substituted or unsubstituted divalent heterocyclic group" is a divalent group derived by removing one hydrogen atom on the heterocyclic ring from the "substituted or unsubstituted heterocyclic group" described above unless otherwise indicated in the description. Specific examples (set of specific examples G13) of the "substituted or unsubstituted divalent heterocyclic group" include divalent groups derived by removing one hydrogen atom on the heterocyclic ring from the "substituted or unsubstituted heterocyclic groups" described in the set of specific examples G2.

### Substituted or Unsubstituted Alkylene Group

In the description herein, the "substituted or unsubstituted alkylene group" is a divalent group derived by removing one hydrogen atom on the alkyl chain from the "substituted or unsubstituted alkyl group" described above unless otherwise indicated in the description. Specific examples (set of specific examples G14) of the "substituted or unsubstituted alkylene group" include divalent groups derived by removing one hydrogen atom on the alkyl chain from the "substituted or unsubstituted alkyl groups" described in the set of specific examples G3.

In the description herein, the substituted or unsubstituted arylene group is preferably any one of the groups represented by the following general formulae (TEMP-42) to (TEMP-68) unless otherwise indicated in the description.

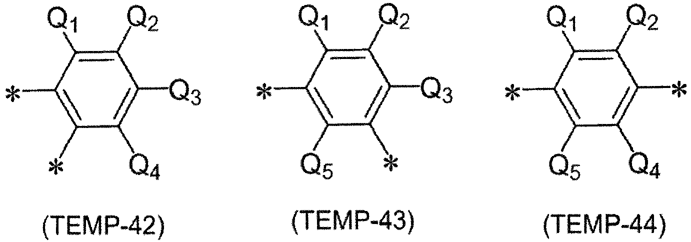

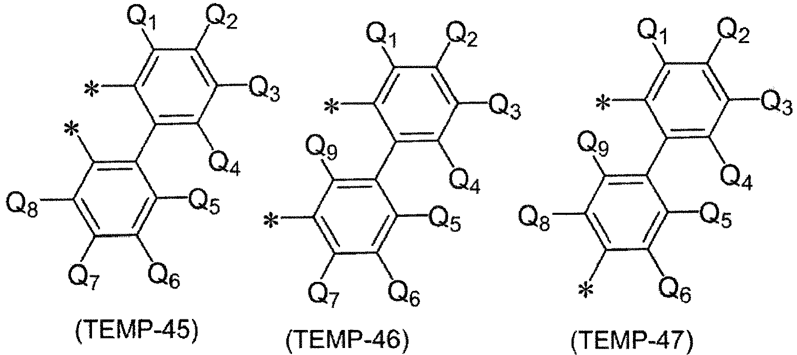

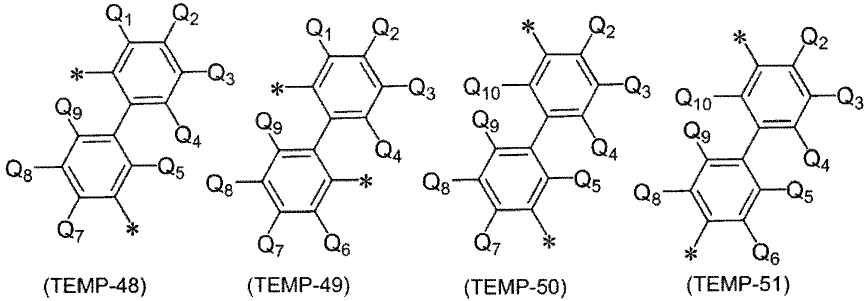

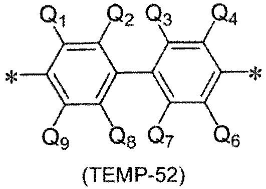

In the general formulae (TEMP-42) to (TEMP-52), Q₁ to Q₁₀ each independently represent a hydrogen atom or a substituent.

In the general formulae (TEMP-42) to (TEMP-52), * represents a bonding site.

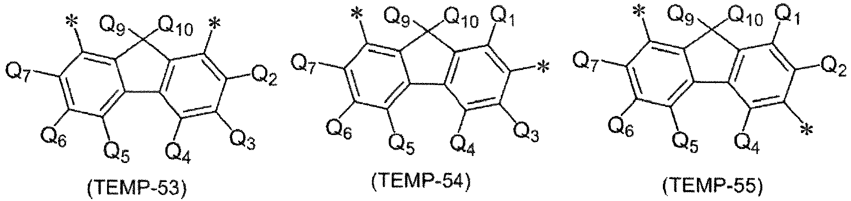

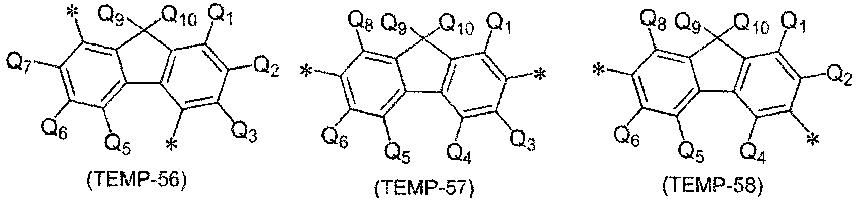

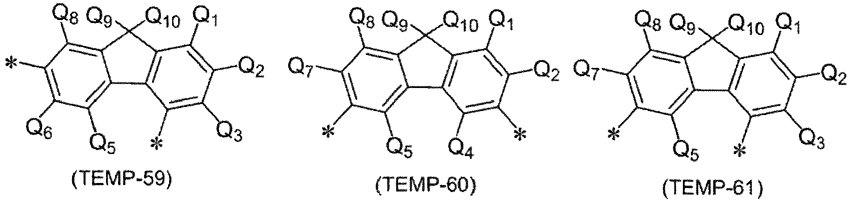

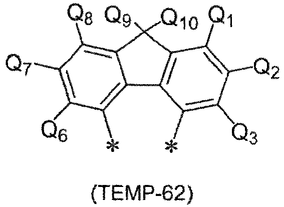

In the general formulae (TEMP-53) to (TEMP-62), Q₁ to Q₁₀ each independently represent a hydrogen atom or a substituent.

The formulae Q₉ and Q₁₀ may be bonded to each other to form a ring via a single bond.

In the general formulae (TEMP-53) to (TEMP-62), * represents a bonding site.

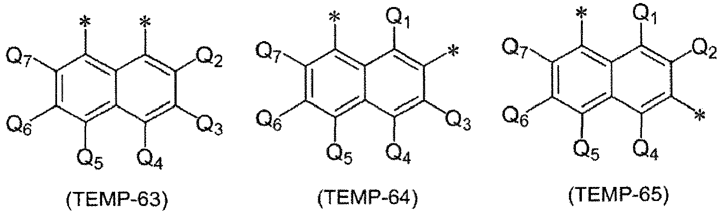

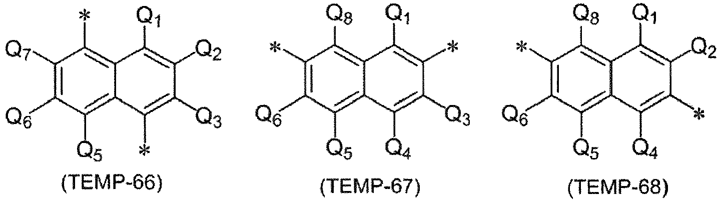

In the general formulae (TEMP-63) to (TEMP-68), Q₁ to Q₈ each independently represent a hydrogen atom or a substituent.

In the general formulae (TEMP-63) to (TEMP-68), * represents a bonding site.

In the description herein, the substituted or unsubstituted divalent heterocyclic group is preferably the groups represented by the following general formulae (TEMP-69) to (TEMP-102) unless otherwise indicated in the description.

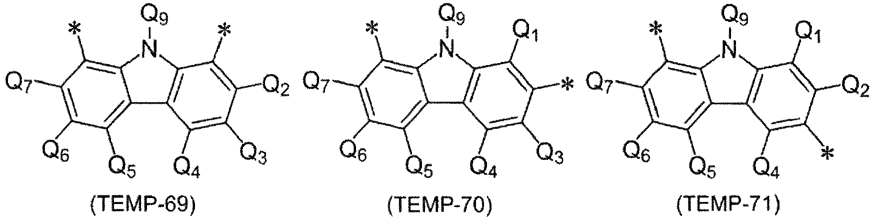

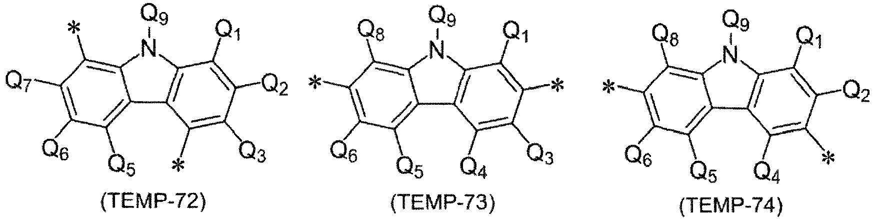

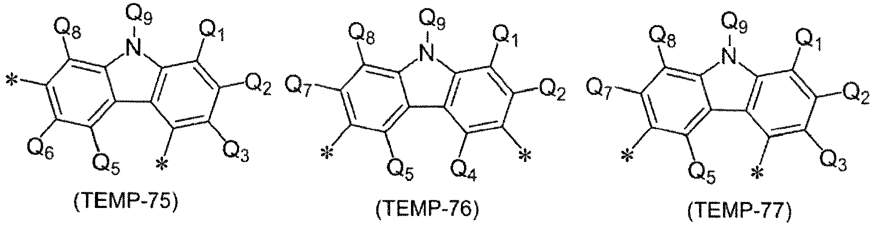

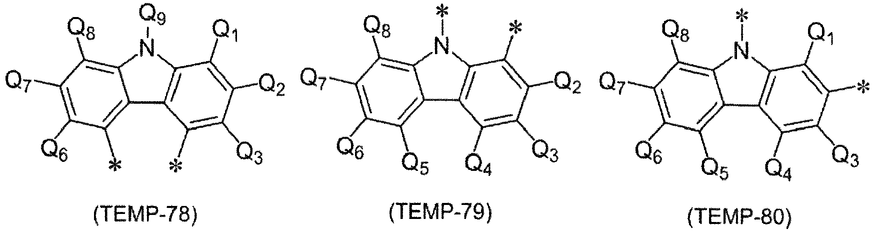

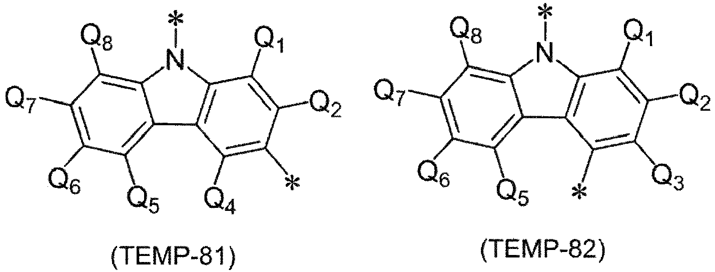

In the general formulae (TEMP-69) to (TEMP-82), Q₁ to Q₉ each independently represent a hydrogen atom or a substituent.

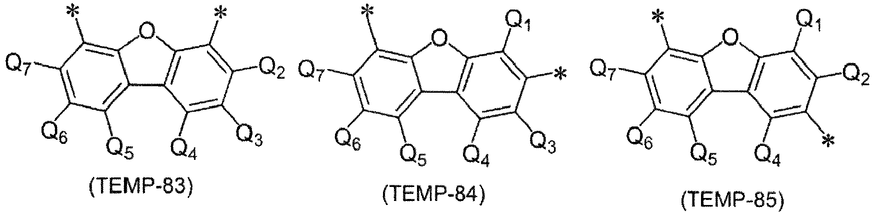

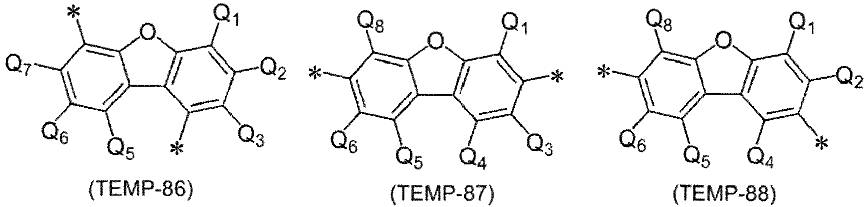

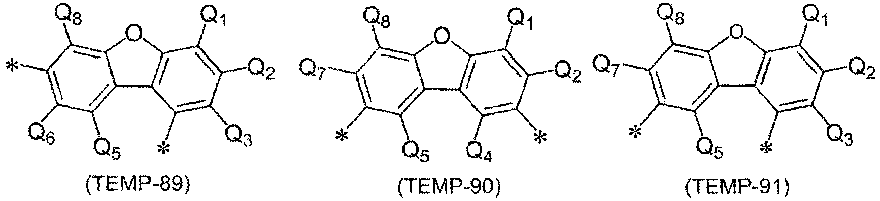

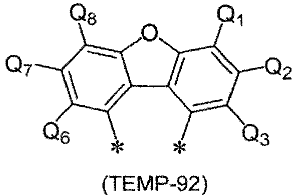

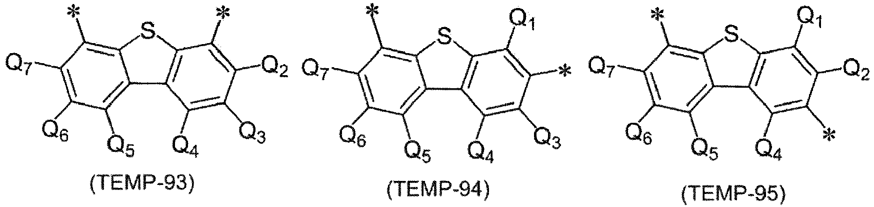

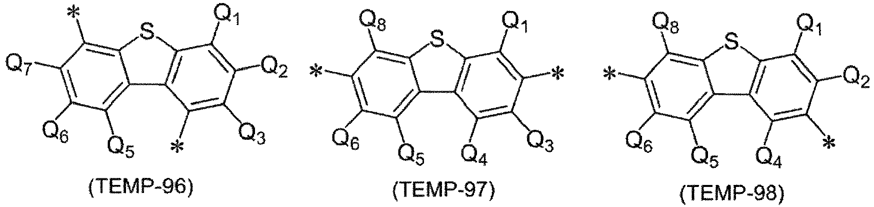

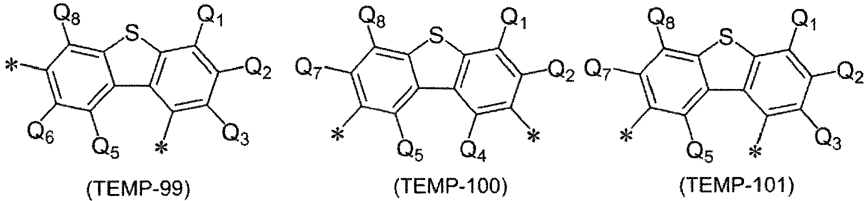

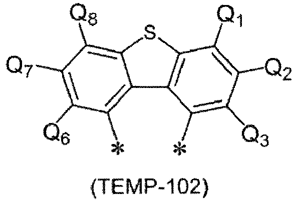

In the general formulae (TEMP-83) to (TEMP-102), Q₁ to Q₈ each independently represent a hydrogen atom or a substituent.

The above are the explanation of the "substituents in the description herein".

### Case forming Ring by bonding

In the description herein, the case where "one or more combinations of combinations each including adjacent two or more each are bonded to each other to form a substituted or unsubstituted monocyclic ring, or each are bonded to each other to form a substituted or unsubstituted condensed ring, or each are not bonded to each other" means a case where "one or more combinations of combinations each including adjacent two or more each are bonded to each other to form a substituted or unsubstituted monocyclic ring", a case where "one or more combinations of combinations each including adjacent two or more each are bonded to each other to form a substituted or unsubstituted condensed ring", and a case where "one or more combinations of combinations each including adjacent two or more each are not bonded to each other".

In the description herein, the case where "one or more combinations of combinations each including adjacent two or more each are bonded to each other to form a substituted or unsubstituted monocyclic ring" and the case where "one or more combinations of combinations each including adjacent two or more each are bonded to each other to form a substituted or unsubstituted condensed ring" (which may be hereinafter collectively referred to as a "case forming a ring by bonding") will be explained below. The cases will be explained for the anthracene compound represented by the following general formula (TEMP-103) having an anthracene core skeleton as an example.

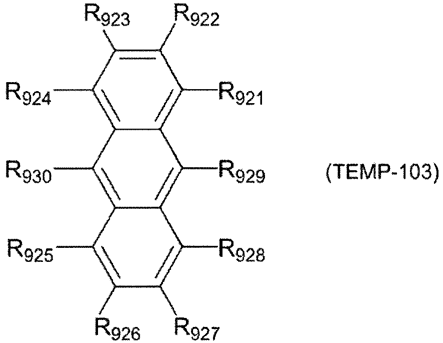

For example, in the case where "one or more combinations of combinations each including adjacent two or more each are bonded to each other to form a ring" among R₉₂₁ to R₉₃₀, the combinations each including adjacent two as one combination include a combination of R₉₂₁ and R₉₂₂, a combination of R₉₂₂ and R₉₂₃, a combination of R₉₂₃ and R₉₂₄, a combination of R₉₂₄ and R₉₃₀, a combination of R₉₃₀ and R₉₂₅, a combination of R₉₂₅ and R₉₂₆, a combination of R₉₂₆ and R₉₂₇, a combination of R₉₂₇ and R₉₂₈, a combination of R₉₂₈ and R₉₂₉, and a combination of R₉₂₉ and R₉₂₁.

The "one or more combinations" mean that two or more combinations each including adjacent two or more may form rings simultaneously. For example, in the case where R₉₂₁ and R₉₂₂ are bonded to each other to form a ring Q_{A}, and simultaneously R₉₂₅ and R₉₂₆ are bonded to each other to form a ring Q_{B}, the anthracene compound represented by the general formula (TEMP-103) is represented by the following general formula (TEMP-104).

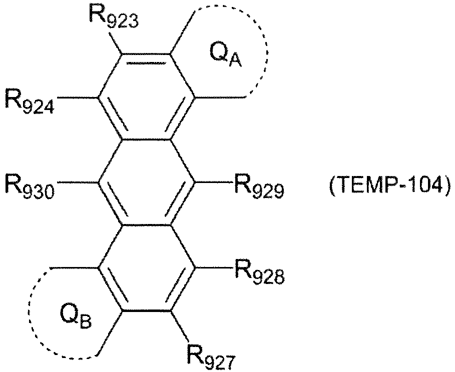

The case where the "combination including adjacent two or more forms rings" encompasses not only the case where adjacent two included in the combination are bonded as in the aforementioned example, but also the case where adjacent three or more included in the combination are bonded. For example, this case means that R₉₂₁ and R₉₂₂ are bonded to each other to form a ring Q_{A}, R₉₂₂ and R₉₂₃ are bonded to each other to form a ring Qc, and adjacent three (R₉₂₁, R₉₂₂, and R₉₂₃) included in the combination are bonded to each other to form rings, which are condensed to the anthracene core skeleton, and in this case, the anthracene compound represented by the general formula (TEMP-103) is represented by the following general formula (TEMP-105). In the following general formula (TEMP-105), the ring Q_{A} and the ring Qc share R₉₂₂.

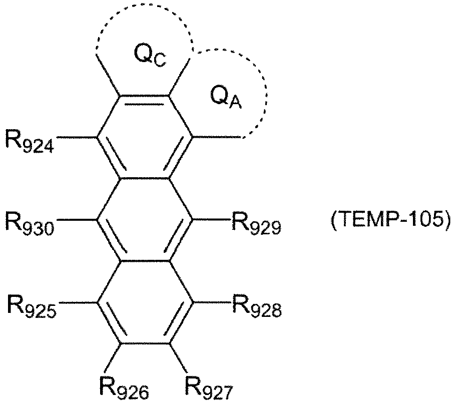

The formed "monocyclic ring" or "condensed ring" may be a saturated ring or an unsaturated ring in terms of structure of the formed ring itself. In the case where the "one combination including adjacent two" forms a "monocyclic ring" or a "condensed ring", the "monocyclic ring" or the "condensed ring" may form a saturated ring or an unsaturated ring. For example, the ring Q_{A} and the ring Q_{B} formed in the general formula (TEMP-104) each are a "monocyclic ring" or a "condensed ring". The ring Q_{A} and the ring Qc formed in the general formula (TEMP-105) each are a "condensed ring". The ring Q_{A} and the ring Qc in the general formula (TEMP-105) form a condensed ring through condensation of the ring Q_{A} and the ring Qc. In the case where the ring Q_{A} in the general formula (TMEP-104) is a benzene ring, the ring Q_{A} is a monocyclic ring. In the case where the ring Q_{A} in the general formula (TMEP-104) is a naphthalene ring, the ring Q_{A} is a condensed ring.

The "unsaturated ring" means an aromatic hydrocarbon ring or an aromatic heterocyclic ring. The "saturated ring" means an aliphatic hydrocarbon ring or a non-aromatic heterocyclic ring.

Specific examples of the aromatic hydrocarbon ring include the structures formed by terminating the groups exemplified as the specific examples in the set of specific examples G1 with a hydrogen atom.

Specific examples of the aromatic heterocyclic ring include the structures formed by terminating the aromatic heterocyclic groups exemplified as the specific examples in the set of specific examples G2 with a hydrogen atom.

Specific examples of the aliphatic hydrocarbon ring include the structures formed by terminating the groups exemplified as the specific examples in the set of specific examples G6 with a hydrogen atom.

The expression "to form a ring" means that the ring is formed only with the plural atoms of the core structure or with the plural atoms of the core structure and one or more arbitrary element. For example, the ring Q_{A} formed by bonding R₉₂₁ and R₉₂₂ each other shown in the general formula (TEMP-104) means a ring formed with the carbon atom of the anthracene skeleton bonded to R₉₂₁, the carbon atom of the anthracene skeleton bonded to R₉₂₂, and one or more arbitrary element. As a specific example, in the case where the ring Q_{A} is formed with R₉₂₁ and R₉₂₂, and in the case where a monocyclic unsaturated ring is formed with the carbon atom of the anthracene skeleton bonded to R₉₂₁, the carbon atom of the anthracene skeleton bonded to R₉₂₂, and four carbon atoms, the ring formed with R₉₂₁ and R₉₂₂is a benzene ring.

Herein, the "arbitrary element" is preferably at least one kind of an element selected from the group consisting of a carbon element, a nitrogen element, an oxygen element, and a sulfur element, unless otherwise indicated in the description. For the arbitrary element (for example, for a carbon element or a nitrogen element), a bond that does not form a ring may be terminated with a hydrogen atom or the like, and may be substituted by an "arbitrary substituent" described later. In the case where an arbitrary element other than a carbon element is contained, the formed ring is a heterocyclic ring.

The number of the "one or more arbitrary element" constituting the monocyclic ring or the condensed ring is preferably 2 or more and 15 or less, more preferably 3 or more and 12 or less, and further preferably 3 or more and 5 or less, unless otherwise indicated in the description.

What is preferred between the "monocyclic ring" and the "condensed ring" is the "monocyclic ring" unless otherwise indicated in the description.

What is preferred between the "saturated ring" and the "unsaturated ring" is the "unsaturated ring" unless otherwise indicated in the description.

The "monocyclic ring" is preferably a benzene ring unless otherwise indicated in the description.

The "unsaturated ring" is preferably a benzene ring unless otherwise indicated in the description.

In the case where the "one or more combinations of combinations each including adjacent two or more" each are "bonded to each other to form a substituted or unsubstituted monocyclic ring", or each are "bonded to each other to form a substituted or unsubstituted condensed ring", it is preferred that the one or more combinations of combinations each including adjacent two or more each are bonded to each other to form a substituted or unsubstituted "unsaturated ring" containing the plural atoms of the core skeleton and 1 or more and 15 or less at least one kind of an element selected from the group consisting of a carbon element, a nitrogen element, an oxygen element, and a sulfur element, unless otherwise indicated in the description.

In the case where the "monocyclic ring" or the "condensed ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

In the case where the "saturated ring" or the "unsaturated ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

The above are the explanation of the case where "one or more combinations of combinations each including adjacent two or more" each are "bonded to each other to form a substituted or unsubstituted monocyclic ring", and the case where "one or more combinations of combinations each including adjacent two or more" each are "bonded to each other to form a substituted or unsubstituted condensed ring" (i.e., the "case forming a ring by bonding").

### Substituent for "Substituted or Unsubstituted"

In one embodiment in the description herein, the substituent for the case of "substituted or unsubstituted" (which may be hereinafter referred to as an "arbitrary substituent") is, for example, a group selected from the group consisting of
an unsubstituted alkyl group having 1 to 50 carbon atoms,
an unsubstituted alkenyl group having 2 to 50 carbon atoms,
an unsubstituted alkynyl group having 2 to 50 carbon atoms,
an unsubstituted cycloalkyl group having 3 to 50 ring carbon atoms,

   -Si(R₉₀₁)(R₉₀₂)(R₉₀₃),

   -O-(R₉₀₄),

   -S-(R₉₀₅),

   -N(R₉₀₆)(R₉₀₇),
a halogen atom, a cyano group, a nitro group,
an unsubstituted aryl group having 6 to 50 ring carbon atoms, and
an unsubstituted heterocyclic group having 5 to 50 ring atoms,
wherein R₉₀₁ to R₉₀₇ each independently represent
a hydrogen atom,
a substituted or unsubstituted alkyl group having 1 to 50 carbon atoms
a substituted or unsubstituted cycloalkyl group having 3 to 50 ring carbon atoms,
a substituted or unsubstituted aryl group having 6 to 50 ring carbon atoms, or
a substituted or unsubstituted heterocyclic group having 5 to 50 ring atoms.

In the case where two or more groups each represented by R₉₀₁ exist, the two or more groups each represented by R₉₀₁are the same as or different from each other,
in the case where two or more groups each represented by R₉₀₂ exist, the two or more groups each represented by R₉₀₂ are the same as or different from each other,
in the case where two or more groups each represented by R₉₀₃ exist, the two or more groups each represented by R₉₀₃ are the same as or different from each other,
in the case where two or more groups each represented by R₉₀₄ exist, the two or more groups each represented by R₉₀₄ are the same as or different from each other,
in the case where two or more groups each represented by R₉₀₅ exist, the two or more groups each represented by R₉₀₅ are the same as or different from each other,
in the case where two or more groups each represented by R₉₀₆ exist, the two or more groups each represented by R₉₀₆ are the same as or different from each other, and
in the case where two or more groups each represented by R₉₀₇ exist, the two or more groups each represented by R₉₀₇ are the same as or different from each other.

In one embodiment, the substituent for the case of "substituted or unsubstituted" may be a group selected from the group consisting of
an alkyl group having 1 to 50 carbon atoms,
an aryl group having 6 to 50 ring carbon atoms, and
a heterocyclic group having 5 to 50 ring atoms.

In one embodiment, the substituent for the case of "substituted or unsubstituted" may be a group selected from the group consisting of
an alkyl group having 1 to 18 carbon atoms,
an aryl group having 6 to 18 ring carbon atoms, and
a heterocyclic group having 5 to 18 ring atoms.

The specific examples of the groups for the arbitrary substituent described above are the specific examples of the substituent described in the section "Substituents in Description" described above.

In the description herein, the arbitrary adjacent substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, and more preferably form a benzene ring, unless otherwise indicated.

In the description herein, the arbitrary substituent may further have a substituent unless otherwise indicated in the description. The definition of the substituent that the arbitrary substituent further has may be the same as the arbitrary substituent.

In the description herein, a numerical range shown by "AA to BB" means a range including the numerical value AA as the former of "AA to BB" as the lower limit value and the numerical value BB as the latter of "AA to BB" as the upper limit value.

The compound of the present invention will be described below.

The compound of the present invention is a compound represented by the following formula (1) and having a molecular weight of 650 or more. In the following description, the compounds of the present invention represented by the formula (1) and the subordinate formulae of the formula (1) described later each may be referred simply to as an "inventive compound".

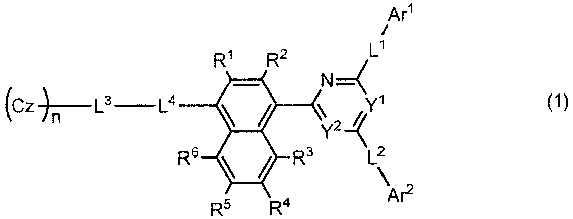

The molecular weight of the compound represented by the formula (1) of the present invention is 650 or more. When the molecular weight of the inventive compound is less than 650, the glass transition temperature Tg tends to be low, and the life of the device may be shortened from the viewpoint of heat resistance to heat generated when the device is driven (hereinafter may be referred to as "driving heat").

The molecular weight of the inventive compound is preferably 650 to 5000, more preferably 650 to 3000, and further more preferably 650 to 1000. When the molecular weight is in the range, the glass transition temperature Tg can be maintained high. Therefore, the inventive compound is relatively stable against heat such as driving heat, deterioration of film characteristics, deformation and the like are suppressed, and the life of the device is improved.

The glass transition temperature of the inventive compound is preferably 125°C or higher, more preferably 130°C or higher, and further more preferably 140°C or higher.

The symbols in the aforementioned formulae and the formulae described later will be explained below. Unless otherwise specified, the same symbols have the same meaning.

One of Y¹ and Y² is a nitrogen atom and the other one is CR. That is, Y¹ is a nitrogen atom and Y² is CR, or Y¹ is CR and Y² is a nitrogen atom.

n is an integer of 1 to 3, and when n is an integer of 2 or 3, Cz's, which are 2 or 3, are the same or different from each other.

R is selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a fluorine atom, and a cyano group. Preferably, R is selected from a hydrogen atom, a substituted or unsubstituted aryl group having 6 to 50 ring carbon atoms, a substituted or unsubstituted heterocyclic group having 5 to 50 ring atoms, and a substituted or unsubstituted alkyl group having 1 to 50 carbon atoms. More preferably, R is a hydrogen atom.

The detail of the substituted or unsubstituted aryl group having 6 to 50 ring carbon atoms is described in "Substituents in Description", more preferably a phenyl group, a p-biphenyl group, an m-biphenyl group, an o-biphenyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-terphenyl-4-yl group, an o-terphenyl-3-yl group, an o-terphenyl-2-yl group, a 1-naphthyl group, a 2-naphthyl group, a fluorenyl group, a 9,9'-spirobifluorenyl group, a 9,9-dimethylfluorenyl group, or a 9,9-diphenylfluorenyl group, and further more preferably a phenyl group, a p-biphenyl group, an m-biphenyl group, an o-biphenyl group, a 1-naphthyl group, a 2-naphthyl group, a fluorenyl group, a 9,9'-spirobifluorenyl group, a 9,9-dimethylfluorenyl group, or a 9,9-diphenylfluorenyl group.

The detail of the substituted or unsubstituted heterocyclic group having 5 to 50 ring atoms is described in "Substituents in Description", more preferably a carbazolyl group (1-carbazolyl group, 2-carbazolyl group, 3-carbazolyl group, 4-carbazolyl group, or 9-carbazolyl group), a dibenzofuranyl group, a naphthobenzofuranyl group, a dibenzothiophenyl group, a naphthobenzothiophenyl group, a phenyldibenzofuranyl group, or a phenyldibenzothiophenyl group, and further more preferably a carbazolyl group (1-carbazolyl group, 2-carbazolyl group, 3-carbazolyl group, 4-carbazolyl group, or 9-carbazolyl group), a dibenzofuranyl group, or a dibenzothiophenyl group.

The detail of the substituted or unsubstituted alkyl group having 1 to 50 carbon atoms is described in "Substituents in Description", preferably a methyl group, an ethyl group, an n-propyl group, an isopropyl group, an n-butyl group, an isobutyl group, an s-butyl group, or a t-butyl group, and more preferably a methyl group, an isopropyl group, or a t-butyl group.

The detail of the substituted or unsubstituted cycloalkyl group having 3 to 50 ring carbon atoms is described in "Substituents in Description", preferably a cyclopropyl group, a cyclobutyl group, a cyclopentyl group, or a cyclohexyl group, and more preferably a cyclopropyl group, a cyclopentyl group, or a cyclohexyl group.

R¹ to R⁶ each are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a fluorine atom, and a cyano group, preferably are selected from a hydrogen atom, a substituted or unsubstituted aryl group having 6 to 50 ring carbon atoms, a substituted or unsubstituted heterocyclic group having 5 to 50 ring atoms, and a substituted or unsubstituted alkyl group having 1 to 50 carbon atoms, and more preferably are a hydrogen atom.

Adjacent two selected from R¹ to R⁶ are not bonded to each other to form a ring.

The details of the substituted or unsubstituted aryl group having 6 to 50 ring carbon atoms, the substituted or unsubstituted heterocyclic group having 5 to 50 ring atoms, the substituted or unsubstituted alkyl group having 1 to 50 carbon atoms, and the substituted or unsubstituted cycloalkyl group having 3 to 50 ring carbon atoms each are the same as the details of the corresponding groups described above with respect to R.

L³ and L⁴ each are independently a single bond or a substituted or unsubstituted arylene group having 6 to 50 ring carbon atoms.

However, when n is an integer of 2 or 3, L⁴ is a substituted or unsubstituted arylene group having 6 to 50 ring carbon atoms.

As an embodiment of L³ and L⁴, it is preferable that L³ and L⁴ each are independently a substituted or unsubstituted arylene group having 6 to 50 ring carbon atoms.

As another embodiment, it is preferable that L³ is a single bond and L⁴ is a substituted or unsubstituted arylene group having 6 to 50 ring carbon atoms, or L³ and L⁴ are single bonds.

The details of the substituted or unsubstituted arylene group having 6 to 50 carbon atoms are described in "Substituents in Description", and examples thereof include a divalent group derived by removing one hydrogen atom on the aryl ring from the aforementioned "substituted or unsubstituted aryl group having 6 to 50 carbon atoms".

Preferably, the unsubstituted arylene groups of the substituted or unsubstituted arylene groups having 6 to 50 ring carbon atoms represented by L³ and L⁴ each are independently selected from a phenylene group, a biphenylene group, a naphthylene group, a phenanthrylene group, and an anthrasenylene group.

Ar¹ and Ar² each are independently selected from a substituted or unsubstituted aryl group having 6 to 50 ring carbon atoms and a substituted or unsubstituted heterocyclic group having 5 to 50 ring atoms.

The details of the substituted or unsubstituted aryl group having 6 to 50 ring carbon atoms are the same as the details of the corresponding groups described above with respect to R.

It is preferable that the unsubstituted aryl groups of the substituted or unsubstituted aryl groups having 6 to 50 ring carbon atoms represented by Ar¹ and Ar² each are independently selected from a phenyl group, a biphenyl group, a naphthyl group, a phenanthryl group, an anthrasenyl group, a chrysenyl group, and a pyrenyl group.

The details of the substituted or unsubstituted heterocyclic group having 5 to 50 ring atoms are the same as the details of the corresponding groups described above with respect to R.

It is preferable that the unsubstituted heterocyclic groups of the substituted or unsubstituted heterocyclic groups having 5 to 50 ring atoms represented by Ar¹ and Ar² each are independently selected from a pyridyl group, a dibenzothiophenyl group, a dibenzofuranyl group, an azadibenzofuranyl group, a pyrimidinyl group, a quinolyl group, an isoquinolyl group, a benzothiophenyl group, and a benzofuranyl group.

L¹ and L² each are independently selected from a single bond or a substituted or unsubstituted arylene group having 6 to 50 ring carbon atoms.

As an embodiment of L¹ and L², it is preferable that one of L¹ and L² is a substituted or unsubstituted arylene group having 6 to 50 ring carbon atoms, and the other one is a single bond.

As another embodiment of L¹ and L², it is preferable that L¹ and L² each are independently a substituted or unsubstituted arylene group having 6 to 50 ring carbon atoms.

As yet another embodiment, it is preferable that L¹ and L² are single bonds.

The details of the substituted or unsubstituted arylene group having 6 to 50 carbon atoms are the same as the details of the arylene group described above with respect to L³ and L⁴.

It is preferable that the unsubstituted arylene groups of the substituted or unsubstituted arylene groups having 6 to 50 ring carbon atoms represented by L¹ and L² each are independently selected from a phenylene group, a biphenylene group, a naphthylene group, a phenanthrylene group, a fluorenylene group, and an anthrasenylene group.

Cz is a group represented by the following formula (1-a) or formula (1-b).

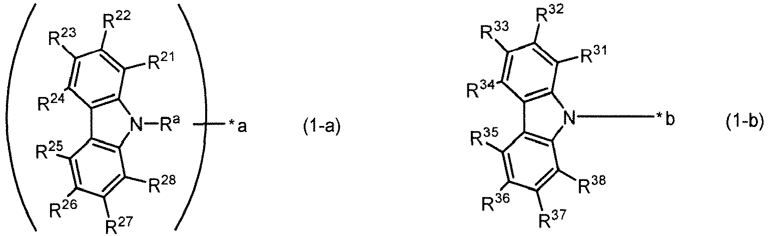

R²¹ to R²⁸ and R³¹ to R³⁸ each are independently a hydrogen atom or a substituent.

The substituent is selected from a substituted or unsubstituted aryl group having 6 to 50 ring carbon atoms, a substituted or unsubstituted heterocyclic group having 5 to 50 ring atoms but not having a 5-membered nitrogen-containing ring and a 7-membered nitrogen-containing ring, a substituted or unsubstituted alkyl group having 1 to 50 carbon atoms, a substituted or unsubstituted cycloalkyl group having 3 to 50 ring carbon atoms, a fluorine atom, and a cyano group. Preferably, the substituent is selected from a hydrogen atom, a substituted or unsubstituted aryl group having 6 to 50 ring carbon atoms, a substituted or unsubstituted heterocyclic group having 5 to 50 ring atoms but not having a 5-membered nitrogen-containing ring and a 7-membered nitrogen-containing ring, a substituted or unsubstituted alkyl group having 1 to 50 carbon atoms, and a cyano group, and more preferably, the substituent is a hydrogen atom.

However, adjacent two selected from R²¹ to R²⁸, adjacent two selected from R³¹ to R³⁸, R²⁴ and R²⁵, and R³⁴ and R³⁵ are not bonded to each other to form a ring.

Moreover, one selected from R²¹ to R²⁸ is a single bond bonded to L³ via *a, and *b represents a position bonded to L³.

The details of the substituted or unsubstituted aryl group having 6 to 50 ring carbon atoms, the substituted or unsubstituted alkyl group having 1 to 50 carbon atoms, and the substituted or unsubstituted cycloalkyl group having 3 to 50 ring carbon atoms each are the same as the details of the corresponding groups described above with respect to R.

Further, the details of the substituted or unsubstituted heterocyclic group having 5 to 50 ring atoms but not having a 5-membered nitrogen-containing ring and a 7-membered nitrogen-containing ring can be found in the details of the heterocyclic group described above with respect to R, which is a heterocyclic group excluding heterocyclic groups having a 5-membered nitrogen-containing ring and a 7-membered nitrogen-containing ring.

As an embodiment of the present invention, it is preferable that at least one selected from R²¹ to R²⁸ that is not a single bond bonded to L³ via *a, or at least one selected from R³¹ to R³⁸ is a substituent.

It is preferable that at least one of the substituents represented by R²¹ to R²⁸ that is not a single bond bonded to L³ via *a and R³¹ to R³⁸ is selected from a substituted or unsubstituted aryl group having 6 to 50 ring carbon atoms and a substituted or unsubstituted alkyl group having 1 to 50 carbon atoms.

In the substituents represented by R²¹ to R²⁸ that is not a single bond bonded to L³ via *a and R³¹ to R³⁸, it is preferable that at least one of the unsubstituted substituents is selected from a methyl group, an ethyl group, an n-propyl group, an isopropyl group, an n-butyl group, an isobutyl group, an s-butyl group, a t-butyl group, a phenyl group, a biphenyl group, a naphthyl group, a phenanthryl group, and a cyano group.

R^{a} is selected from a substituted or unsubstituted aryl group having 6 to 50 ring carbon atoms and a substituted or unsubstituted alkyl group having 1 to 50 carbon atoms.

The details of the substituted or unsubstituted aryl group having 6 to 50 ring carbon atoms and the substituted or unsubstituted alkyl group having 1 to 50 carbon atoms each are the same as the details of the corresponding groups described above with respect to R.

It is preferable that the unsubstituted aryl group of the substituted or unsubstituted aryl group having 6 to 50 ring carbon atoms represented by R^{a} is selected from a phenyl group, a biphenyl group, a naphthyl group, and a phenanthryl group.

It is preferable that the unsubstituted alkyl group of the substituted or unsubstituted alkyl group having 1 to 50 ring carbon atoms represented by R^{a} is selected from a methyl group, an ethyl group, an n-propyl group, an isopropyl group, an n-butyl group, an isobutyl group, an s-butyl group, and a t-butyl group.

In a preferred embodiment of the present invention, the inventive compound includes a compound represented by the formula (1-b-1) or (1-b-2).

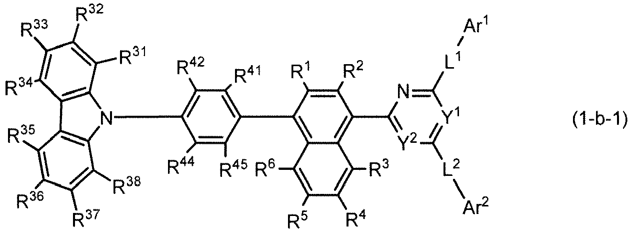

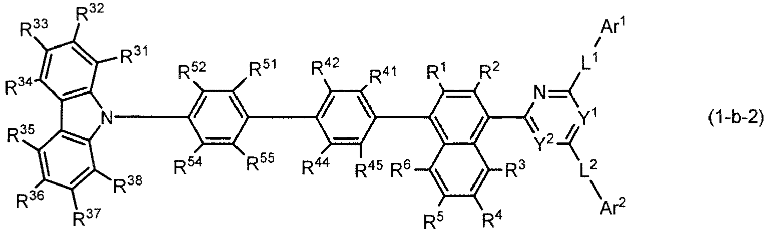

Y¹, Y², Ar¹, Ar², L¹, L², R¹ to R⁶ and R³¹ to R³⁸ are defined above, and R⁴¹ to R⁴², R⁴⁴ to R⁴⁵, R⁵¹ to R⁵², and R⁵⁴ to R⁵⁵ each are independently selected from a hydrogen atom, a substituted or unsubstituted aryl group having 6 to 50 ring carbon atoms, and a substituted or unsubstituted alkyl group having 1 to 50 carbon atoms.

The details of the substituted or unsubstituted aryl group having 6 to 50 ring carbon atoms and the substituted or unsubstituted alkyl group having 1 to 50 carbon atoms represented by R⁴¹ to R¹², R⁴⁴ to R⁴⁵, R⁵¹ to R⁵², and R⁵⁴ to R⁵⁵ each are the same as the details of the corresponding groups described above with respect to R.

At least one set of adjacent two selected from R⁴¹ to R⁴², R⁴⁴ to R⁴⁵, R⁵¹ to R⁵² and R⁵⁴ to R⁵⁵, that is, at least one set of adjacent two selected from R⁴¹ and R⁴², R⁴⁴ and R⁴⁵, R⁵¹ and R⁵², and R⁵⁴ and R⁵⁵ may each be independently bonded to each other to form a substituted or unsubstituted benzene ring, or may not be bonded to each other and thus may not form the benzene ring. In an embodiment of the present invention, it is preferable not to form the benzene ring. In another embodiment of the present invention, it is preferable to form the benzene ring.

In a preferred embodiment of the present invention, the inventive compound includes a compound represented by any of formulae (1-b-11) to (1-b-14).

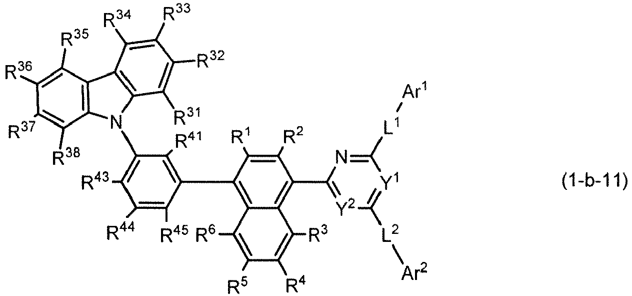

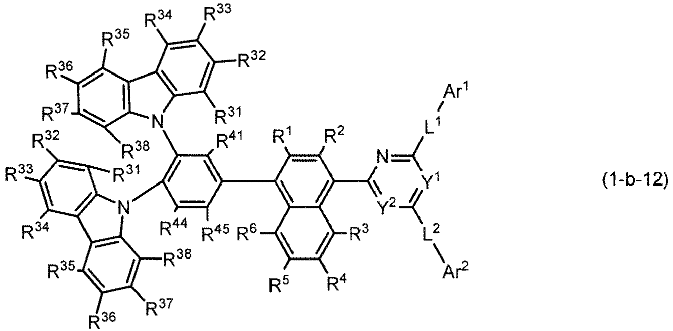

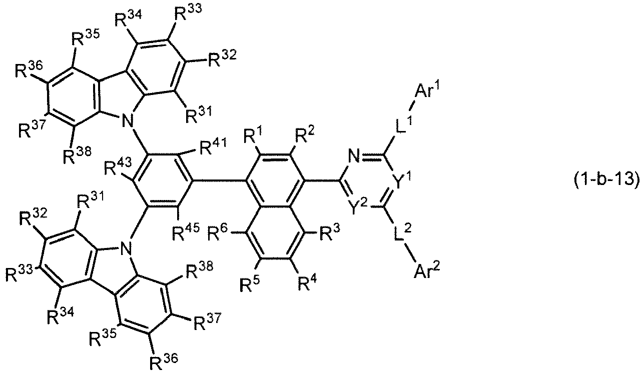

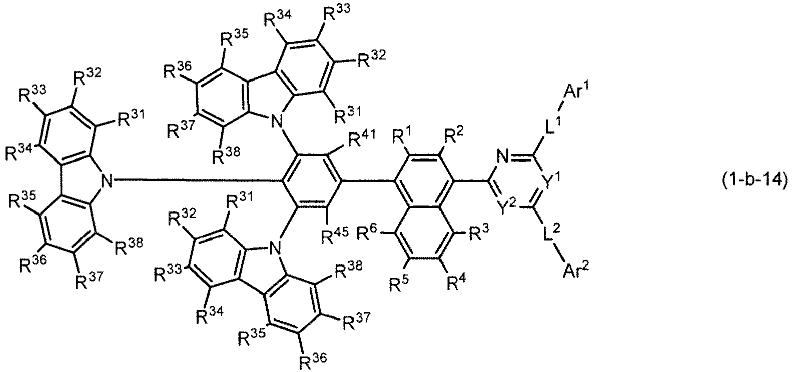

Y¹, Y², Ar¹, Ar², L¹, L², R¹ to R⁶, R³¹ to R³⁸, R⁴¹ and R⁴⁴ to R⁴⁵ each are defined above. R⁴³ is selected from a hydrogen atom, a substituted or unsubstituted aryl group having 6 to 50 ring carbon atoms, and a substituted or unsubstituted alkyl group having 1 to 50 carbon atoms.

The details of the substituted or unsubstituted aryl group having 6 to 50 ring carbon atoms and the substituted or unsubstituted alkyl group having 1 to 50 carbon atoms represented by R⁴³ are the same as the details of the corresponding groups described above with respect to R.

In a preferred embodiment of the present invention, the inventive compound includes a compound represented by any of formulae (1-a-1) to (1-a-4).

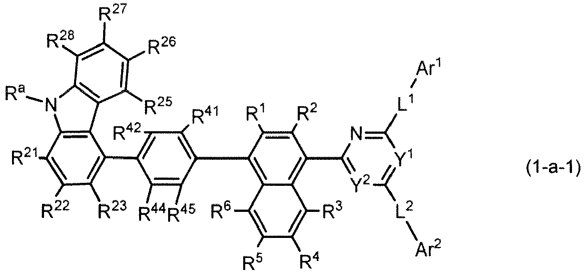

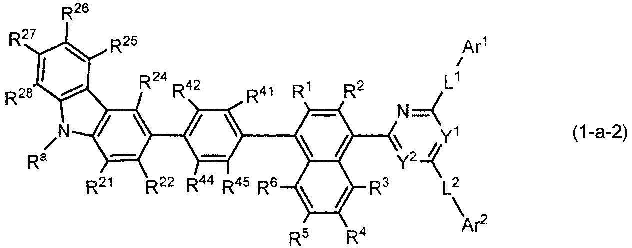

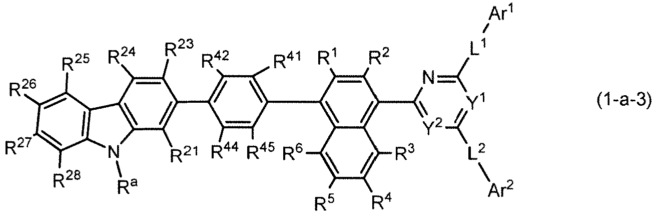

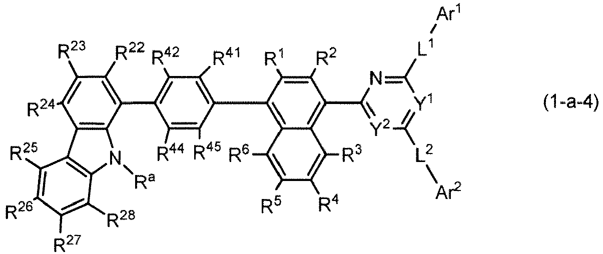

Y¹, Y², Ar¹, Ar², L¹, L², R^{a}, R¹ to R⁶, R²¹ to R²⁸, R⁴¹ to R⁴², and R⁴⁴ to R⁴⁵ are defined above.

In one embodiment of the present invention,
(1) R¹ to R⁶ may all be hydrogen atoms,
(2) R⁴¹ to R⁴⁵ may all be hydrogen atoms,
(3) R⁵¹ to R⁵² and R⁵⁴ to R⁵⁵ may all be hydrogen atoms,
(4) all except the substituents selected from R²¹ to R²⁸ that are not single bonds bonded to L³ via *a may be hydrogen atoms,
(5) all except the substituents selected from R³¹ to R³⁸ may be hydrogen atoms.

The inventive compound may satisfy two or more of the above conditions (1) to (5) simultaneously depending on the compositions thereof.

As described above, the "hydrogen atom" used in the description herein includes a protium atom, a deuterium atom, and a tritium atom. Accordingly, the inventive compound may contain a naturally-derived deuterium atom.

Further, a deuterium atom may be intentionally introduced into the inventive compound by using a deuterated compound as a part or all of the raw material compound. Accordingly, in one embodiment of the present invention, the inventive compound contains at least one deuterium atom. That is, the inventive compound may be a compound represented by the formula (1) or the aforementioned formula included in the formula (1) in which at least one hydrogen atom contained in the compound is a deuterium atom.

In the formula (1), at least one hydrogen atom selected from the following hydrogen atoms may be a deuterium atom: a hydrogen atom represented by R, R¹ to R⁶, R²¹ to R²⁸ that is not a single bond bonded to *a, R³¹ to R³⁸, R⁴¹ to R⁴⁵, R⁵¹ to R⁵², and R⁵⁴ to R⁵⁵; a hydrogen atom of a substituent represented by R, R¹ to R⁶, R²¹ to R²⁸ that is not a single bond bonded to *a, R³¹ to R³⁸, R⁴¹ to R⁴⁵, R⁵¹ to R⁵², and R⁵⁴ to R⁵⁵; a hydrogen atom of an arylene group represented by L¹, L², L³, and L⁴; a hydrogen of an aryl group represented by R^{a}; and a hydrogen atom of an aryl group or a heterocyclic group represented by Ar¹ and Ar².

The deuteration rate of the inventive compound (the proportion of the number of deuterium atoms with respect to the number of all hydrogen atoms in the inventive compound) depends on the deuteration rate of the raw material compound used. The deuteration rate of the inventive compound is less than 100% since it is generally difficult to make the deuteration rates of all the raw material compounds used to 100%.

The deuteration rate of the inventive compound is 1% or more, preferably 3% or more, more preferably 5% or more, and further more preferably 10% or more.

The inventive compound may be a mixture of a deuterated compound (a compound having deuterium atoms intentionally introduced thereto) and a non-deuterated compound, or a mixture of two or more compounds having different deuteration rates from each other. The deuteration rate of the mixture (the proportion of the number of deuterium atoms with respect to the number of all hydrogen atoms in the inventive compound contained in the mixture) is 1% or more, preferably 3% or more, more preferably 5% or more, and still more preferably 10% or more, and is less than 100%.

In the inventive compound, at least one hydrogen atom selected from a hydrogen atom represented by R or a hydrogen atom of a substituent represented by R may be a deuterium atom. The deuteration rate (the proportion of the number of deuterium atoms with respect to the number of hydrogen atoms represented by R, or the number of all hydrogen atoms of the substituent represented by R) is 1% or more, preferably 3% or more, more preferably 5% or more, and still more preferably 10% or more, and is less than 100%.

In the inventive compound, at least one hydrogen atom selected from a hydrogen atom represented by R¹ to R⁶ or a hydrogen atom of the substituent represented by R¹ to R⁶ may be a deuterium atom. The deuteration rate (the proportion of the number of deuterium atoms with respect to the number of hydrogen atoms represented by R¹ to R⁶ or all hydrogen atoms of the substituent represented by R¹ to R⁶) is 1% or more, preferably 3% or more, more preferably 5% or more, and still more preferably 10% or more, and is less than 100%.

In the inventive compound, at least one hydrogen atom selected from a hydrogen atom represented by R²¹ to R²⁸ that is not a single bond bonded to *a and a hydrogen atom of the substituent represented by R²¹ to R²⁸ that is not a single bond bonded to *a may be a deuterium atom. The deuteration rate (the proportion of the number of deuterium atoms with respect to the number of hydrogen atoms represented by R²¹ to R²⁸ that is not a single bond bonded to *a or all hydrogen atoms of the substituent represented by R²¹ to R²⁸ that is not a single bond bonded to *a) is 1% or more, preferably 3% or more, more preferably 5% or more, and still more preferably 10% or more, and is less than 100%.

In the inventive compound, at least one hydrogen atom selected from a hydrogen atom represented by R³¹ to R³⁸ and a hydrogen atom of the substituent represented by R³¹ to R³⁸ may be a deuterium atom. The deuteration rate (the proportion of the number of deuterium atoms with respect to the number of hydrogen atoms represented by R³¹ to R³⁸ or all hydrogen atoms of the substituent represented by R³¹ to R³⁸) is 1% or more, preferably 3% or more, more preferably 5% or more, and still more preferably 10% or more, and is less than 100%.

In the inventive compound, at least one hydrogen atom selected from a hydrogen atom of the arylene group represented by L¹ and L² may be a deuterium atom. The deuteration rate (the proportion of the number of deuterium atoms with respect to the number of all hydrogen atoms of the arylene group represented by L¹ and L²) is 1% or more, preferably 3% or more, more preferably 5% or more, and still more preferably 10% or more, and is less than 100%.

In the inventive compound, at least one hydrogen atom selected from a hydrogen atom of the arylene group represented by L³ and L⁴ may be a deuterium atom. The deuteration rate (the proportion of the number of deuterium atoms with respect to the number of all hydrogen atoms of the arylene group represented by L³ and L⁴) is 1% or more, preferably 3% or more, more preferably 5% or more, and still more preferably 10% or more, and is less than 100%.

In the inventive compound, at least one hydrogen atom selected from a hydrogen atom of the aryl group represented by R^{a} may be a deuterium atom. The deuteration rate (the proportion of the number of deuterium atoms with respect to the number of all hydrogen atoms of the aryl group represented by R^{a}) is 1% or more, preferably 3% or more, more preferably 5% or more, and still more preferably 10% or more, and is less than 100%.

In the inventive compound, at least one hydrogen atom selected from a hydrogen atom of the aryl group or the heterocyclic group represented by Ar¹ and Ar² may be a deuterium atom. The deuteration rate (the proportion of the number of deuterium atoms with respect to the number of all hydrogen atoms of the aryl group or the heterocyclic group represented by Ar¹ and Ar²) is 1% or more, preferably 3% or more, more preferably 5% or more, and still more preferably 10% or more, and is less than 100%.

The details of the substituent (arbitrary substituent) in the expression "substituted or unsubstituted" included in the definitions of the aforementioned formulae are the same as in the "substituent in the expression 'substituted or unsubstituted‴.

The inventive compound can be readily produced by a person skilled in the art with reference to the following synthesis examples and the known synthesis methods.

Specific examples of the inventive compound will be described below; however, the inventive compound is not limited to the following example compounds.

In the following specific examples, D represents a deuterium atom.

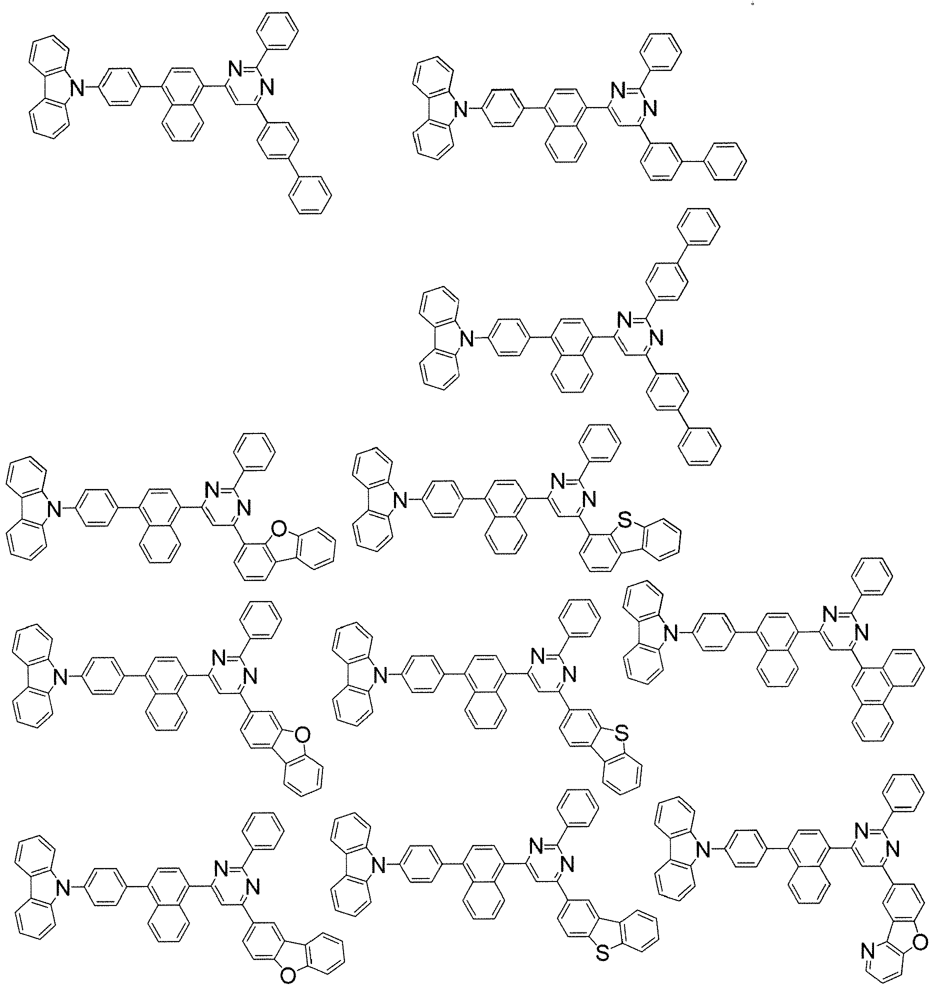

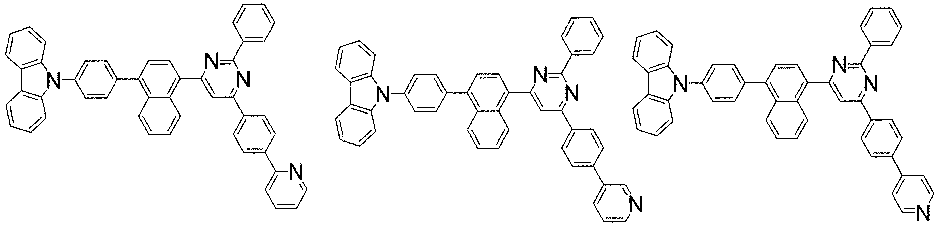

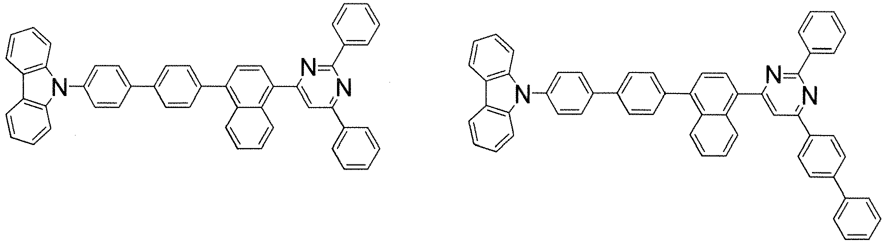

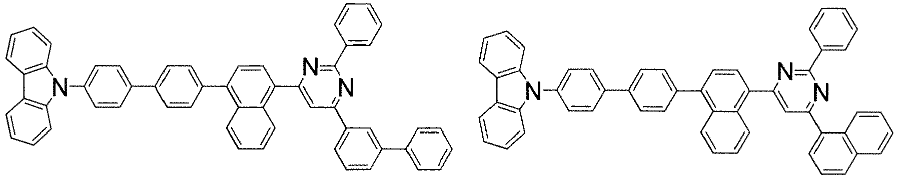

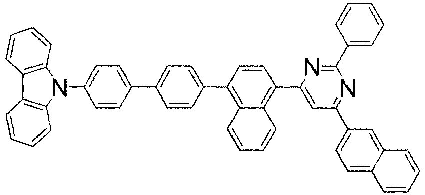

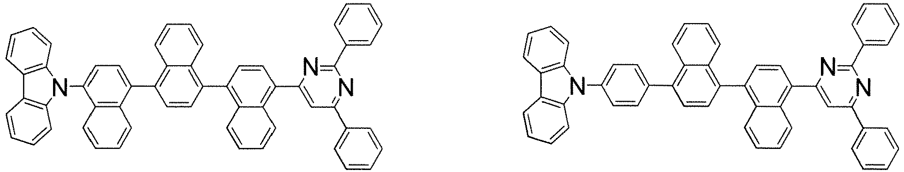

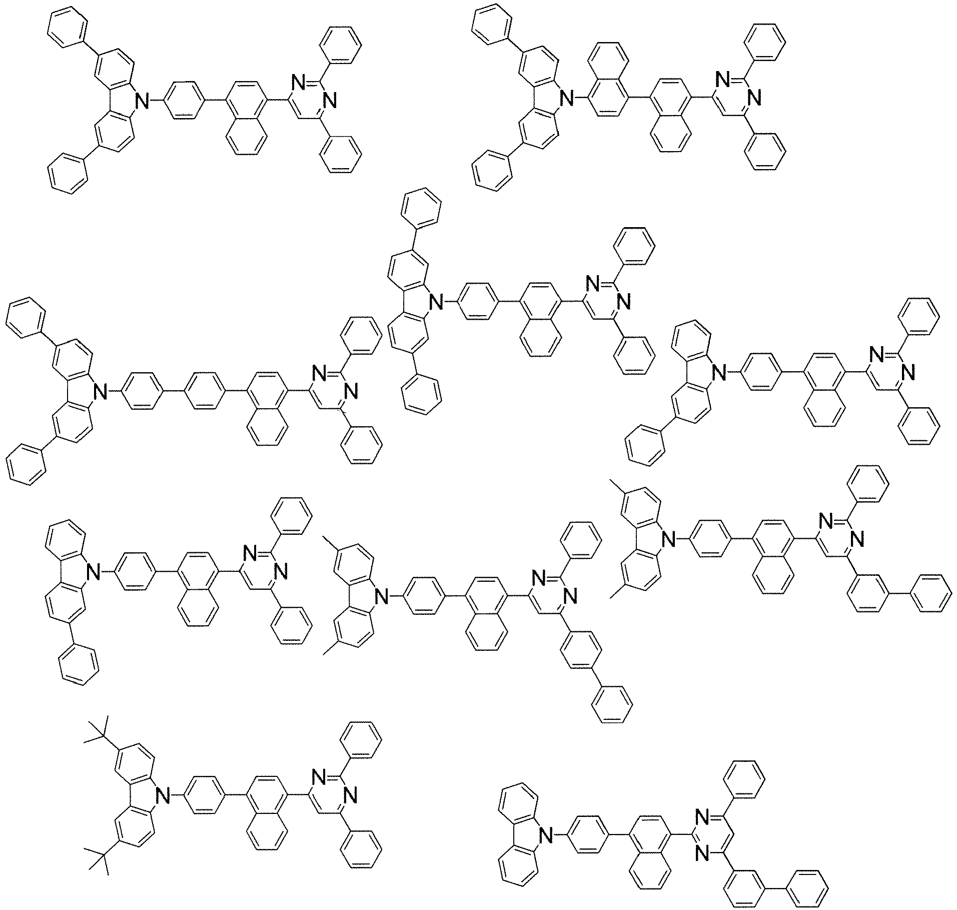

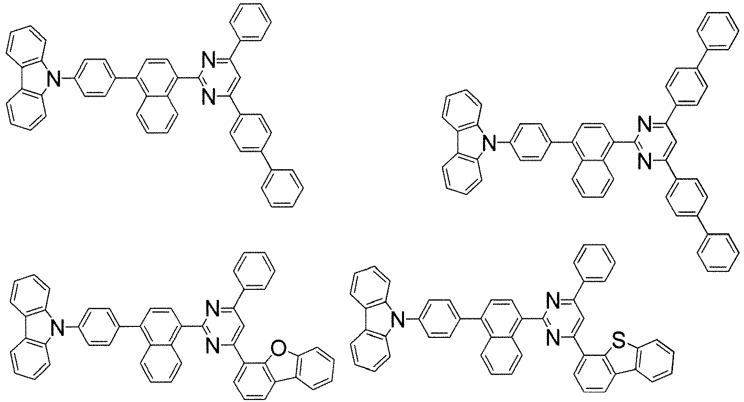

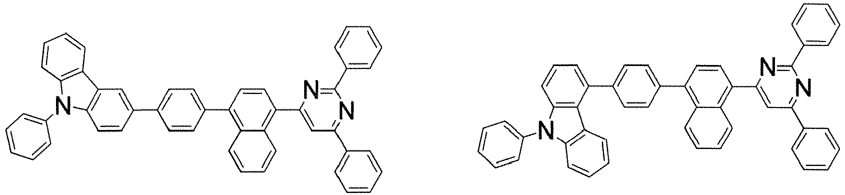

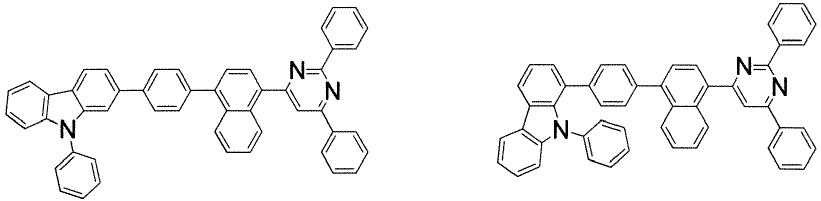

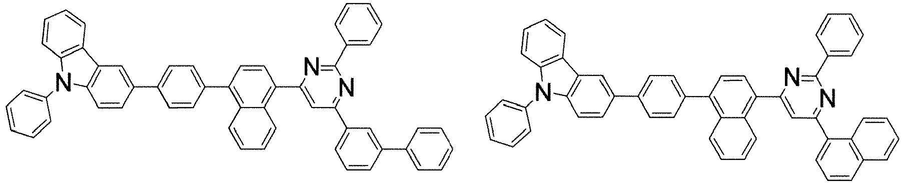

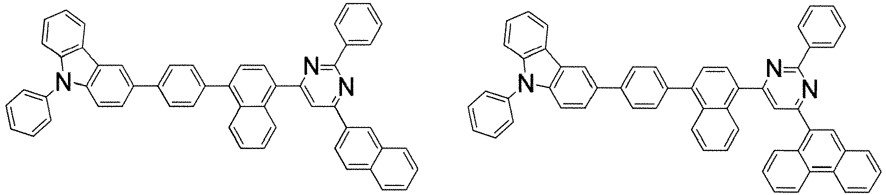

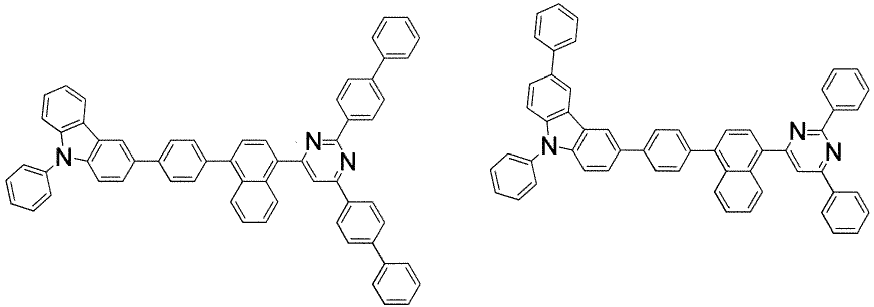

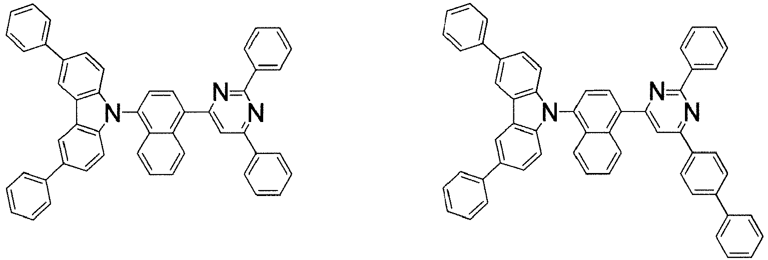

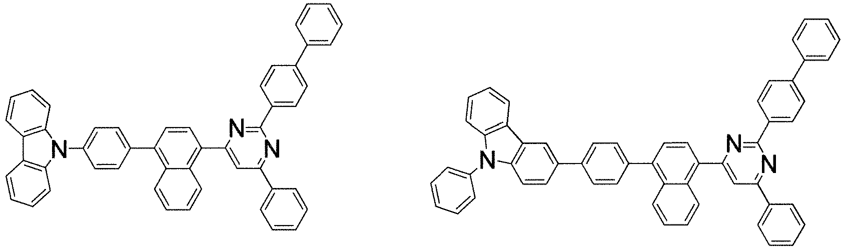

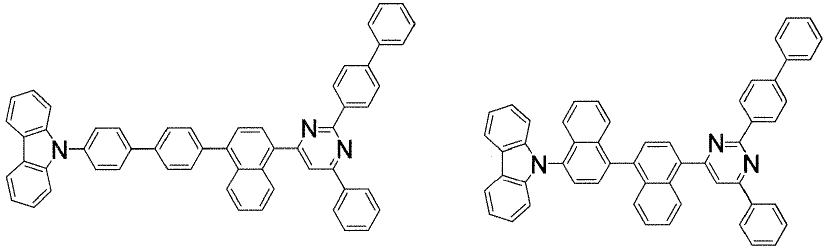

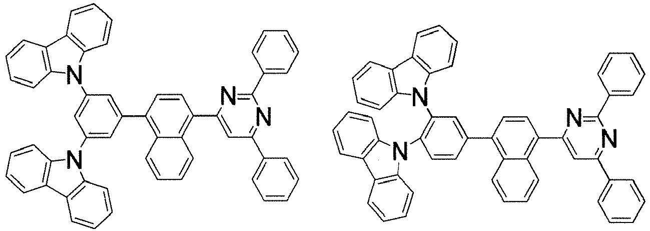

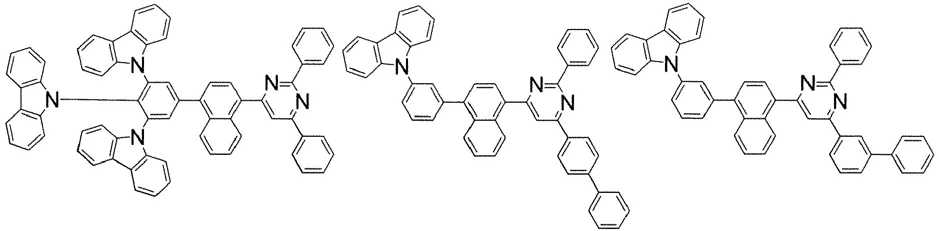

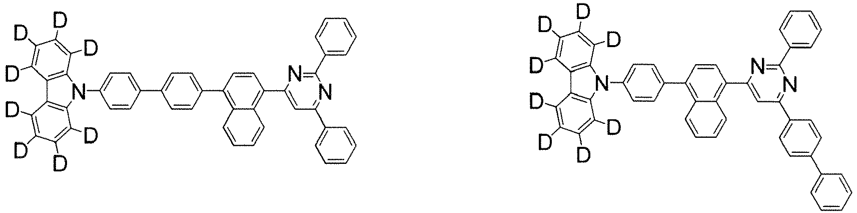

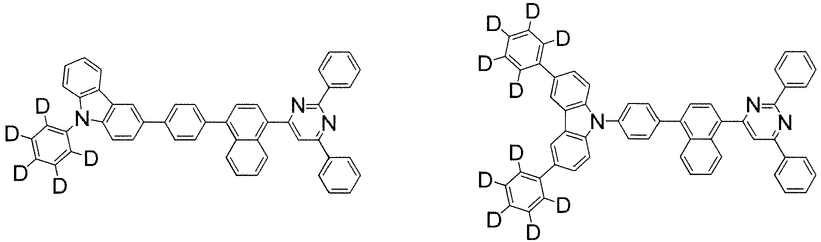

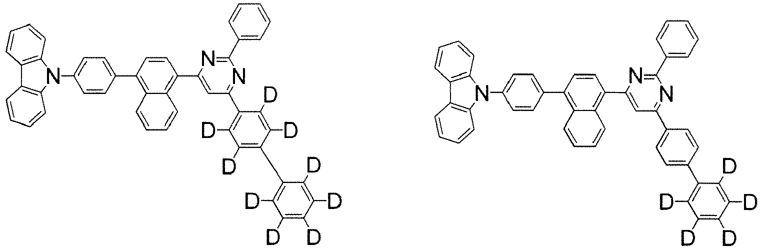

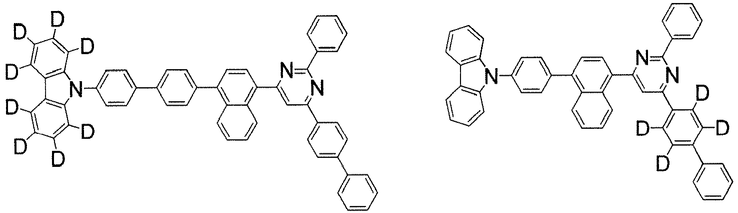

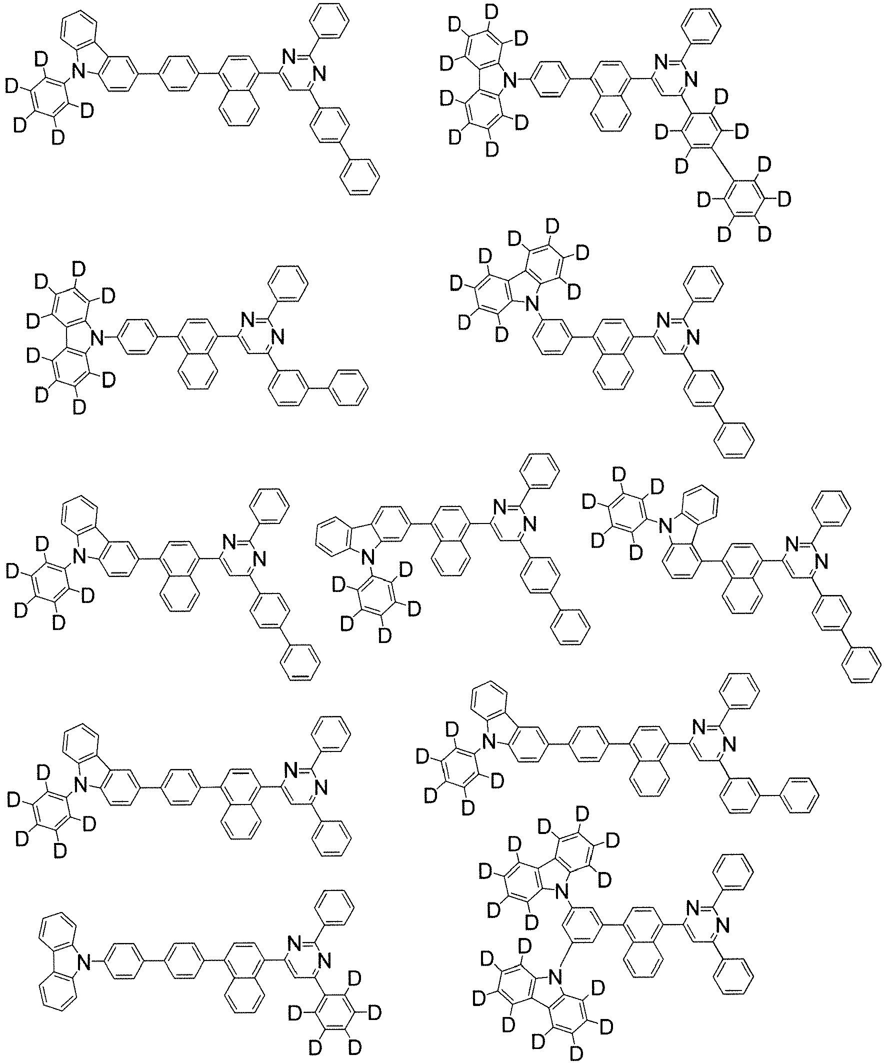

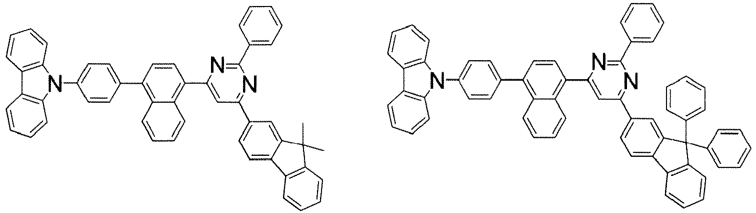

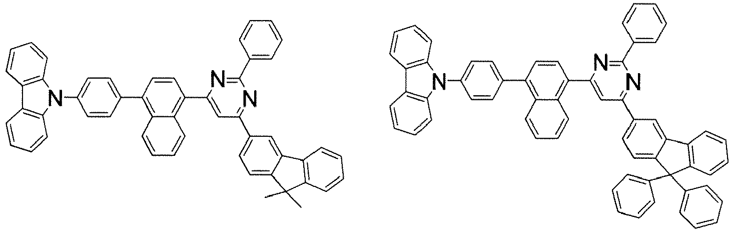

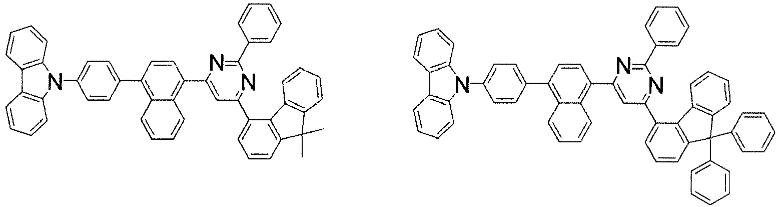

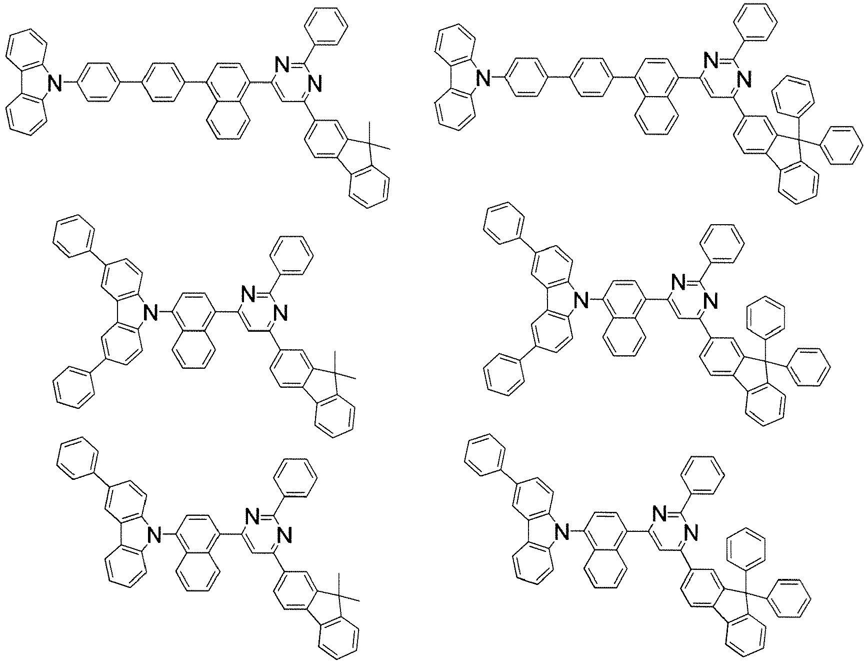

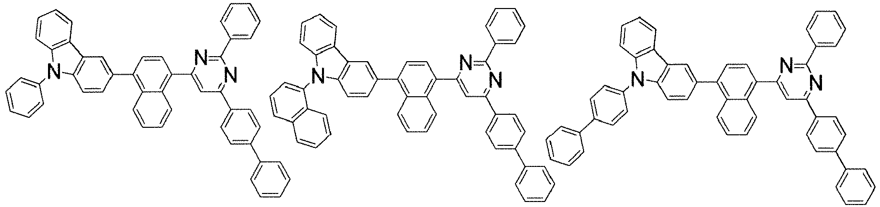

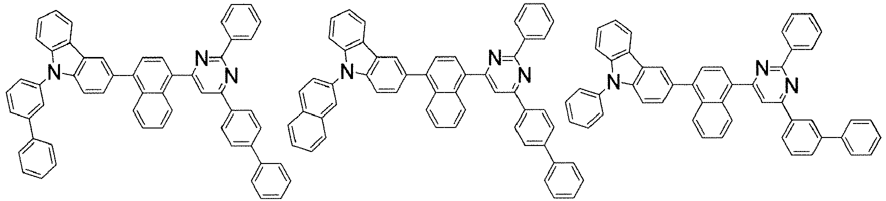

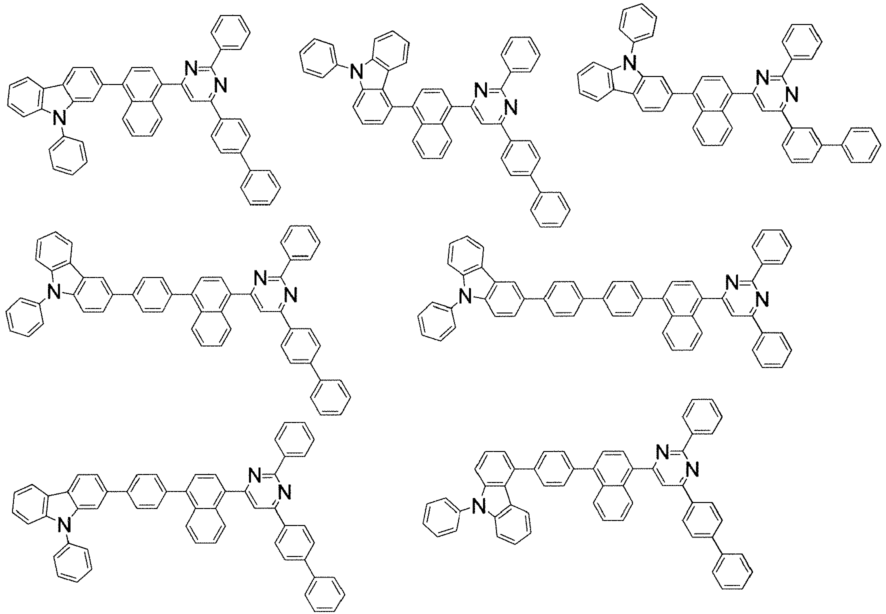

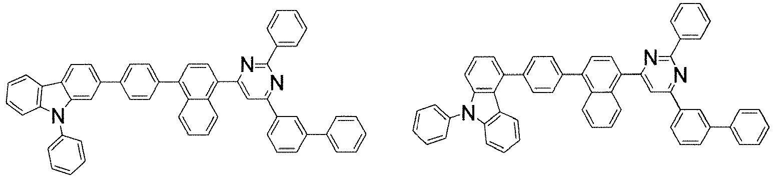

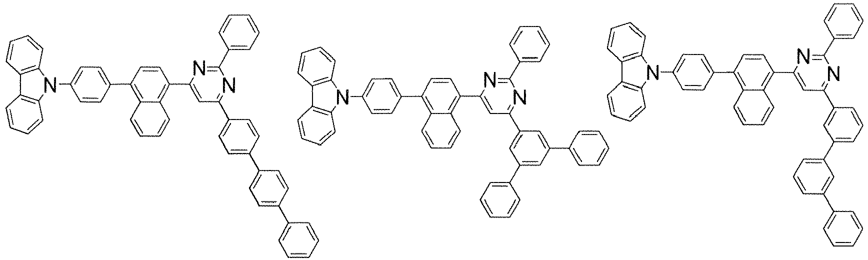

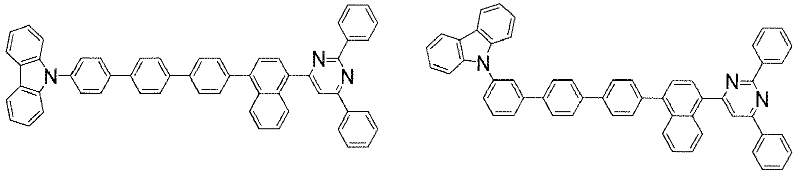

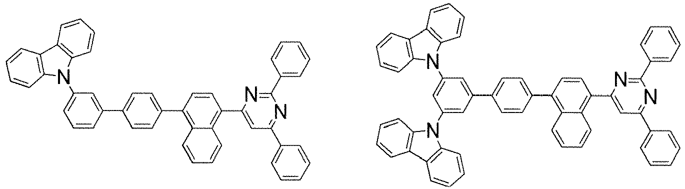

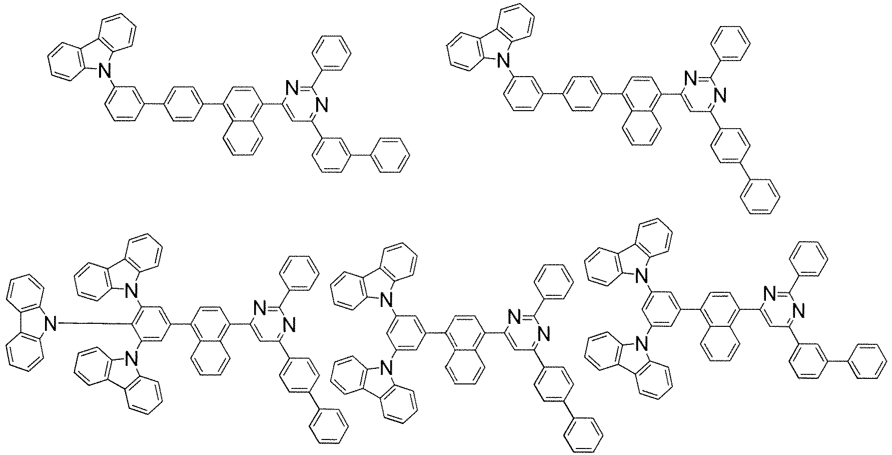

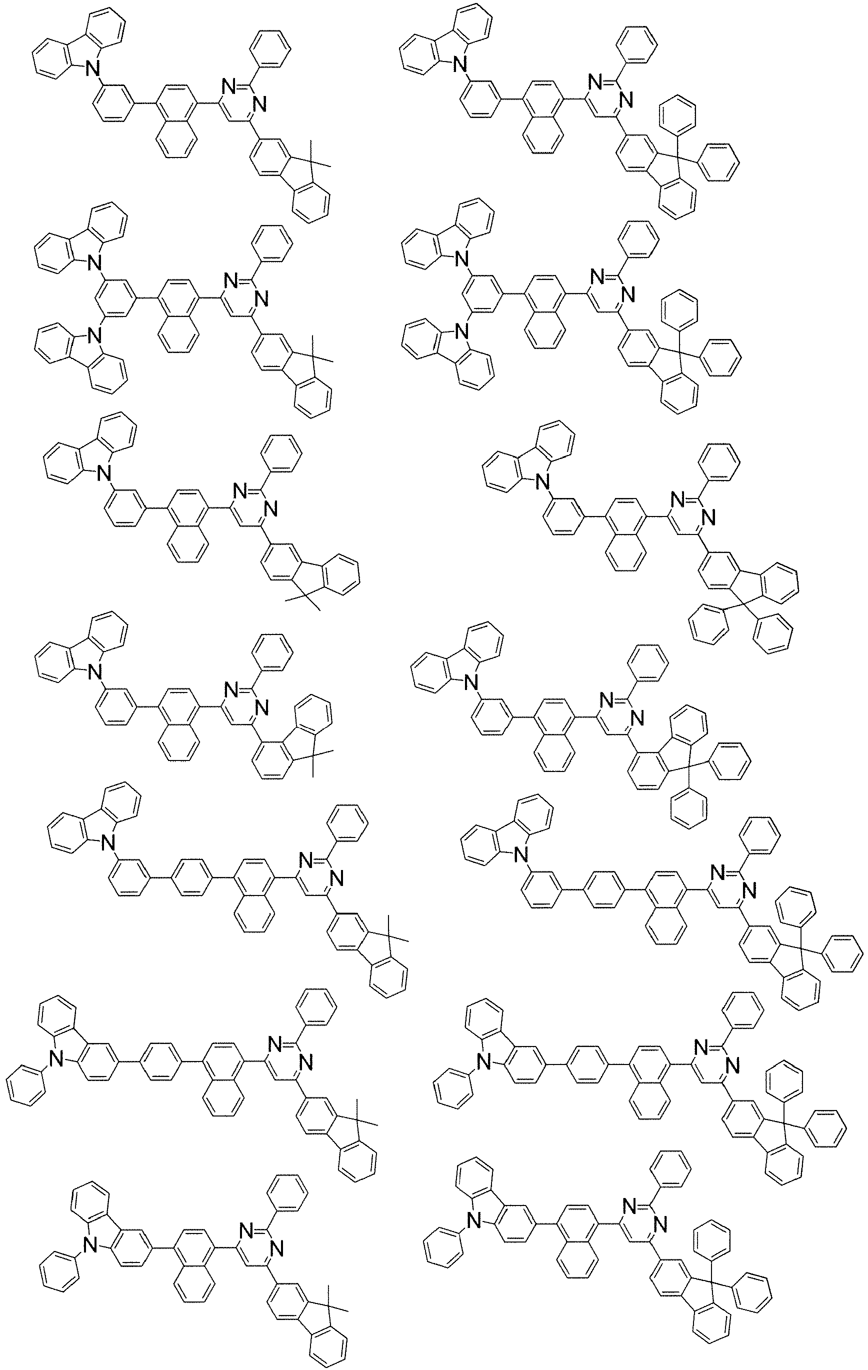

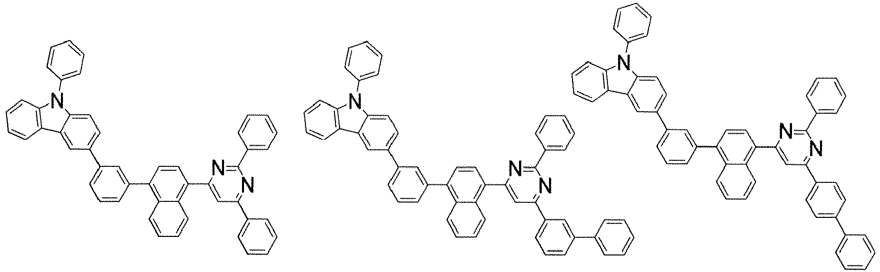

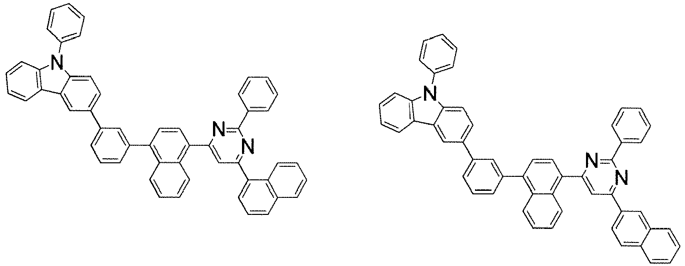

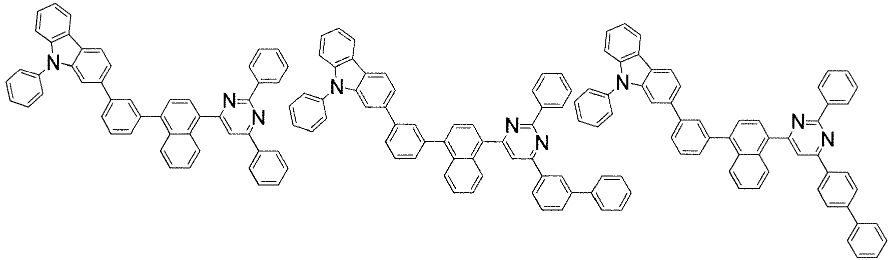

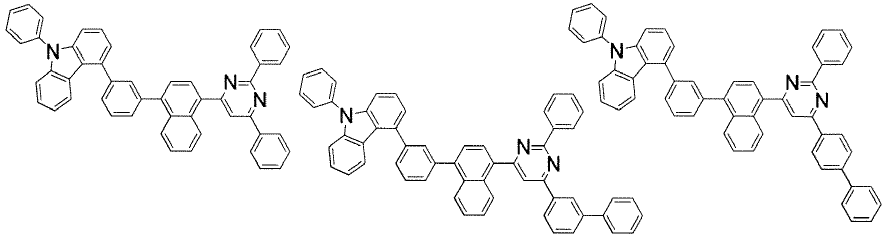

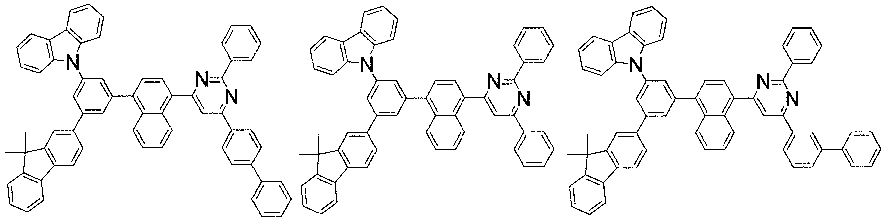

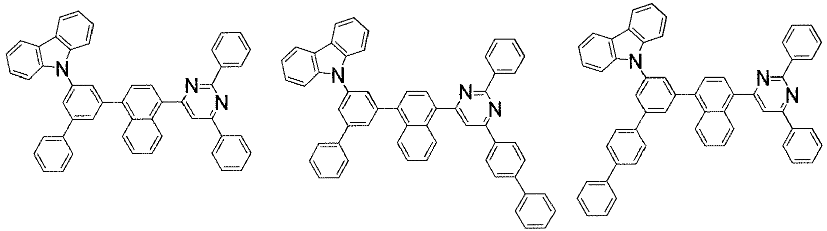

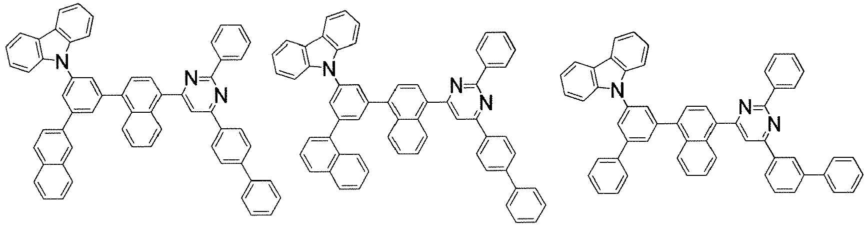

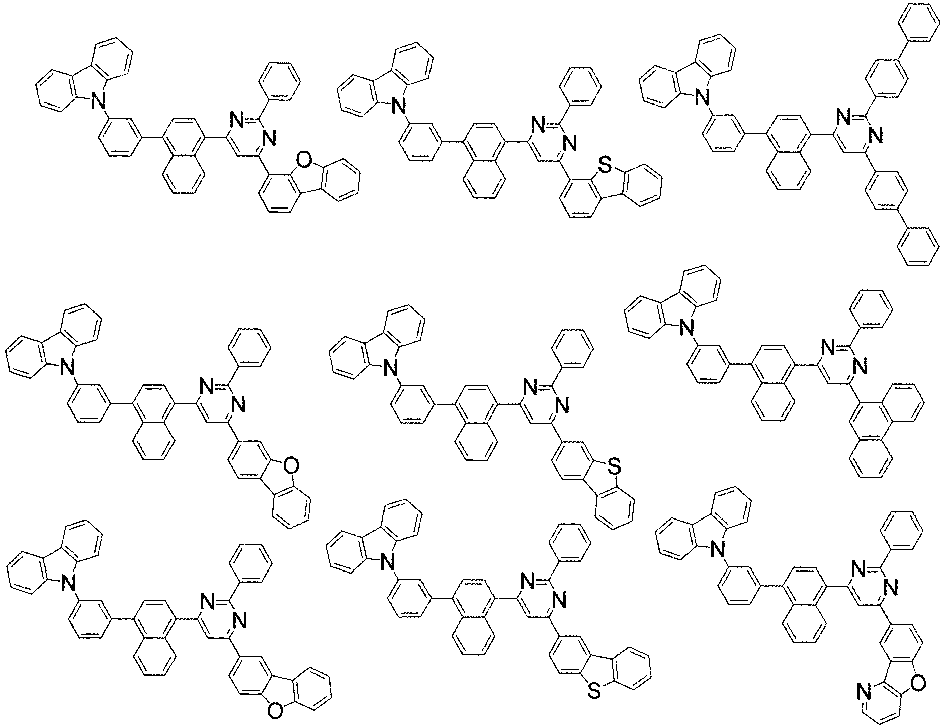

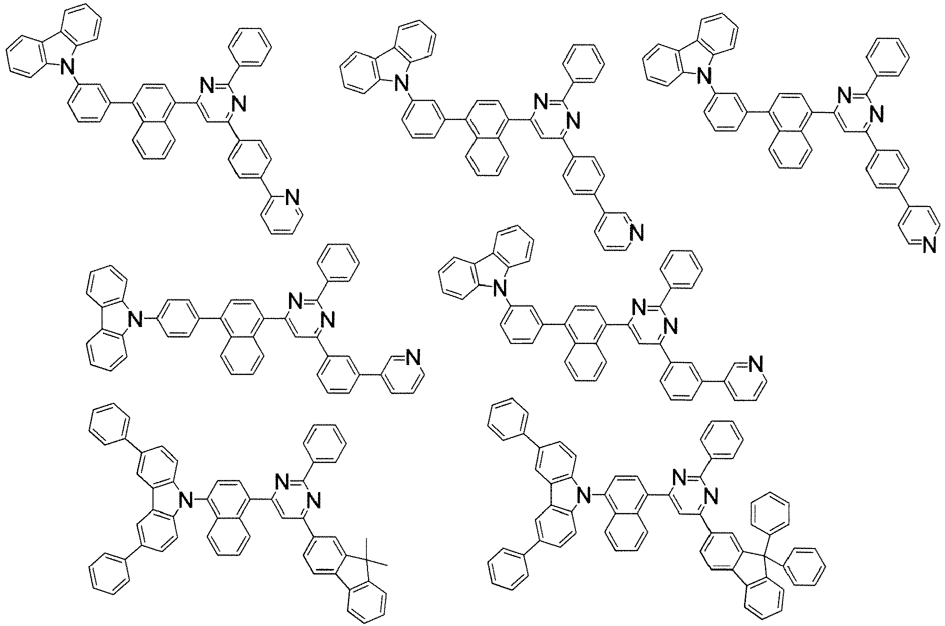

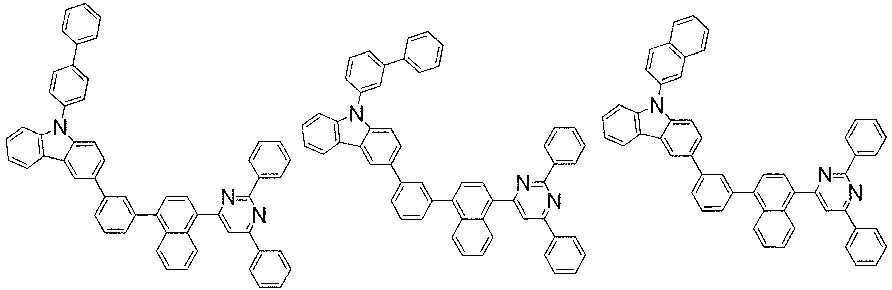

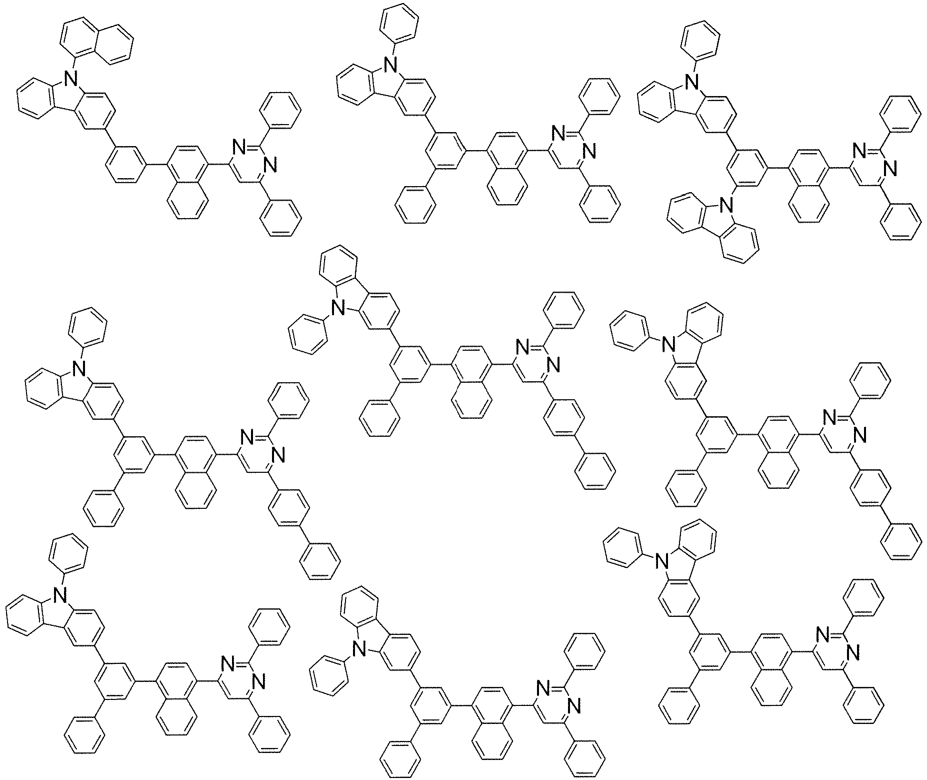

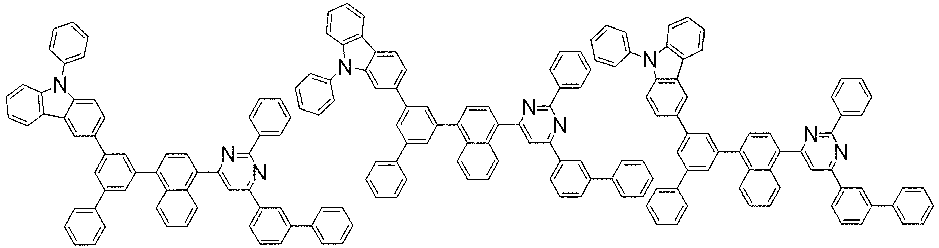

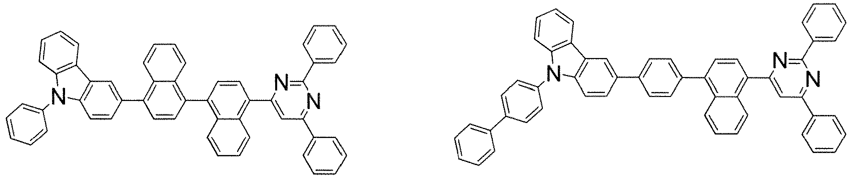

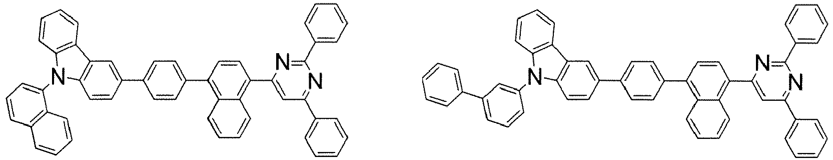

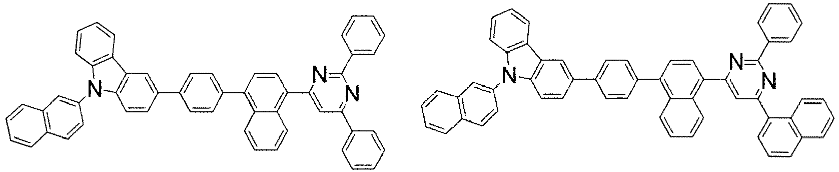

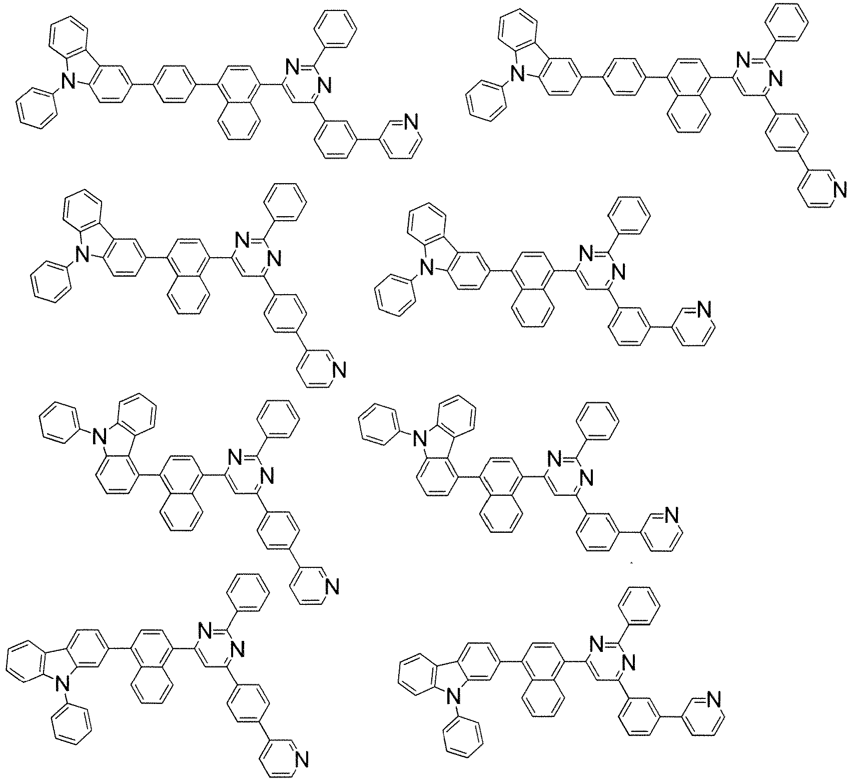

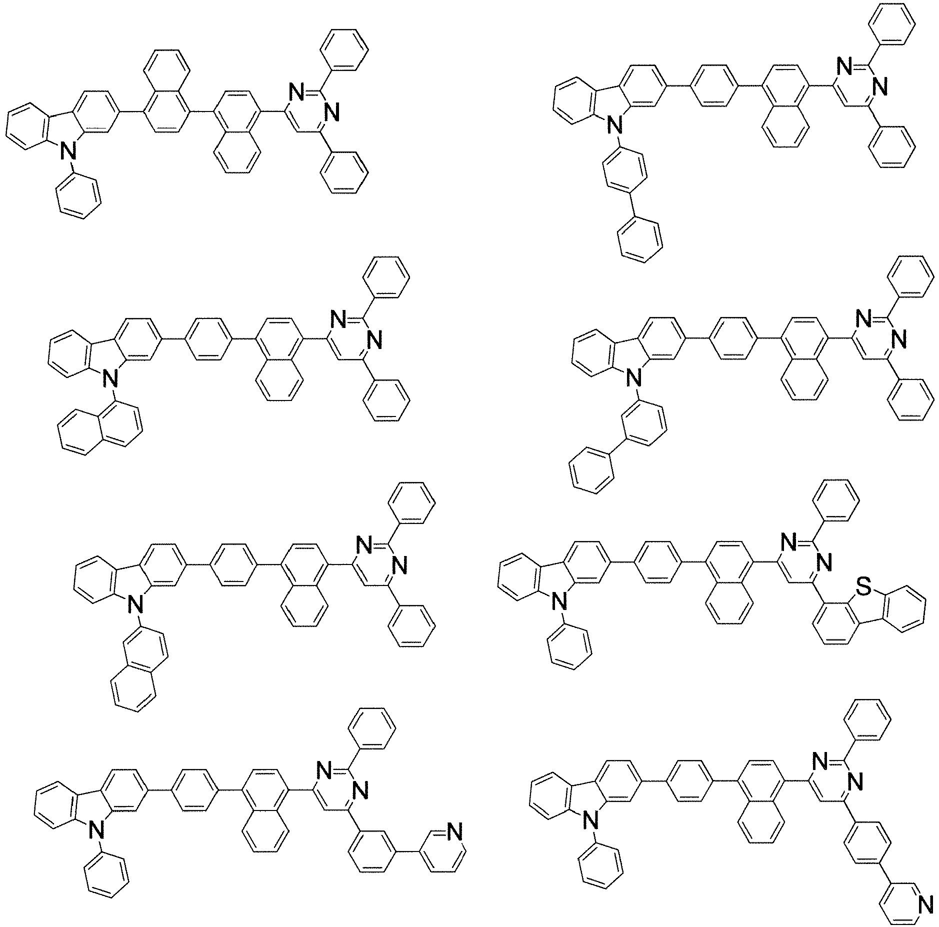

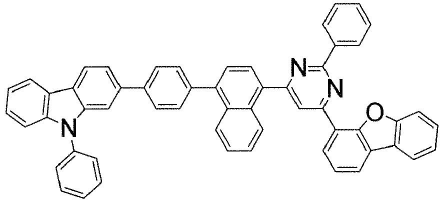

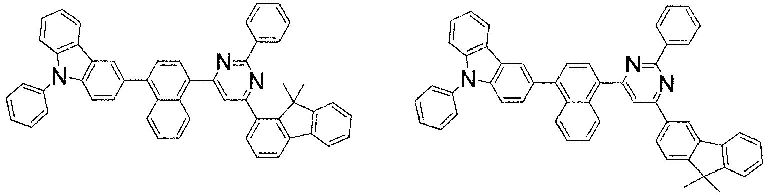

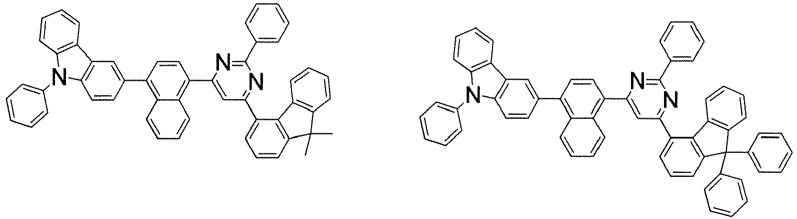

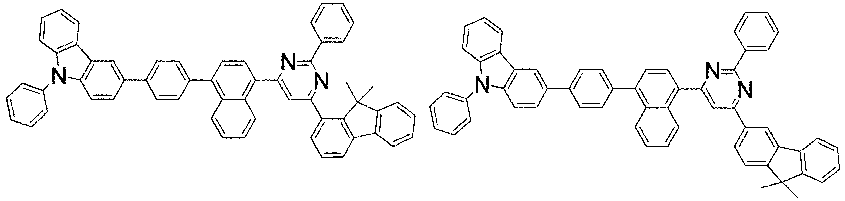

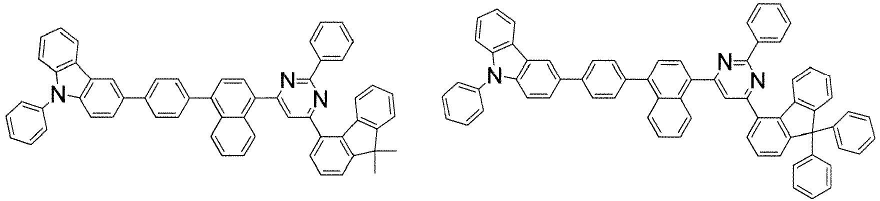

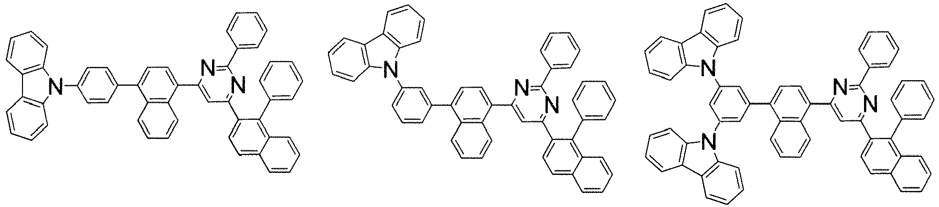

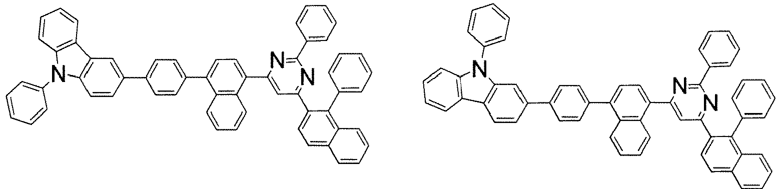

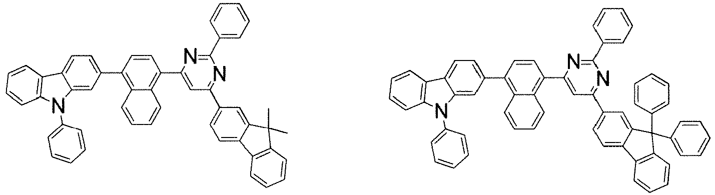

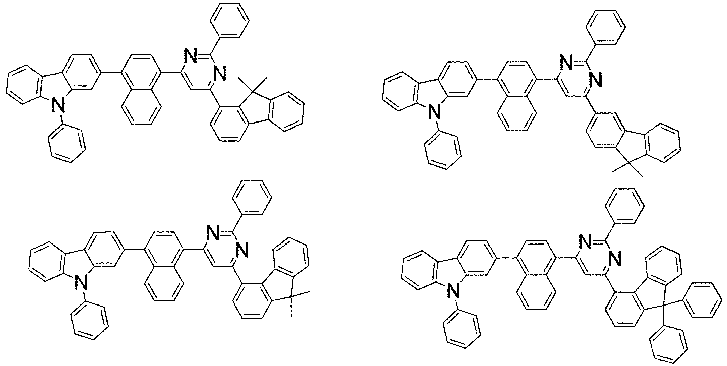

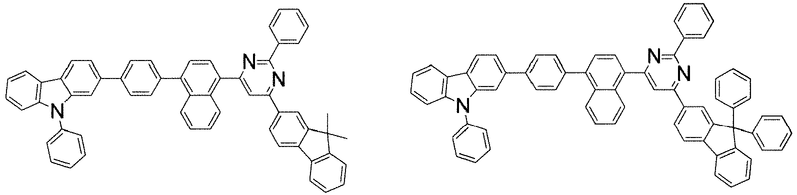

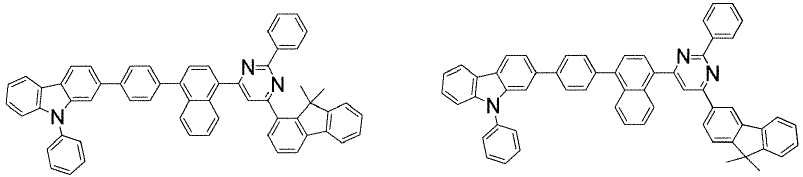

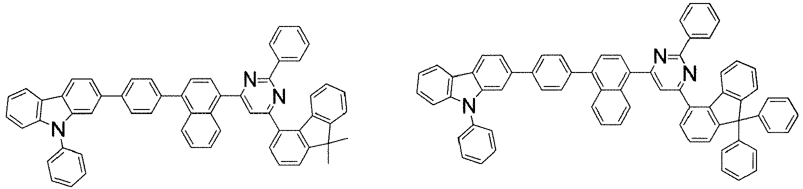

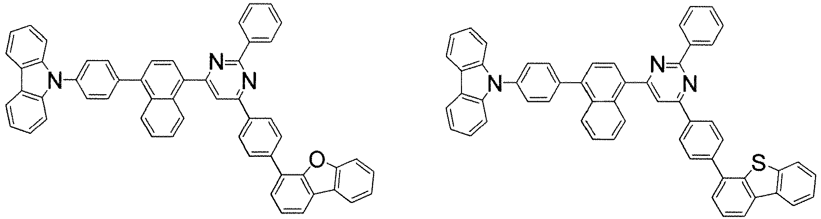

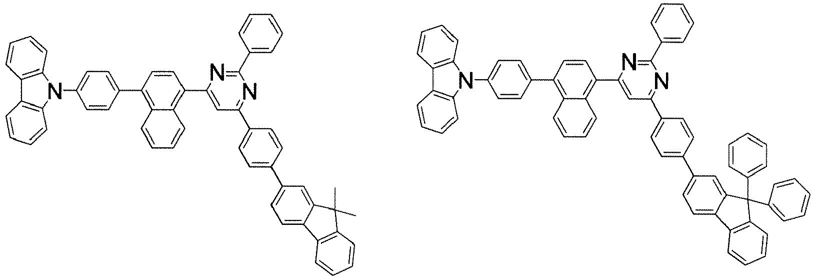

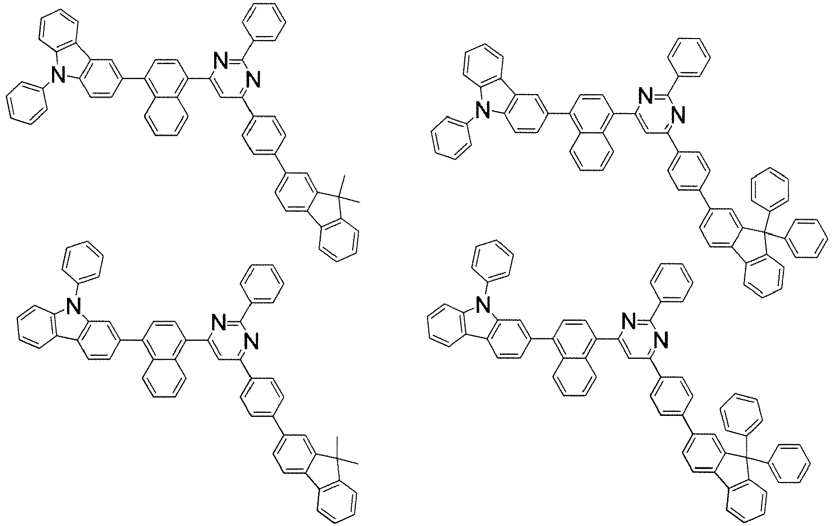

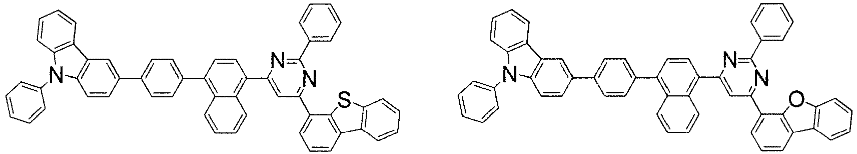

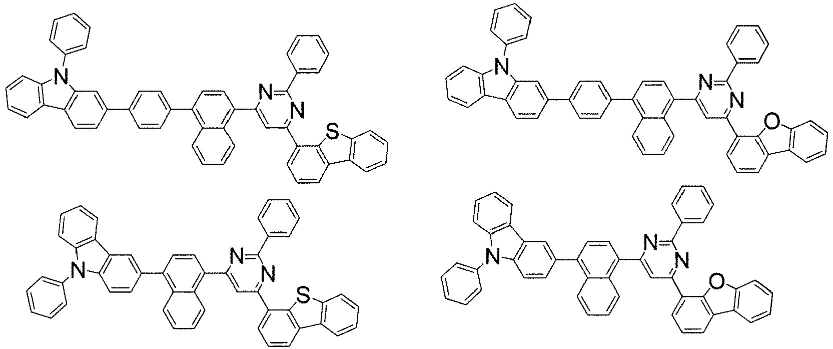

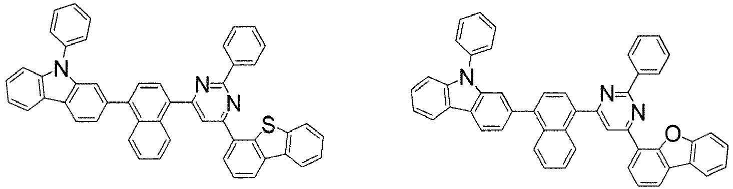

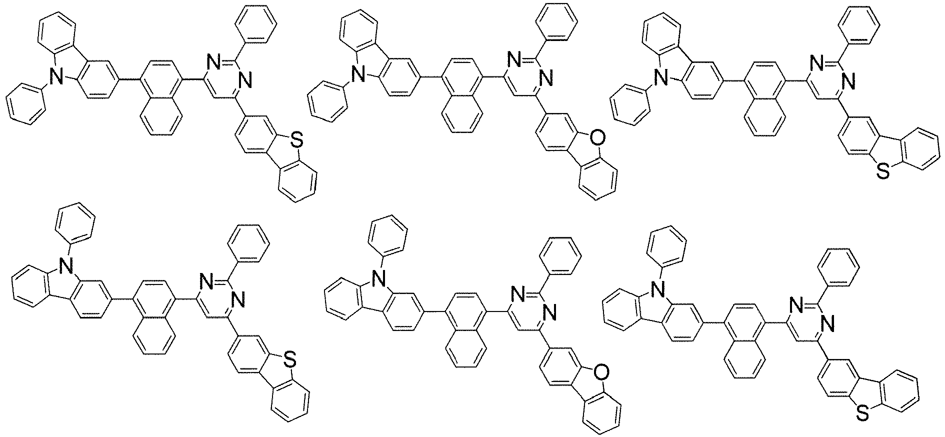

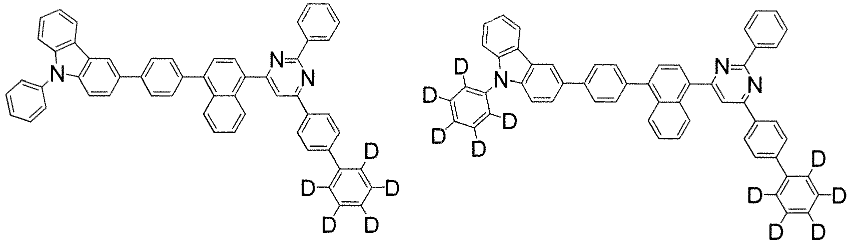

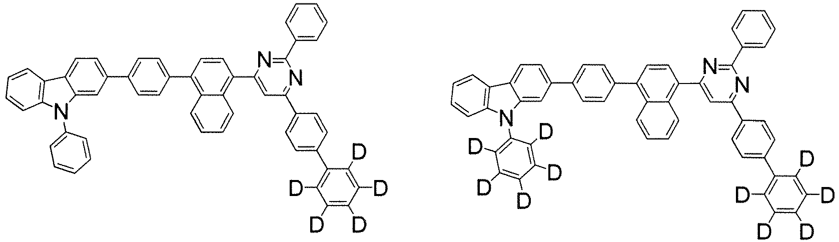

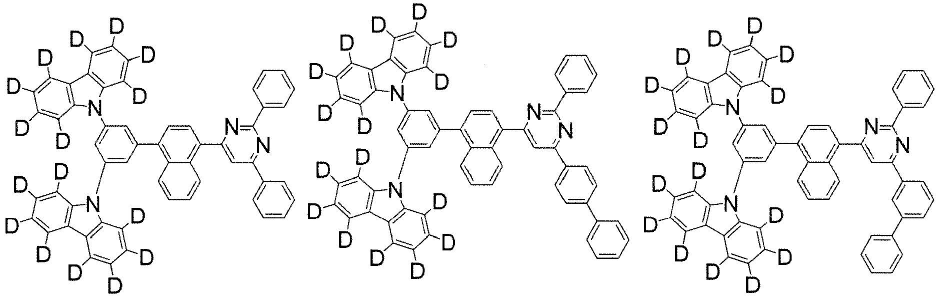

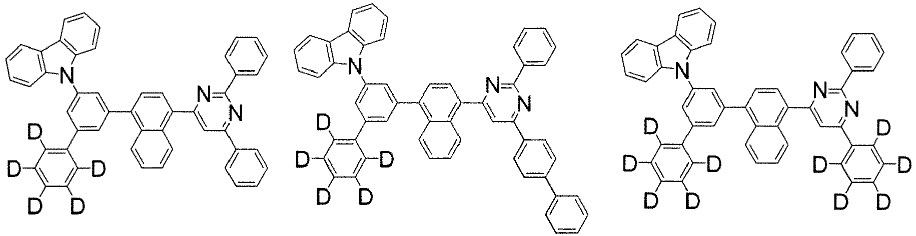

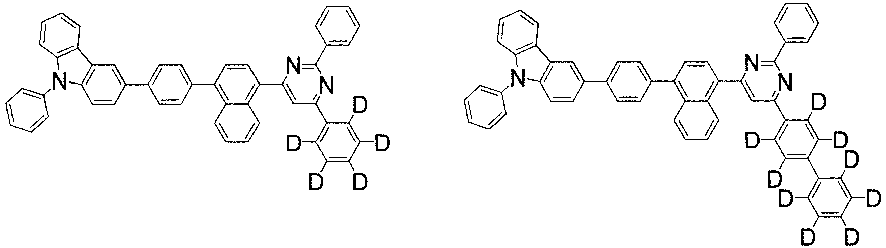

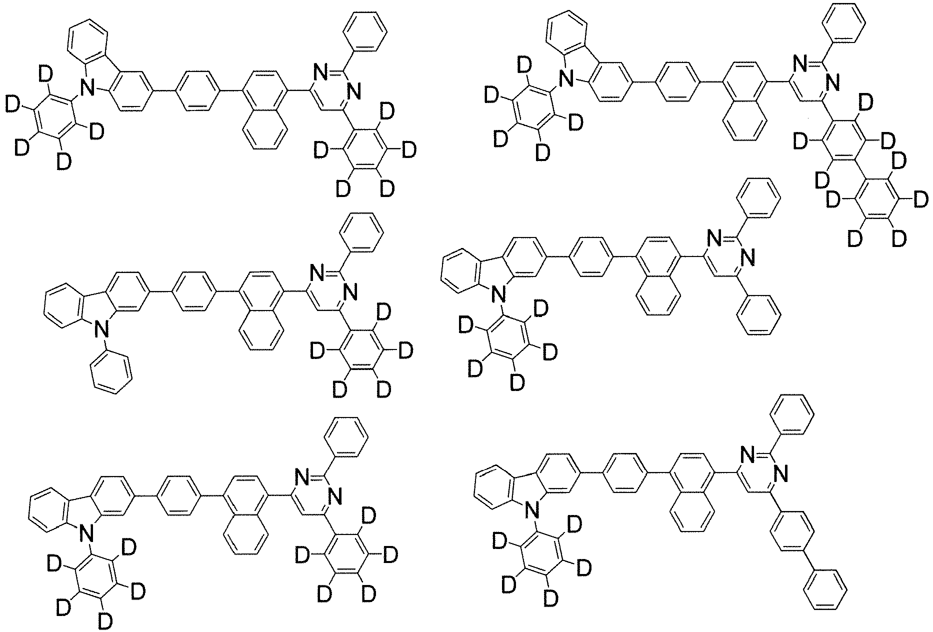

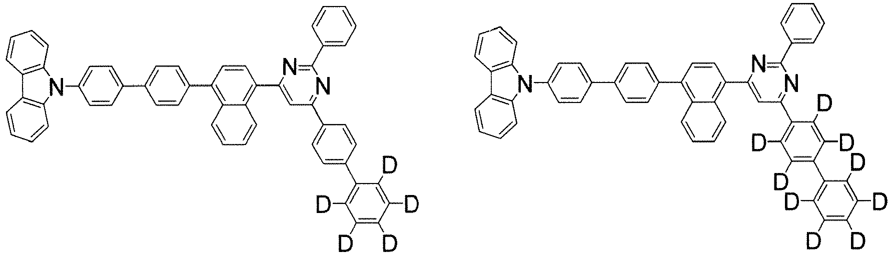

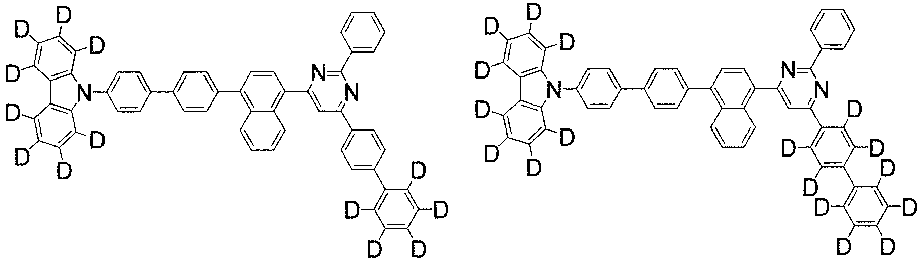

### Material for Organic EL Devices

The material for organic EL devices of the present invention contains the inventive compound. The content of the inventive compound in the material for organic EL devices is 1% by mass or more (including 100%), preferably 10% by mass or more (including 100%), more preferably 50% by mass or more (including 100%), further preferably 80% by mass or more (including 100%), still further preferably 90% by mass or more (including 100%), still more further preferably 95% by mass or more (including 100%), yet more further preferably 99% by mass of more (including 100%), and most preferably 99.9% by mass of more (including 100%). The material for organic EL devices of the present invention is useful for the production of an organic EL device.

### Organic EL Device

The organic EL device of the present invention includes an anode, a cathode, and organic layers intervening between the anode and the cathode. The organic layers include a light emitting layer, and at least one layer of the organic layers contains the inventive compound.

Examples of the organic layer containing the inventive compound include a hole transporting zone (such as a hole injecting layer, a hole transporting layer, an electron blocking layer, and an exciton blocking layer) intervening between the anode and the light emitting layer, the light emitting layer, a space layer, and an electron transporting zone (such as an electron injecting layer, an electron transporting layer, and a hole blocking layer) intervening between the cathode and the light emitting layer, but are not limited thereto. The inventive compound is preferably used as a material for the electron transporting zone or the light emitting layer in a fluorescent or phosphorescent EL device, more preferably as a material for the electron transporting zone, further preferably as a material for the electron injecting layer, the electron transporting layer, or the hole blocking layer, and particularly preferably as a material for the hole blocking layer.

The organic EL device of the present invention may be a fluorescent or phosphorescent light emission-type monochromatic light emitting device or a fluorescent/phosphorescent hybrid-type white light emitting device, and may be a simple type having a single light emitting unit or a tandem type having a plurality of light emitting units. Among them, the fluorescent light emission-type device is preferable. The "light emitting unit" referred to herein refers to a minimum unit that emits light through recombination of injected holes and electrons, which includes organic layers among which at least one layer is a light emitting layer.

For example, as a representative device configuration of the simple type organic EL device, the following device configuration may be exemplified.

### (1) Anode/Light Emitting Unit/Cathode

The light emitting unit may be a multilayer type having a plurality of phosphorescent light emitting layers or fluorescent light emitting layers. In this case, a space layer may intervene between the light emitting layers for the purpose of preventing excitons generated in the phosphorescent light emitting layer from diffusing into the fluorescent light emitting layer. Representative layer configurations of the simple type light emitting unit are described below. Layers in parentheses are optional.
(a) (hole injecting layer/) hole transporting layer/fluorescent light emitting layer/electron transporting layer (/electron injecting layer)
(b) (hole injecting layer/) hole transporting layer/phosphorescent light emitting layer electron transporting layer (/electron injecting layer)
(c) (hole injecting layer/) hole transporting layer/first fluorescent light emitting layer/second fluorescent light emitting layer/electron transporting layer (/electron injecting layer)
(d) (hole injecting layer/) hole transporting layer/first phosphorescent light emitting layer/second phosphorescent light emitting layer/electron transporting layer (/electron injecting layer)
(e) (hole injecting layer/) hole transporting layer/phosphorescent light emitting layer/space layer/fluorescent light emitting layer/electron transporting layer (/electron injecting layer)
(f) (hole injecting layer/) hole transporting layer/first phosphorescent light emitting layer/second phosphorescent light emitting layer/space layer/fluorescent light emitting layer/electron transporting layer (/electron injecting layer)
(g) (hole injecting layer/) hole transporting layer/first phosphorescent light emitting layer/space layer/second phosphorescent light emitting layer/space layer/fluorescent light emitting layer/electron transporting layer (/electron injecting layer)
(h) (hole injecting layer/) hole transporting layer/phosphorescent light emitting layer/space layer/first fluorescent light emitting layer/second fluorescent light emitting layer/electron transporting layer (/electron injecting layer)
(i) (hole injecting layer/) hole transporting layer/electron blocking layer/fluorescent light emitting layer/electron transporting layer (/electron injecting layer)
(j) (hole injecting layer/) hole transporting layer/electron blocking layer/phosphorescent light emitting layer/electron transporting layer (/electron injecting layer)
(k) (hole injecting layer/) hole transporting layer/exciton blocking layer/fluorescent light emitting layer/electron transporting layer (/electron injecting layer)
(l) (hole injecting layer/) hole transporting layer/exciton blocking layer/phosphorescent light emitting layer/electron transporting layer (/electron injecting layer)
(m) (hole injecting layer/) first hole transporting layer/second hole transporting layer/fluorescent light emitting layer/electron transporting layer (/electron injecting layer)
(n) (hole injecting layer/) first hole transporting layer/second hole transporting layer/phosphorescent light emitting layer/electron transporting layer (/electron injecting layer)
(o) (hole injecting layer/) first hole transporting layer/second hole transporting layer/fluorescent light emitting layer/first electron transporting layer/second electron transporting layer (/electron injecting layer)
(p) (hole injecting layer/) first hole transporting layer/second hole transporting layer/phosphorescent light emitting layer/first electron transporting layer/second electron transporting layer (/electron injecting layer)
(q) (hole injecting layer/) hole transporting layer/fluorescent light emitting layer/hole blocking layer/electron transporting layer (/electron injecting layer)
(r) (hole injecting layer/) hole transporting layer/phosphorescent light emitting layer/hole blocking layer/electron transporting layer (/electron injecting layer)
(s) (hole injecting layer/) hole transporting layer/fluorescent light emitting layer/exciton blocking layer/electron transporting layer (/electron injecting layer)
(t) (hole injecting layer/) hole transporting layer/phosphorescent light emitting layer/exciton blocking layer/electron transporting layer (/electron injecting layer)

The phosphorescent and fluorescent light emitting layers each can emit emission colors different from each other. Specifically, in the light emitting unit (f), a layer configuration, such as (hole injecting layer/) hole transporting layer/first phosphorescent light emitting layer (red light emission)/second phosphorescent light emitting layer (green light emission)/space layer/fluorescent light emitting layer (blue light emission)/electron transporting layer, may be exemplified.

An electron blocking layer may be properly provided between each light emitting layer and the hole transporting layer or the space layer. Further, a hole blocking layer may be properly provided between each light emitting layer and the electron transporting layer. The employment of the electron blocking layer or the hole blocking layer allows improving the emission efficiency by trapping electrons or holes within the light emitting layer and increasing the probability of charge recombination in the light emitting layer.

As a representative device configuration of the tandem type organic EL device, the following device configuration may be exemplified.

### (2) Anode/First Light Emitting Unit/Intermediate Layer/Second Light Emitting Unit/Cathode

Here, for example, each of the first light emitting unit and the second light emitting unit may be independently selected from the above-described light emitting units.

The intermediate layer is also generally referred to as an intermediate electrode, an intermediate conductive layer, a charge generation layer, an electron withdrawing layer, a connecting layer, or an intermediate insulating layer, and a known material configuration, in which electrons are supplied to the first light emitting unit and holes are supplied to the second light emitting unit, can be used.

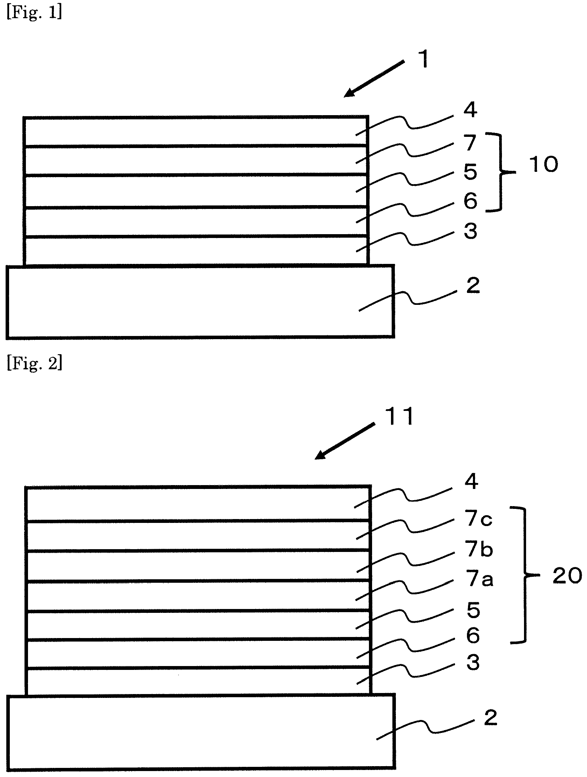

Fig. 1 is a schematic view showing an example of the configuration of the organic EL device of the present invention. The organic EL device 1 includes a substrate 2, an anode 3, a cathode 4, and a light emitting unit 10 disposed between the anode 3 and the cathode 4. The light emitting unit 10 includes a light emitting layer 5. A hole transporting zone 6 (such as a hole injecting layer and a hole transporting layer) is provided between the light emitting layer 5 and the anode 3, and an electron transporting zone 7 (such as an electron injecting layer and an electron transporting layer) is provided between the light emitting layer 5 and the cathode 4. In addition, an electron blocking layer (which is not shown in the figure) may be provided on the side of the anode 3 of the light emitting layer 5, and a hole blocking layer (which is not shown in the figure) may be provided on the side of the cathode 4 of the light emitting layer 5. As a result, electrons and holes are trapped in the light emitting layer 5, thereby making it possible to further increase the production efficiency of excitons in the light emitting layer 5.

Fig. 2 is a schematic view showing another configuration of the organic EL device of the present invention. An organic EL device 11 includes the substrate 2, the anode 3, the cathode 4, and a light emitting unit 20 disposed between the anode 3 and the cathode 4. The light emitting unit 20 includes the light emitting layer 5. In addition, a hole transporting zone 6 (such as a hole injecting layer and a hole transporting layer) is disposed between the anode 3 and the light emitting layer 5. Further, an electron transporting zone is disposed between the light emitting layer 5 and the cathode 4, and the electron transporting zone includes a hole blocking layer 7a, an electron transporting layer 7b, and an electron injecting layer 7c.

In the present invention, a host combined with a fluorescent dopant (a fluorescent light emitting material) is referred to as a fluorescent host, and a host combined with a phosphorescent dopant is referred to as a phosphorescent host. The fluorescent host and the phosphorescent host are not distinguished from each other merely by the molecular structures thereof. That is, the phosphorescent host means a material that forms a phosphorescent light emitting layer containing a phosphorescent dopant, and does not mean unavailability as a material that forms a fluorescent light emitting layer. The same also applies to the fluorescent host.

### Substrate

The substrate is used as a support of the organic EL device. Examples of the substrate include a plate of glass, quartz, and plastic. In addition, a flexible substrate may be used. Examples of the flexible substrate include a plastic substrate made of polycarbonate, polyarylate, polyether sulfone, polypropylene, polyester, polyvinyl fluoride, or polyvinyl chloride. In addition, an inorganic vapor deposition film can be used.

### Anode

It is preferable that a metal, an alloy, an electrically conductive compound, and a mixture thereof, which has a high work function (specifically 4.0 eV or more) are used for the anode formed on the substrate. Specific examples thereof include indium oxide-tin oxide (ITO: Indium Tin Oxide), indium oxide-tin oxide containing silicon or silicon oxide, indium oxide-zinc oxide, indium oxide containing tungsten oxide and zinc oxide, and graphene. In addition, examples thereof include gold (Au), platinum (Pt), nickel (Ni), tungsten (W), chromium (Cr), molybdenum (Mo), iron (Fe), cobalt (Co), copper (Cu), palladium (Pd), titanium (Ti), and nitrides of the metals (for example, titanium nitride).

These materials are usually deposited by a sputtering method. For example, through a sputtering method, it is possible to form indium oxide-zinc oxide by using a target in which 1 to 10 wt% of zinc oxide is added to indium oxide, and to form indium oxide containing tungsten oxide and zinc oxide by using a target containing 0.5 to 5 wt% of tungsten oxide and 0.1 to 1 wt% of zinc oxide with respect to indium oxide. In addition, the production may be performed by a vacuum vapor deposition method, a coating method, an inkjet method, a spin coating method, or the like.

The hole injecting layer formed in contact with the anode is formed by using a material that facilitates hole injection regardless of a work function of the anode, and thus it is possible to use materials generally used as an electrode material (for example, metals, alloys, electrically conductive compounds, and mixtures thereof, elements belonging to Group 1 or Group 2 of the periodic table of the elements).

It is also possible to use elements belonging to Group 1 or Group 2 of the periodic table of the elements, which are materials having low work functions, that is, alkali metals such as lithium (Li) and cesium (Cs), alkaline earth metals such as magnesium (Mg), calcium (Ca), and strontium (Sr), and alloys containing these (such as MgAg and AlLi), as well as rare earth metals such as europium (Eu) and ytterbium (Yb), and alloys containing these. When the anode is formed by using the alkali metals, the alkaline earth metals, and alloys containing these, a vacuum vapor deposition method or a sputtering method can be used. Further, when a silver paste or the like is used, a coating method, an inkjet method, or the like can be used.

### Hole Injecting Layer

The hole injecting layer is a layer containing a material having a high hole injection capability (a hole injecting material) and is provided between the anode and the light emitting layer, or between the hole transporting layer, if exists, and the anode.

As the hole injecting material, molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, and the like can be used.

Examples of the hole injecting layer material also include aromatic amine compounds as low-molecular weight organic compounds, such as 4,4',4"-tris(N,N-diphenylamino)triphenylamine (abbreviation: TDATA), 4,4', 4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1).

High-molecular weight compounds (such as oligomers, dendrimers, and polymers) may also be used. Examples thereof include high-molecular weight compounds, such as poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). In addition, high-molecular weight compounds to which an acid, such as poly(3,4-ethylenedioxythiophene)/poly (styrene sulfonic acid) (PEDOT/PSS) and polyaniline/poly (styrenesulfonic acid) (PAni/PSS), is added, can also be used.

Furthermore, it is also preferable to use an acceptor material, such as a hexaazatriphenylene (HAT) compound represented by the following formula (K).

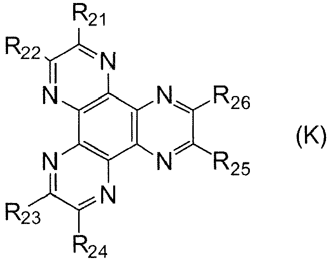

In the above formula, R₂₁ to R₂₆ each independently represent a cyano group, -CONH₂, a carboxy group, or -COOR₂₇(R₂₇ represents an alkyl group having 1 to 20 carbon atoms or a cycloalkyl group having 3 to 20 carbon atoms). In addition, adjacent two selected from R₂₁ and R₂₂, R₂₃ and R₂₄, and R₂₅ and R₂₆ may be bonded to each other to form a group represented by -CO-O-CO-.

Examples of R₂₇ include a methyl group, an ethyl group, an n-propyl group, an isopropyl group, an n-butyl group, an isobutyl group, a t-butyl group, a cyclopentyl group, and a cyclohexyl group.

### Hole Transporting Layer

The hole transporting layer is a layer containing a material having a high hole transporting capability (a hole transporting material) and is provided between the anode and the light emitting layer, or between the hole injecting layer, if exists, and the light emitting layer.

The hole transporting layer may have a single layer structure or a multilayer structure including two or more layers. For example, the hole transporting layer may have a two-layer structure including a first hole transporting layer (anode side) and a second hole transporting layer (cathode side). In one embodiment of the present invention, the hole transporting layer having a single layer structure is preferably disposed adjacent to the light emitting layer, and the hole transporting layer that is closest to the cathode in the multilayer structure, such as the second hole transporting layer in the two-layer structure, is preferably disposed adjacent to the light emitting layer. In another embodiment of the present invention, an electron blocking layer described later and the like may be disposed between the hole transporting layer having a single layer structure and the light emitting layer, or between the hole transporting layer that is closest to the light emitting layer in the multilayer structure and the light emitting layer.

As the hole transporting layer material, for example, an aromatic amine compound, a carbazole derivative, an anthracene derivative, and the like can be used.

Examples of the aromatic amine compound include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluoren-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylaminolbiphenyl (abbreviation: BSPB). The aforementioned compounds have a hole mobility of 10⁻⁶cm²/Vs or more.

Examples of the carbazole derivative include 4,4'-di(9-carbazolyl)biphenyl (abbreviation: CBP), 9-[4-(9-carbazolyl)phenyl]-10-phenylanthracene (abbreviation: CzPA), and 9-phenyl-3-[4-(10-phenyl-9-anthryl)phenyl]-9H-carbazole (abbreviation: PCzPA).

Examples of the anthracene derivative include 2-t-butyl-9,10-di(2-naphthyl)anthracene (abbreviation: t-BuDNA), 9,10-di(2-naphthyl)anthracene (abbreviation: DNA), and 9,10-diphenylanthracene (abbreviation: DPAnth).

High-molecular weight compounds, such as poly(N-vinylcarbazole) (abbreviation: PVK) and poly(4-vinyltriphenylamine) (abbreviation: PVTPA), can also be used.

However, compounds other than those as mentioned above can also be used as long as they are compounds high in the hole transporting capability rather than in the electron transporting capability.

### Dopant Material of Light Emitting Layer

The light emitting layer is a layer containing a material having a high light emitting property (a dopant material), and various materials can be used. For example, a fluorescent light emitting material or a phosphorescent light emitting material can be used as the dopant material. The fluorescent light emitting material is a compound that emits light from a singlet excited state, and the phosphorescent light emitting material is a compound that emits light from a triplet excited state.

Examples of a blue-based fluorescent light emitting material that can be used for the light emitting layer include a pyrene derivative, a styrylamine derivative, a chrysene derivative, a fluoranthene derivative, a fluorene derivative, a diamine derivative, and a triarylamine derivative. Specific examples thereof include N,N'-bis[4-(9H-carbazole-9-yl)phenyl]-N,N'-diphenylstilbene-4,4'-diamine (abbreviation: YGA2S), 4-(9H-carbazole-9-yl)-4'-(10-phenyl-9-anthryl)triphenylamine (abbreviation: YGAPA), and 4-(10-phenyl-9-anthryl)-4'-(9-phenyl-9H-carbazole-3-yl)triphenylamine (abbreviation: PCBAPA).

Examples of a green-based fluorescent light emitting material that can be used for the light emitting layer include an aromatic amine derivative. Specific examples thereof include N-(9,10-diphenyl-2-anthryl)-N,9-diphenyl-9H-carbazole-3-amine (abbreviation: 2PCAPA), N-[9,10-bis(1,1'-biphenyl-2-yl)-2-anthryl]-N,9-diphenyl-9H-carbazole-3-amine (abbreviation: 2PCABPhA), N-(9,10-diphenyl-2-anthryl)-N,N',N'-triphenyl-1,4-phenylenediamine (abbreviation: 2DPAPA), N-[9,10-bis(1,1'-biphenyl-2-yl)-2-anthryl]-N,N',N'-triphenyl-1,4-phenylenediamine (abbreviation: 2DPABPhA), N-[9,10-bis(1,1'-biphenyl-2-yl)]-N-[4-(9H-carbazole-9-yl)phenyl]-N-phenylanthracene-2-amine (abbreviation: 2YGABPhA), and N,N,9-triphenylanthracene-9-amine (abbreviation: DPhAPhA).

Examples of a red-based fluorescent light emitting material that can be used for the light emitting layer include a tetracene derivative and a diamine derivative. Specific examples thereof include N,N,N',N'-tetrakis(4-methylphenyl)tetracene-5,11-diamine (abbreviation: p-mPhTD) and 7,14-diphenyl-N,N,N',N'-tetrakis(4-methylphenyl)acenaphtho[1,2-a]fluoranthene-3,10-diamine (abbreviation: p-mPhAFD).

Examples of a blue-based phosphorescent light emitting material that can be used for the light emitting layer include a metal complex, such as an iridium complex, an osmium complex, and a platinum complex. Specific examples thereof include bis[2-(4',6'-difluorophenyl)pyridinato-N,C2']iridium(III)tetrakis(1-pyrazolyl)borate (abbreviation: FIr6), bis[2-(4',6'-difluorophenyl)pyridinato-N,C2']iridium(III)picolinate (abbreviation: FIrpic), bis[2-(3',5'bistrifluoromethylphenyl)pyridinato-N,C2']iridium(III)picolinate (abbreviation: Ir(CF3ppy)2(pic)), and bis[2-(4',6'-difluorophenyl)pyridinato-N,C2']iridium(III)acetylacetonate (abbreviation: FIracac).

Examples of a green-based phosphorescent light emitting material that can be used for the light emitting layer include an iridium complex. Examples thereof include tris(2-phenylpyridinato-N,C2')iridium(III) (abbreviation: Ir(ppy)3), bis(2-phenylpyridinato-N,C2')iridium(III)acetylacetonate (abbreviation: Ir(ppy)2(acac)), bis(1,2-diphenyl-1H-benzimidazolato)iridium(III)acetylacetonate (abbreviation: Ir(pbi)2(acac)), and bis(benzo[h]quinolinato)iridium(III)acetylacetonate (abbreviation: Ir(bzq)2(acac)).

Examples of a red-based phosphorescent light emitting material that can be used for the light emitting layer include a metal complex, such as an iridium complex, a platinum complex, a terbium complex, and a europium complex. Specific examples thereof include organic metal complexes, such as bis[2-(2'-benzo[4,5-α]thienyl)pyridinato-N,C3']iridium(III)acetylacetonate (abbreviation: Ir(btp)2(acac)), bis(1-phenylisoquinolinato-N,C2')iridium(III)acetylacetonate (abbreviation: Ir(piq)2(acac)), (acetylacetonate)bis[2,3-bis(4-fluorophenyl)quinoxalinato]iridium(III) (abbreviation: Ir(Fdpq)2(acac)), and 2,3,7,8,12,13,17,18-octaethyl-21H,23H-porphyrinplatinum(II) (abbreviation: PtOEP).

In addition, rare earth metal complexes, such as tris(acetylacetonate) (monophenanthroline)terbium(III) (abbreviation: Tb(acac)3(Phen)), tris(1,3-diphenyl-1,3- propanedionate)(monophenanthroline)europium(III) (abbreviation: Eu(DBM)3(Phen)), and tris[1-(2-thenoyl)-3,3,3-trifluoroacetonate](monophenanthroline)europium(III) (abbreviation: Eu(TTA)3(Phen)), emit light from rare earth metal ions (electron transition between different multiplicities), and thus may be used as the phosphorescent light emitting material.

### Host Material of Light Emitting Layer

The light emitting layer may have a configuration in which the aforementioned dopant material is dispersed in another material (a host material). The host material is preferably a material that has a higher lowest unoccupied orbital level (LUMO level) and a lower highest occupied orbital level (HOMO level) than the dopant material.

Examples of the host material include:
(1) a metal complex, such as an aluminum complex, a beryllium complex, and a zinc complex,
(2) a heterocyclic compound, such as an oxadiazole derivative, a benzimidazole derivative, and a phenanthroline derivative,
(3) a fused aromatic compound, such as a carbazole derivative, an anthracene derivative, a phenanthrene derivative, a pyrene derivative, and a chrysene derivative, or
(4) an aromatic amine compound, such as a triarylamine derivative and a fused polycyclic aromatic amine derivative.

For example,
metal complexes, such as tris(8-quinolinolato)aluminum(III) (abbreviation: Alq), tris(4-methyl-8-quinolinolato)aluminum(III) (abbreviation: Almq3), bis(10-hydroxybenzo[h]quinolinato)beryllium(II) (abbreviation: BeBq2), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), and bis[2-(2-benzothiazolyl)phenolato]zinc(II) (abbreviation: ZnBTZ);
heterocyclic compounds, such as 2-(4-biphenylyl)-5-(4-tert-butylphenyl)-1,3,4-oxadiazole (abbreviation: PBD), 1,3-bis[5-(p-tert-butylphenyl)-1,3,4-oxadiazole-2-yl]benzene (abbreviation: OXD-7), 3-(4-biphenylyl)-4-phenyl-5-(4-tert-butylphenyl)-1,2,4-triazole (abbreviation: TAZ), 2,2',2"-(1,3,5-benzenetriyl)tris(1-phenyl-1H-benzimidazole) (abbreviation: TPBI), and bathophenanthroline (abbreviation: BPhen), and bathocuproine (abbreviation: BCP);
fused aromatic compounds, such as 9-[4-(10-phenyl-9-anthryl)phenyl]-9H-carbazole (abbreviation: CzPA), 3,6-diphenyl-9-[4-(10-phenyl-9-anthryl)phenyl]-9H-carbazole (abbreviation: DPCzPA), 9,10-bis(3,5-diphenylphenyl)anthracene (abbreviation: DPPA), 9,10-di(2-naphthyl)anthracene (abbreviation: DNA), 2-tert-butyl-9,10-di(2-naphthyl)anthracene (abbreviation: t-BuDNA), 9,9'-bianthryl(abbreviation: BANT), 9,9'-(stilbene-3,3'-diyl)diphenanthrene (abbreviation: DPNS), 9,9'-(stilbene-4,4'-diyl)diphenanthrene (abbreviation: DPNS2), 3,3',3"-(benzene-1,3,5-triyl)tripyrene (abbreviation: TPB3), 9,10-diphenylanthracene (abbreviation: DPAnth), and 6,12-dimethoxy-5,11-diphenylchrysene; and
aromatic amine compounds, such as N,N-diphenyl-9-[4-(10-phenyl-9-anthryl)phenyl]-9H-carbazole-3-amine (abbreviation: CzA1PA), 4-(10-phenyl-9-anthryl)triphenylamine (abbreviation: DPhPA), N,9-diphenyl-N-[4-(10-phenyl-9-anthryl)phenyl]-9H-carbazole-3-amine (abbreviation: PCAPA), N,9-diphenyl-N-{4-[4-(10-phenyl-9-anthryl)phenyl]phenyl}-9H-carbazole-3-amine (abbreviation: PCAPBA), N-(9,10-diphenyl-2-anthryl)-N,9-diphenyl-9H-carbazole-3-amine (abbreviation: 2PCAPA), 4,4'-bis[N-(1-naphthyl)-N-phenylamino]biphenyl (abbreviation: NPB or α-NPD), N,N'-bis(3-methylphenyl)-N,N'-diphenyl-[1,1'-biphenyl]-4,4'-diamine (abbreviation: TPD), 4,4'-bis[N-(9,9-dimethylfluorene-2-yl)-N-phenylamino]biphenyl (abbreviation: DFLDPBi), and 4,4'-bis[N-(spiro-9,9'-bifluorene-2-yl)-N-phenylamino]biphenyl (abbreviation: BSPB) can be used. A plurality of host materials may be used.

In particular, in the case of a blue fluorescent device, it is preferable to use the following anthracene compounds as the host material.

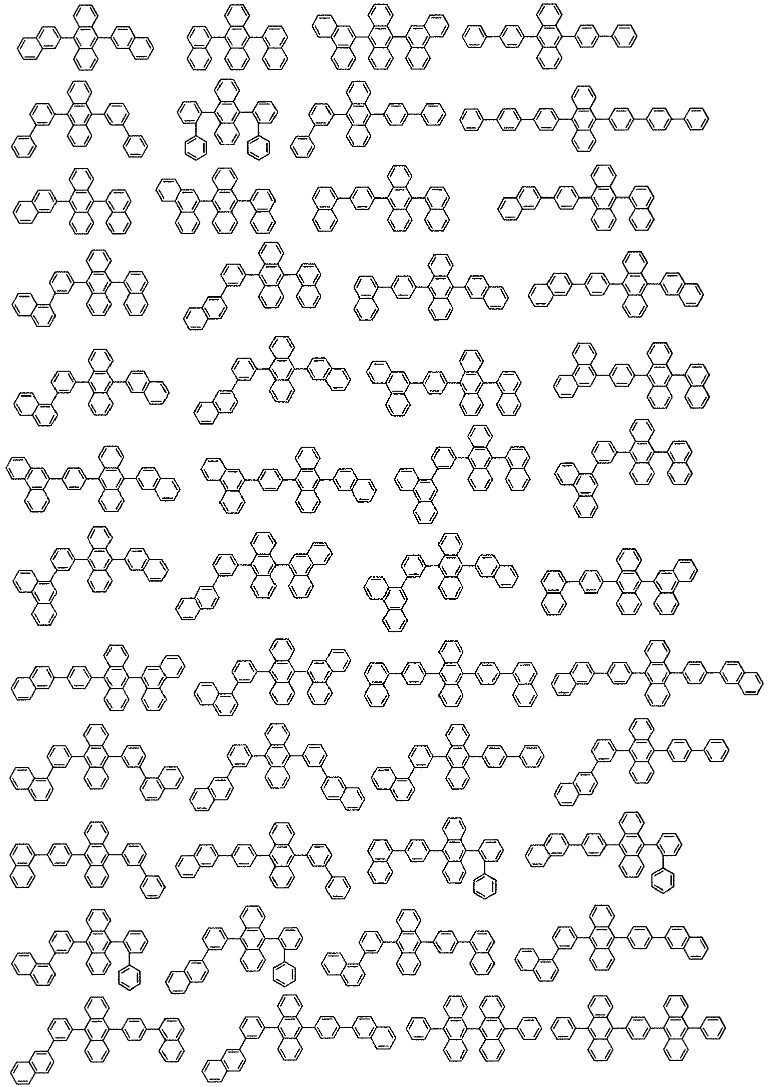

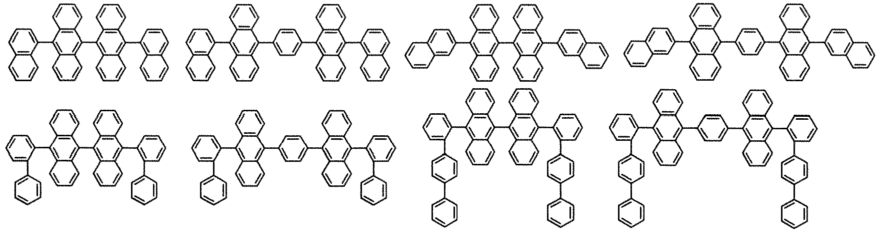

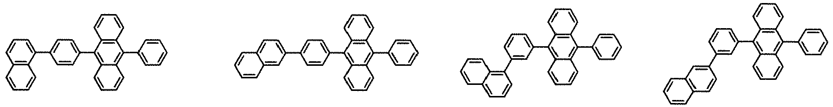

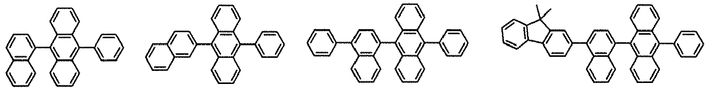

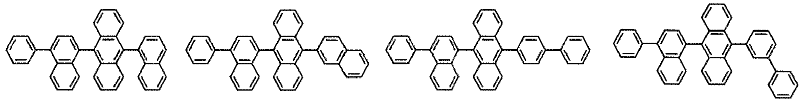

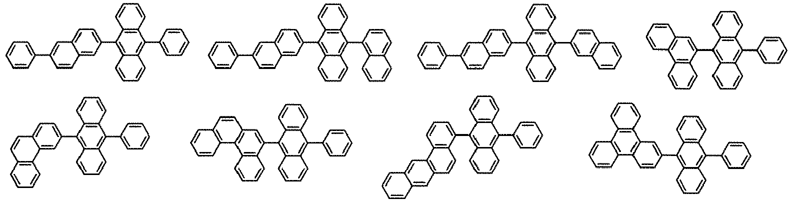

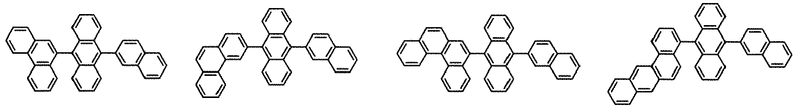

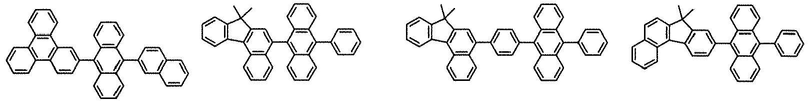

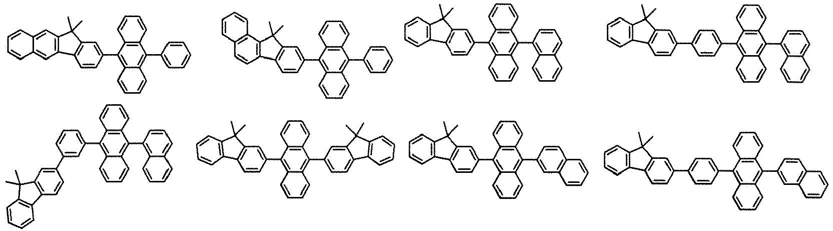

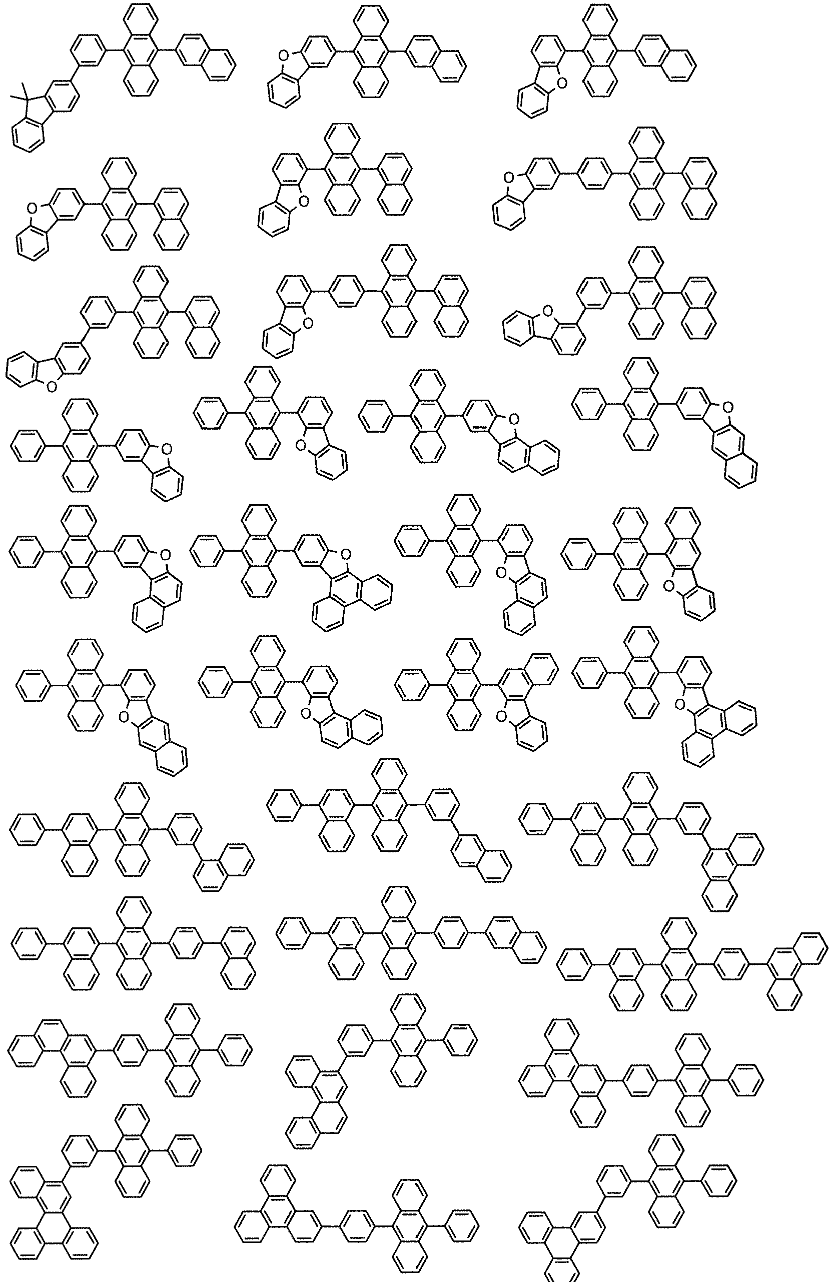

### Electron Transporting Layer

The electron transporting layer is a layer containing a material having a high electron transporting capability (an electron transporting material) and is provided between the light emitting layer and the cathode, or between the electron injecting layer, if exists, and the light emitting layer.

It is preferable that the inventive compound is used alone in the electron transporting layer or is used as a combination with the following compounds in the electron transporting layer.

The electron transporting layer may have a single layer structure or a multilayer structure including two or more layers. For example, the electron transporting layer may have a two-layer structure including a first electron transporting layer (anode side) and a second electron transporting layer (cathode side). In one embodiment of the present invention, the electron transporting layer having a single layer structure is preferably disposed adjacent to the light emitting layer, and the electron transporting layer that is closest to the anode in the multilayer structure, such as the first electron transporting layer in the two-layer structure, is preferably disposed adjacent to the light emitting layer. In another embodiment of the present invention, a hole blocking layer described later and the like may be disposed between the electron transporting layer having a single layer structure and the light emitting layer, or between the electron transporting layer that is closest to the light emitting layer in the multilayer structure and the light emitting layer.

In the electron transporting layer having a two-layer structure, the inventive compound may be contained in one of the first electron transporting layer and the second electron transporting layer, and may also be contained in both of the first electron transporting layer and the second electron transporting layer.

In one embodiment of the present invention, it is preferable that the inventive compound is contained only in the first electron transporting layer adjacent to the light emitting layer, and in another embodiment thereof, it is preferable that the inventive compound is contained only in the second electron transporting layer, and in still another embodiment thereof, it is preferable that the inventive compound is contained in the first electron transporting layer and the second electron transporting layer.

In one embodiment of the present invention, the inventive compound contained in one or both of the first electron transporting layer and the second electron transporting layer is preferably a light hydrogen body from the viewpoint of production cost.

The light hydrogen body refers to the inventive compound in which all hydrogen atoms in the formula (1) are protium atoms.

Therefore, the present invention includes an organic EL device containing the inventive compound in which one or both of the first electron transporting layer and the second electron transporting layer are substantially composed of only a light hydrogen body. The "inventive compound substantially composed of only of a light hydrogen body" means that the content ratio of the light hydrogen body to the total amount of the compound represented by the formula (1) is 90 mol% or more, preferably 95 mol% or more, more preferably 99 mol% or more (each including 100%).

Examples of the material which can be used for the electron transporting layer other than the inventive compound include:
(1) a metal complex, such as an aluminum complex, a beryllium complex, and a zinc complex;
(2) a heteroaromatic compound, such as an imidazole derivative, a benzimidazole derivative, an azine derivative, a carbazole derivative, and a phenanthroline derivative; and
(3) a high-molecular weight compound.

Examples of the metal complex include tris(8-quinolinolato)aluminum(III) (abbreviation: Alq), tris(4-methyl-8-quinolinolato)aluminum (abbreviation: Almq3), bis(10-hydroxybenzo[h]quinolinato)beryllium (abbreviation: BeBq₂), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), and bis[2-(2-benzothiazolyl)phenolato]zinc(II) (abbreviation: ZnBTZ).

Examples of the heteroaromatic compound include 2-(4-biphenylyl)-5-(4-tert-butylphenyl)-1,3,4-oxadiazole (abbreviation: PBD), 1,3-bis[5-(p-tert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzxazol-2-yl)stilbene (abbreviation: BzOs).

Examples of the high-molecular weight compound include poly[(9,9-dihexylfluorene-2,7-diyl)-co-(pyridine-3,5-diyl)] (abbreviation: PF-Py), and poly[(9,9-dioctylfluorene-2,7-diyl)-co-(2,2'-bipyridine-6,6'-diyl)] (abbreviation: PF-BPy).

The materials are materials having an electron mobility of 10⁻⁶ cm²/Vs or more. Materials other than those as mentioned above may also be used in the electron transporting layer as long as they are materials high in the electron transporting capability rather than in the hole transporting capability.

### Electron Injecting Layer

The electron injecting layer is a layer containing a material having a high electron injection capability (an electron injecting material), and is formed between the cathode and the light emitting layer, or between the electron transporting layer, if exists, and the cathode. The inventive compound can be used in the electron injecting layer.

As the electron injecting material other than the inventive compound, alkali metals such as lithium (Li) and cesium (Cs), alkaline earth metals such as magnesium (Mg), calcium (Ca), and strontium (Sr), rare earth metals such as europium (Eu) and ytterbium (Yb), and compounds containing these metals can be used. Examples of the compounds include an alkali metal oxide, an alkali metal halide, an alkali metal-containing organic complex, an alkaline earth metal oxide, an alkaline earth metal halide, an alkaline earth metal-containing organic complex, a rare earth metal oxide, a rare earth metal halide, and a rare earth metal-containing organic complex. Further, these compounds may be used as a mixture of a plurality thereof.

In addition, a material having an electron transporting capability, in which an alkali metal, an alkaline earth metal, or a compound thereof is contained, specifically Alq in which magnesium (Mg) is contained may be used. In this case, electron injection from the cathode can be more efficiently performed.

Otherwise, in the electron injecting layer, a composite material obtained by mixing an organic compound with an electron donor may be used. Such a composite material is excellent in the electron injection capability and the electron transporting capability because the organic compound receives electrons from the electron donor. In this case, the organic compound is preferably a material excellent in transporting received electrons, and specifically, for example, a material constituting the aforementioned electron transporting layer (such as a metal complex and a heteroaromatic compound) can be used. As the electron donor, a material having an electron donation property for the organic compound may be used. Specifically, alkali metals, alkaline earth metals, and rare earth metals are preferred, and examples thereof include lithium, cesium, magnesium, calcium, erbium, and ytterbium. In addition, an alkali metal oxide or an alkaline earth metal oxide is preferred, and examples thereof include lithium oxide, calcium oxide, and barium oxide. In addition, a Lewis base, such as magnesium oxide, can also be used. In addition, an organic compound, such as tetrathiafulvalene (abbreviation: TTF), can also be used.

### Cathode

It is preferable that a metal, an alloy, an electrically conductive compound, and a mixture thereof, which has a low work function (specifically 3.8 eV or less) are used for the cathode. Specific examples of such a cathode material include elements belonging to Group 1 or Group 2 of the periodic table of the elements, that is, alkali metals such as lithium (Li) and cesium (Cs), alkaline earth metals such as magnesium (Mg), calcium (Ca), and strontium (Sr), as well as alloys containing these (such as MgAg, and AlLi), rare earth metals such as europium (Eu), and ytterbium (Yb), and alloys containing these.

When the cathode is formed by using the alkali metals, the alkaline earth metals, and the alloys containing these, a vacuum vapor deposition method or a sputtering method can be used. In addition, when a silver paste or the like is used, a coating method, an inkjet method, or the like can be used.

By providing the electron injecting layer, the cathode can be formed using various conductive materials, such as Al, Ag, ITO, graphene, and indium oxide-tin oxide containing silicon or silicon oxide regardless of the magnitude of a work function. These conductive materials can be deposited by using a sputtering method, an inkjet method, a spin coating method, or the like.

### Insulating Layer

The organic EL device applies an electric field to an ultrathin film, and thus pixel defects are likely to occur due to leaks or short-circuiting. In order to prevent it, an insulating layer formed of an insulating thin film layer may be inserted between a pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. A mixture or a laminate of these may also be used.

### Space Layer

The space layer is, for example, a layer provided between a fluorescent light emitting layer and a phosphorescent light emitting layer for the purpose of preventing excitons generated in the phosphorescent light emitting layer from diffusing into the fluorescent light emitting layer, or adjusting a carrier balance, in the case where the fluorescent light emitting layers and the phosphorescent light emitting layers are laminated. In addition, the space layer can also be provided among a plurality of phosphorescent light emitting layers.

Since the space layer is provided between the light emitting layers, a material having both an electron transporting capability and a hole transporting capability is preferable. Also, one having a triplet energy of 2.6 eV or more is preferable in order to prevent triplet energy diffusion in an adjacent phosphorescent light emitting layer. Examples of the material used for the space layer include the same materials as those used for the hole transporting layer as described above.

### Blocking Layer

The blocking layer, such as the electron blocking layer, the hole blocking layer, and the exciton blocking layer, may be provided adjacent to the light emitting layer. The electron blocking layer is a layer that prevents electrons from leaking from the light emitting layer to the hole transporting layer, and the hole blocking layer is a layer that prevents holes from leaking from the light emitting layer to the electron transporting layer.

As one embodiment, it is preferable to use the inventive compound as the hole blocking layer.

The exciton blocking layer has a function of preventing excitons generated in the light emitting layer from diffusing into the surrounding layers, and trapping the excitons within the light emitting layer.

Each layer of the organic EL device may be formed by a conventionally known vapor deposition method, a coating method, or the like. For example, each layer can be formed by a known method using a vapor deposition method such as a vacuum vapor deposition method and a molecular beam vapor deposition method (MBE method), or a coating method using a solution of a compound forming a layer, such as a dipping method, a spin-coating method, a casting method, a bar-coating method, and a roll-coating method.

The film thickness of each layer is not particularly limited, but is typically 5 nm to 10 pm, and more preferably 10 nm to 0.2 µm because in general, when the film thickness is too small, defects such as pinholes are likely to occur, and conversely, when the film thickness is too large, a high driving voltage is required and the efficiency decreases.

The organic EL device can be used in electronic devices, such as display components of an organic EL panel module and the like, display devices of a television, a mobile phone, a personal computer, and the like, and light emitting devices of lightings and vehicular lamps.

### Examples

Hereinafter, the present invention will be described in more detail with reference to Examples. However, the present invention is not limited to the following Examples.

### Inventive Compounds used in Production of Organic EL Devices of Examples 1 to 19 below

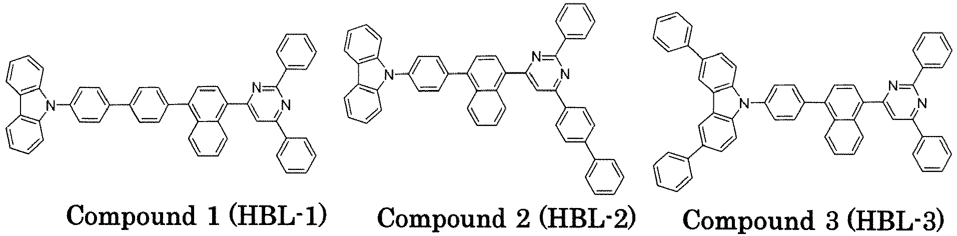

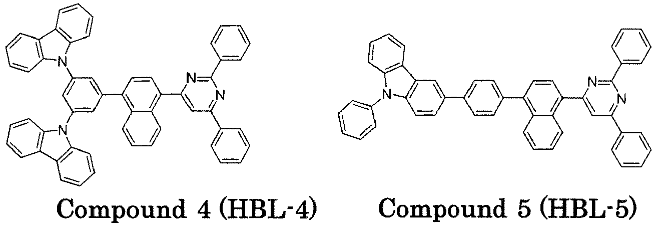

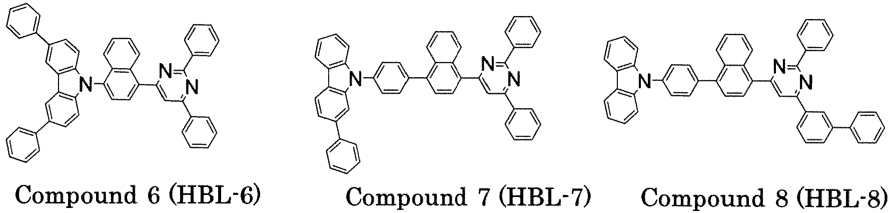

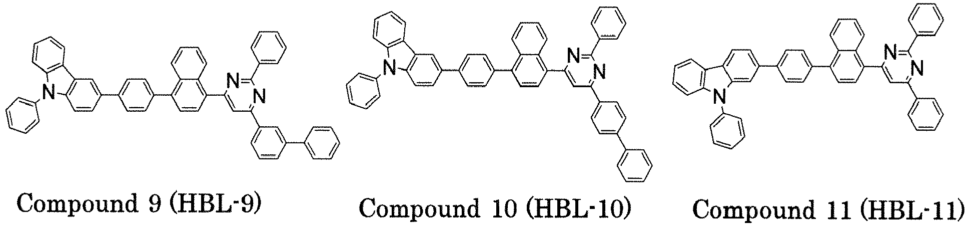

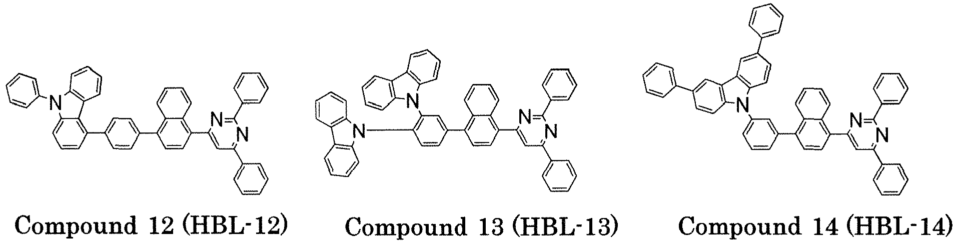

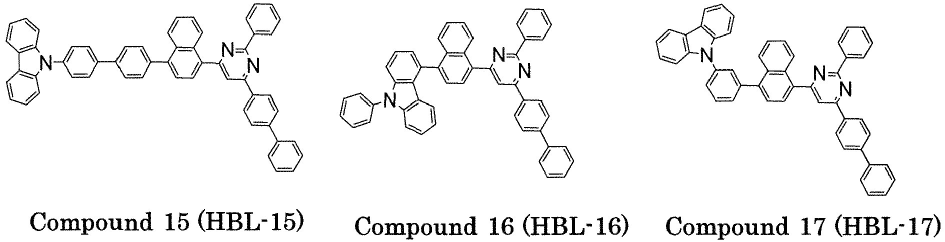

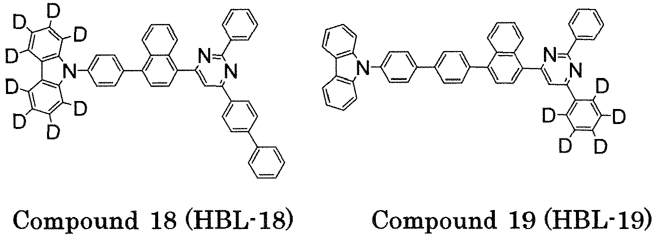

### Comparative Compounds used in Production of Organic EL Devices of Comparative Examples 1 to 3 below

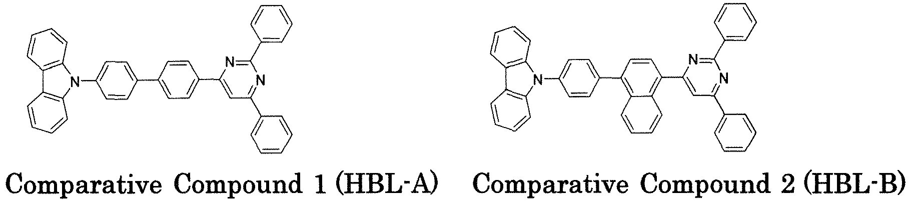

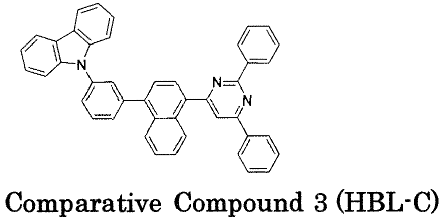

### Other Compounds used in Production of Organic EL Devices of Examples 1 to 19 and Comparative Examples 1 to 3 below

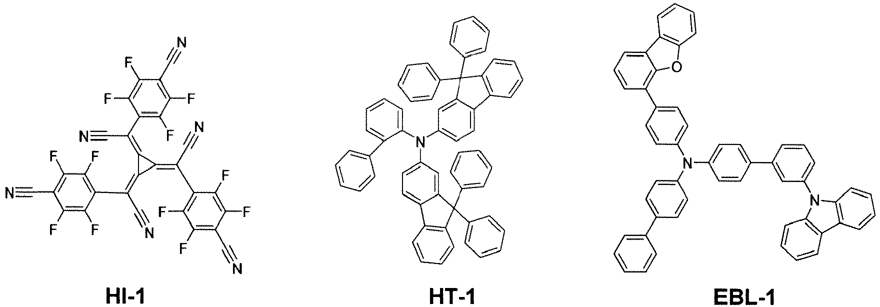

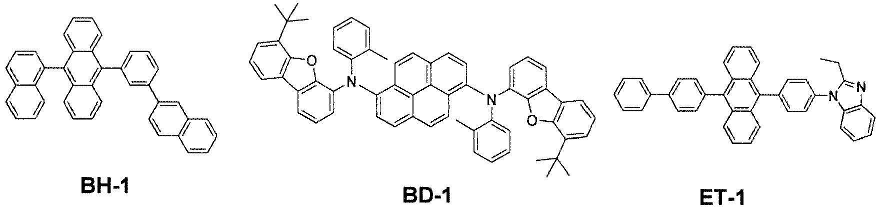

Each organic EL device was produced in the following manner, and each device was evaluated for the EL device performance.

### Production of Organic EL Device

### Example 1

A glass substrate of 25 mm × 75 mm × 1.1 mm provided with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate provided with the ITO transparent electrode lines was mounted on a substrate holder of a vacuum vapor deposition apparatus, and firstly, Compound HT-1 and Compound HI-1 were vapor co-deposited on a surface having the transparent electrode lines formed thereon, so as to cover the transparent electrode, resulting in a hole injecting layer with a film thickness of 10 nm. The mass ratio of Compound HT-1 and Compound HI-1 was 97 : 3.

Subsequently, on the hole injecting layer, Compound HT-1 was vapor deposited to form a first hole transporting layer with a film thickness of 80 nm.

Subsequently, on the first hole transporting layer, Compound EBL-1 was vapor deposited to form a second hole transporting layer (electron blocking layer) with a film thickness of 10 nm.

Subsequently, on the second hole transporting layer (electron blocking layer), Compound BH-1 (host material) and Compound BD-1 (dopant material) were vapor co-deposited to form a light emitting layer with a film thickness of 25 nm. The mass ratio of Compound BH-1 and Compound BD-1 was 96 : 4.

Subsequently, on the light emitting layer, Compound 1 (HBL-1) was vapor deposited to form a hole blocking layer with a film thickness of 10 nm.

Compound ET-1 was vapor deposited to form an electron transporting layer with a film thickness of 15 nm.

Subsequently, on the electron transporting layer, Yb was vapor deposited to form an electron injecting electrode (cathode) with a film thickness of 1 nm.

Then, on the electron injecting electrode, metal Al was vapor deposited to form a metal cathode with a film thickness of 80 nm.

The layer configuration of the organic EL device of Example 1 thus obtained was as follows.

ITO (130)/(HT-1 : HI-1 = 97 : 3) (10)/HT-1 (80)/EBL-1 (10)/(BH-1 : BD-1 = 96 : 4 (25)/HBL-1 (10)/ET-1 (15)/Yb (1)/Al (80)

In the layer configuration, the numeral in parentheses indicates the film thickness (nm), and the ratio of HT-1 and HI-1, and the ratio of BH-1 and BD-1 each are a mass ratio.

### Examples 2 to 19

Organic EL devices of Examples 2 to 19 were produced in the same manner as in Example 1 except that Compound 2 (HBL-2) to Compound 19 (HBL-19) were used respectively in this order instead of Compound 1 (HBL-1) of the hole blocking layer in Example 1.

### Comparative Examples 1 to 3

Organic EL devices of Comparative Examples 1 to 3 were produced in the same manner as in Example 1 except that Comparative Compound 1 (HBL-A), Comparative Compound 2 (HBL-B), and Comparative Compound 3 (HBL-C) were used respectively in this order instead of Compound 1 (HBL-1) of the hole blocking layer in Example 1.

### Evaluation of Organic EL Device

The organic EL devices thus produced each were evaluated for device lifetime. The evaluation results are shown in Table 1.

### Device Lifetime (LT95)

The resulting organic EL device was driven with direct current at a current density of 50 mA/cm², and the period of time until the luminance was reduced to 95% of the initial luminance was measured and was defined as LT95 (95% lifetime).

**Table 1**

| | Compound HBL | LT95 (hour) at 50 mA/cm² |
|---|---|---|
| Example 1 | Compound 1 (HBL-1) | 44 |
| Example 2 | Compound 2 (HBL-2) | 69 |
| Example 3 | Compound 3 (HBL-3) | 62 |
| Example 4 | Compound 4 (HBL-4) | 65 |
| Example 5 | Compound 5 (HBL-5) | 58 |
| Example 6 | Compound 6 (HBL-6) | 82 |
| Example 7 | Compound 7 (HBL-7) | 49 |
| Example 8 | Compound 8 (HBL-8) | 46 |
| Example 9 | Compound 9 (HBL-9) | 48 |
| Example 10 | Compound 10 (HBL-10) | 66 |
| Example 11 | Compound 11 (HBL-11) | 55 |
| Example 12 | Compound 12 (HBL-12) | 45 |
| Example 13 | Compound 13 (HBL-13) | 56 |
| Example 14 | Compound 14 (HBL-14) | 60 |
| Example 15 | Compound 15 (HBL-15) | 85 |
| Example 16 | Compound 16 (HBL-16) | 60 |
| Example 17 | Compound 17 (HBL-17) | 65 |
| Example 18 | Compound 18 (HBL-18) | 70 |
| Example 19 | Compound 19 (HBL-19) | 45 |
| Comparative Example 1 | Comparative Compound 1 (HBL-A) | 34 |
| Comparative Example 2 | Comparative Compound 2 (HBL-B) | 38 |
| Comparative Example 3 | Comparative Compound 3 (HBL-C) | 38 |

### Evaluation of Glass Transition Temperature Tg

The glass transition temperature Tg was evaluated for the compound used as the hole blocking layer.

The glass transition temperature was measured by a method specified in JIS K7121 (1987) using a differential scanning calorimeter DSC7000X (Hitachi High-Tech Science Corporation). The measurement results are shown in Table 2.

**Table 2**

| Compound HBL | Tg (°C) |
|---|---|
| Compound 1 (HBL-1) | 133 |
| Compound 2 (HBL-2) | 138 |
| Compound 3 (HBL-3) | 151 |
| Compound 4 (HBL-4) | 159 |
| Compound 5 (HBL-5) | 128 |
| Compound 6 (HBL-6) | 156 |
| Compound 7 (HBL-7) | 136 |
| Compound 8 (HBL-8) | 128 |
| Compound 9 (HBL-9) | 130 |
| Compound 10 (HBL-10) | 141 |
| Compound 11 (HBL-11) | 133 |
| Compound 12 (HBL-12) | 131 |
| Compound 13 (HBL-13) | 157 |
| Compound 14 (HBL-14) | 146 |
| Compound 15 (HBL-15) | 146 |
| Compound 16 (HBL-16) | 149 |
| Compound 17 (HBL-17) | 128 |
| Compound 18 (HBL-18) | 138 |
| Compound 19 (HBL-19) | 133 |
| Comparative Compound 1 (HBL-A) | 108 |
| Comparative Compound 2 (HBL-B) | 123 |
| Comparative Compound 3 (HBL-C) | 113 |

As apparent from the results in Tables 1 and 2, the compounds of the present invention with a high glass transition temperature having a molecular weight of 650 or more and having a structure in which a specific carbazole skeleton is bonded to a pyrimidine skeleton via a single bond or an arylene group and further via a naphthylene group, exhibited an improved device lifetime as compared with Comparative Compound 1 to Comparative Compound 3, which did not satisfy the structural requirement or the molecular weight of the present invention.

### Synthesis of Compound

### Synthesis of Intermediate A

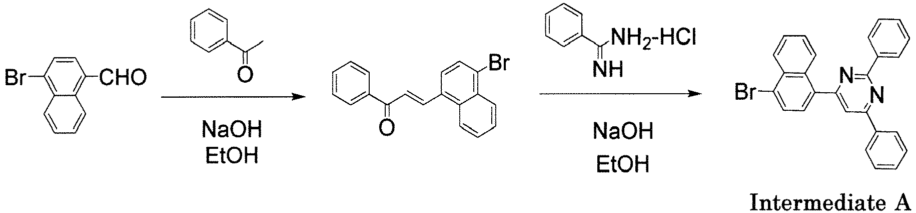

4-bromo-1-naphthoaldehyde (9.0 g), acetophenone (4.6 g) and sodium hydroxide (0.15 g) were added to 300 mL of ethanol and stirred at room temperature for 5 hours. Then, benzamidine hydrochloride (6.0 g) and sodium hydroxide (1.8 g) were added, and the mixture was stirred at 70°C for 5 hours. After completion of the reaction, the precipitate was separated by filtration and purified by silica gel column chromatography (developing solvent; hexane/toluene) to obtain Intermediate A as a white solid (7.6 g, yield 45%).

### Synthesis Example 1: Synthesis of Compound 1

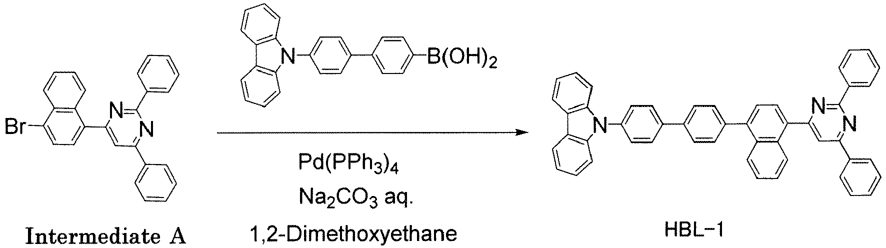

Intermediate A (5.0 g) and [4'-(carbazole-9-yl)-4-biphenylyl] boronic acid (4.5 g) were added to 1,2-dimethoxyethane (80 mL), and argon gas was passed through the solution for 5 minutes. Pd (PPh₃)₄ (0.4 g) and a sodium carbonate aqueous solution (2M, 12 mL) were added to the solution, and the mixture was heated under reflux conditions for 7 hours while stirring in an argon atmosphere. The solvent of the reaction solution was distilled off, and the resulting solid was purified by silica gel column chromatography (developing solvent; hexane/toluene) to obtain HBL-1 as a white solid (6.6 g, yield 85%). The result of mass spectrum analysis revealed m/e = 676 with respect to a molecular weight of 675.84, from which the compound was identified as Compound 1 (HBL-1).

### Synthesis of Intermediate B

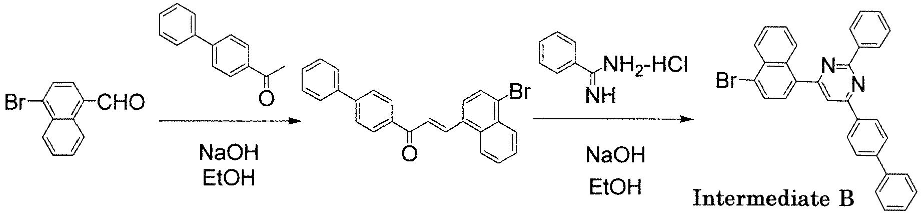

4-bromo-1-naphthaldehyde (8.0 g), 4-acetylbiphenyl (7.0 g) and sodium hydroxide (0.27 g) were added to 600 mL of ethanol and stirred at room temperature for 5 hours. Then, benzamidine hydrochloride (8.0 g) and sodium hydroxide (2.7 g) were added, and the mixture was stirred at 70°C for 5 hours. After completion of the reaction, the precipitate was separated by filtration and purified by silica gel column chromatography (developing solvent; hexane/toluene) to obtain Intermediate B as a white solid (5.0 g, yield 28%).

### Synthesis Example 2: Synthesis of Compound 2

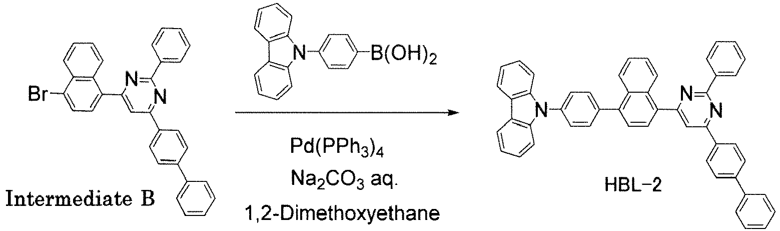

HBL-2 was obtained as a white solid (4.5 g, yield 68%) by the same method as the synthesis method of Synthesis Example 1 using Intermediate B (5.0 g). The result of mass spectrum analysis revealed m/e = 676 with respect to a molecular weight of 675.84, from which the compound was identified as Compound 2 (HBL-2).

### Synthesis of Intermediate C

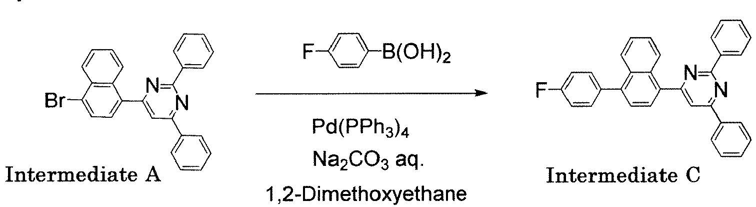

Intermediate C was obtained as a white solid (9.0 g, yield 83%) by the same method as the synthesis method of Synthesis Example 1 using Intermediate A (10.0 g) and 4-fluorophenylboronic acid (3.5 g).

### Synthesis Example 3: Synthesis of Compound 3

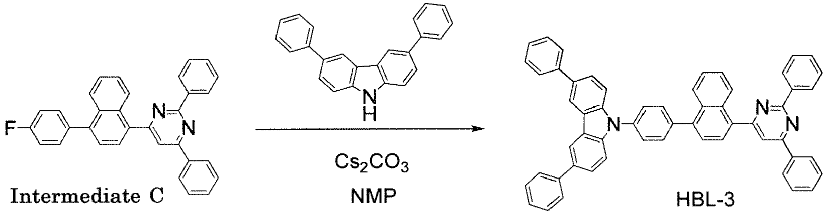

Intermediate C (3.8 g), 3,6-diphenylcarbazole (4.0 g) and cesium carbonate (8.2 g) were added to 30 mL of N-methylpyrrolidone (NMP) and stirred at 160°C for 24 hours. After the reaction solution was cooled to room temperature, 100 mL of methanol and 100 mL of water were added and the mixture was stirred at room temperature for 1 hour. The precipitated solid was collected by filtration and purified by silica gel column chromatography (developing solvent; hexane/toluene) to obtain HBL-3 as a white solid (6.3 g, yield 94%). The result of mass spectrum analysis revealed m/e = 752 with respect to a molecular weight of 751.93, from which the compound was identified as Compound 3 (HBL-3).

### Synthesis of Intermediate D

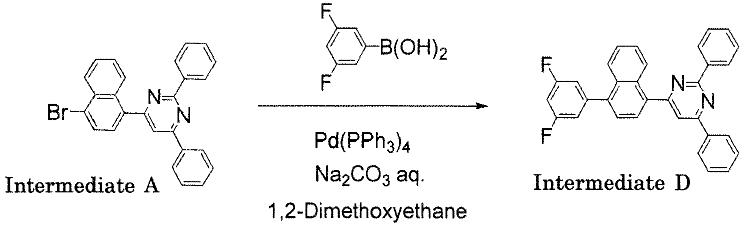

Intermediate D was obtained as a white solid (4.2 g, yield 78%) by the same method as the synthesis method of Synthesis Example 1 using Intermediate A (5.0 g) and 3,5-difluorophenylboronic acid (2.0 g).

### Synthesis Example 4: Synthesis of Compound 4

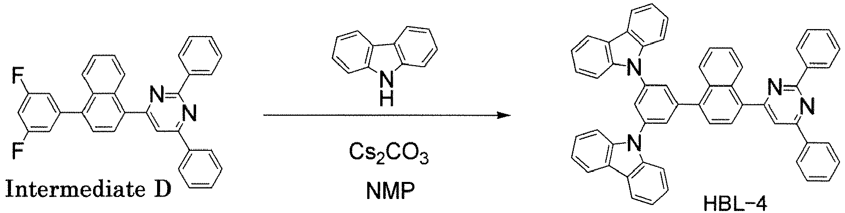

HBL-4 was obtained as a white solid (4.1 g, yield 60%) by the same method as the synthesis method of Synthesis Example 3 using Intermediate D (4.2 g) and carbazole (4.5 g). The result of mass spectrum analysis revealed m/e = 765 with respect to a molecular weight of 764.93, from which the compound was identified as Compound 4 (HBL-4).

### Synthesis Example 5: Synthesis of Compound 5

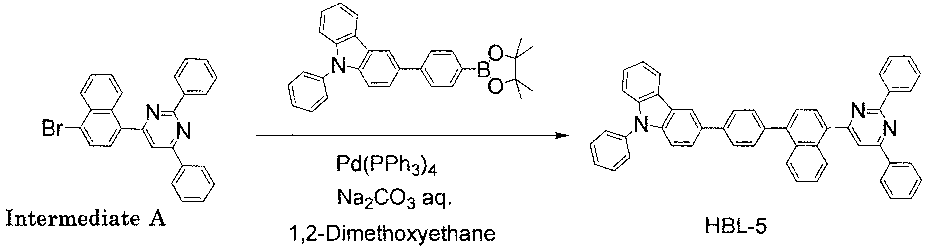

HBL-5 was obtained as a white solid (4.7 g, yield 78%) by the same method as the synthesis method of Synthesis Example 1 using Intermediate A (3.9 g) and 9-phenyl-3-[4-(4,4,5,5-tetramethyl-1,3,2-dioxaborolan-2-yl)phenyl]-9H-carbazole (4.0 g). The result of mass spectrum analysis revealed m/e = 676 with respect to a molecular weight of 675.84, from which the compound was identified as Compound 5 (HBL-5).

### Synthesis Example 6: Synthesis of Compound 6

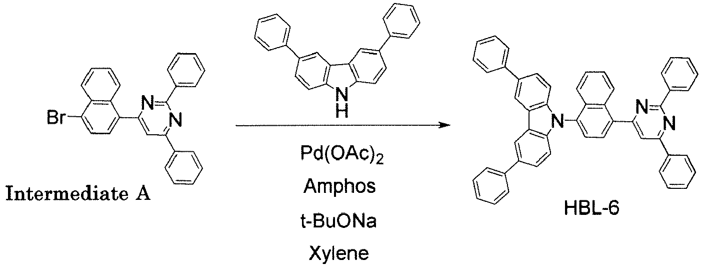

Intermediate A (6.0 g), 3,6-diphenylcarbazole (4.2 g), palladium acetate (0.48 g), Amphos (1.3 g), and t-BuONa (3.8 g) were added to 168 mL of dehydrated xylene and stirred under reflux conditions for 36 hours. After the reaction solution was cooled to room temperature, 100 mL of methanol and 100 mL of water were added and the mixture was stirred at room temperature for 1 hour. The precipitated solid was collected by filtration and purified by silica gel column chromatography (developing solvent; hexane/toluene) to obtain HBL-6 as a white solid (4.8 g, yield 52%).

The result of mass spectrum analysis revealed m/e = 675 with respect to a molecular weight of 675.84, from which the compound was identified as Compound 6 (HBL-6).

### Synthesis Example 7: Synthesis of Compound 7

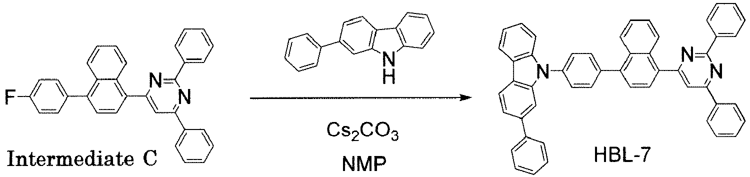

HBL-7 was obtained as a white solid (5.9 g, yield 61%) under the same conditions as those described in Synthesis Example 3 using Intermediate C (5.6 g).

The result of mass spectrum analysis revealed m/e = 675 with respect to a molecular weight of 675.84, from which the compound was identified as Compound 7 (HBL-7).

### Synthesis Example 8: Synthesis of Compound 8

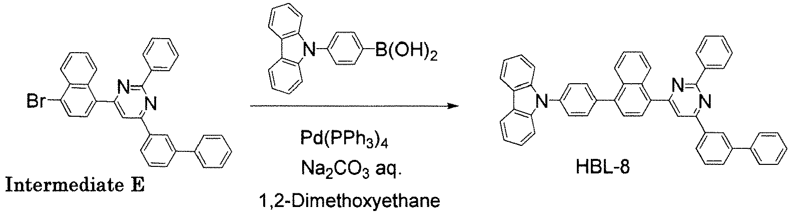

HBL-8 was obtained as a white solid (10.3 g, yield 87%) under the same conditions as those described in Synthesis Example 1 using Intermediate E (8.0 g), which was synthesized according to the method of synthesizing Intermediate B using 3-acetylbiphenyl as a starting material.

The result of mass spectrum analysis revealed m/e = 675 with respect to a molecular weight of 675.84, from which the compound was identified as Compound 8 (HBL-8).

### Synthesis Example 9: Synthesis of Compound 9

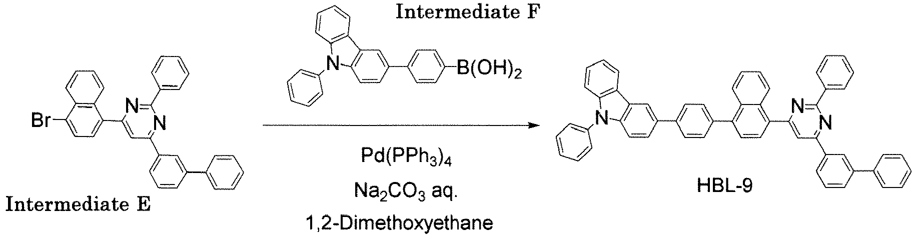

HBL-9 was obtained as a white solid (5.7 g, yield 78%) under the same conditions as those described in Synthesis Example 1 using Intermediate E (5.0 g) and Intermediate F (3.6 g).

The result of mass spectrum analysis revealed m/e = 751 with respect to a molecular weight of 751.93, from which the compound was identified as Compound 9 (HBL-9).

### Synthesis Example 10: Synthesis of Compound 10

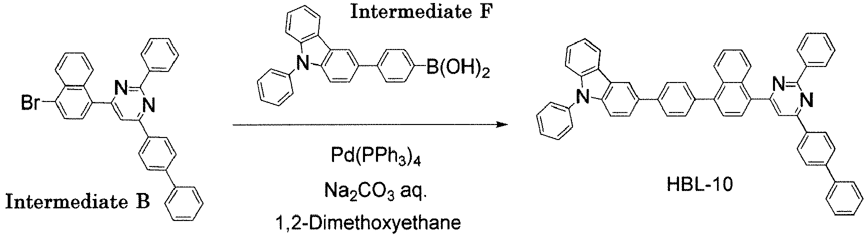

HBL-10 was obtained as a white solid (5.1 g, yield 87%) under the same conditions as those described in Synthesis Example 1 using Intermediate B (4.0 g) and Intermediate F (2.8 g).

The result of mass spectrum analysis revealed m/e = 751 with respect to a molecular weight of 751.93, from which the compound was identified as Compound 10 (HBL-10).

### Synthesis of Intermediate G

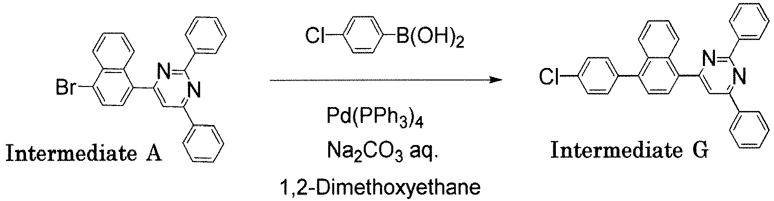

Intermediate G was obtained as a white solid (9.8 g, yield 91%) under the same conditions as those described in Synthesis Example 1 using Intermediate A (10.0 g) and 4-chlorophenylboronic acid (3.6 g).

### Synthesis Example 11: Synthesis of Compound 11

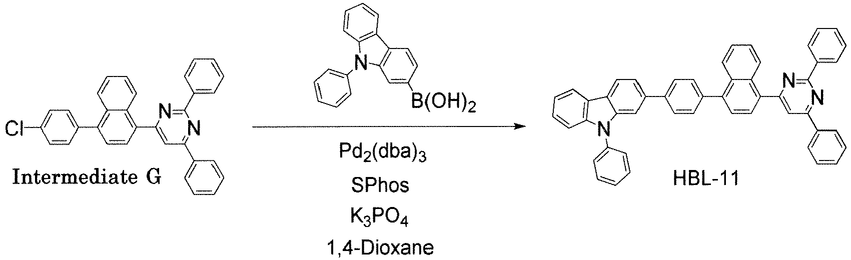

Intermediate G (4.5 g), N-phenylcarbazole-2-boronic acid (4.1 g), and tripotassium phosphate (8.2 g) were added to 1,4-dioxane (100 mL), and argon gas was passed through the solution for 5 minutes. Pd₂(dba)₃ (0.18 g) and SPhos (0.32 g) were added to the solution, and the mixture was heated under reflux conditions for 24 hours while stirring in an argon atmosphere. The solvent of the reaction solution was distilled off, and the resulting solid was purified by silica gel column chromatography (developing solvent; hexane/toluene) to obtain HBL-11 as a white solid (5.8 g, yield 90%).

The result of mass spectrum analysis revealed m/e = 675 with respect to a molecular weight of 675.84, from which the compound was identified as Compound 11 (HBL-11).

### Synthesis Example 12: Synthesis of Compound 12

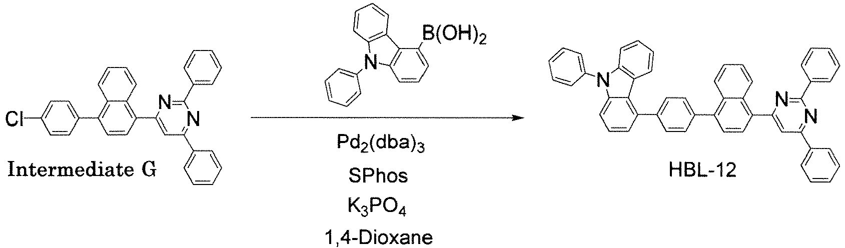

HBL-12 was obtained as a white solid (3.1 g, yield 62%) under the same conditions as those described in Synthesis Example 11 using Intermediate G (3.5 g) and N-phenylcarbazole-4-boronic acid (3.2 g).

The result of mass spectrum analysis revealed m/e = 675 with respect to a molecular weight of 675.84, from which the compound was identified as Compound 12 (HBL-12).

### Synthesis of Intermediate H

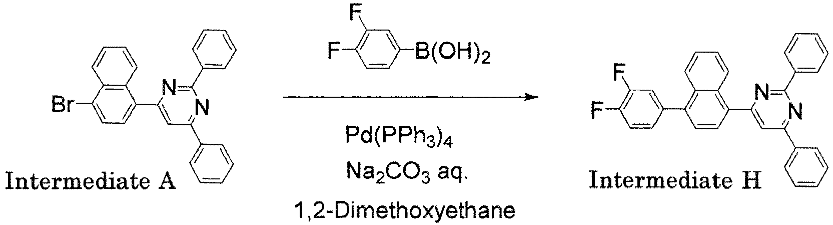

Intermediate H was obtained as a white solid (4.3 g, yield 80%) under the same conditions as those described in Synthesis Example 1 using Intermediate A (5.0 g) and 3,4-difluorophenylboronic acid (2.5 g).

### Synthesis Example 13: Synthesis of Compound 13

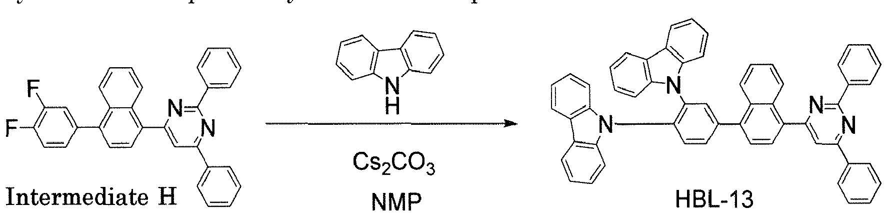

HBL-13 was obtained as a white solid (5.5 g, yield 79%) under the same conditions as those described in Synthesis Example 3 using Intermediate H (4.3 g).

The result of mass spectrum analysis revealed m/e = 764 with respect to a molecular weight of 764.93, from which the compound was identified as Compound 13 (HBL-13).

### Synthesis of Intermediate I

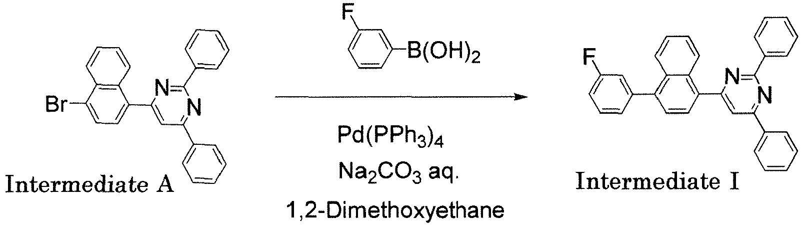

Intermediate I was obtained as a white solid (4.8 g, yield 92%) under the same conditions as those described in Synthesis Example 1 using Intermediate A (5.0 g) and 3-fluorophenylboronic acid (2.2 g).

### Synthesis Example 14: Synthesis of Compound 14

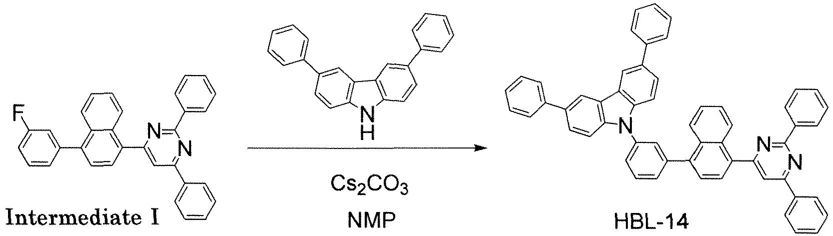

HBL-14 was obtained as a white solid (7.2 g, yield 90%) under the same conditions as those described in Synthesis Example 3 using Intermediate I (4.8 g) and 3,6-diphenylcarbazole (5.0 g).

The result of mass spectrum analysis revealed m/e = 751 with respect to a molecular weight of 751.93, from which the compound was identified as Compound 14 (HBL-14).

### Synthesis Example 15: Synthesis of Compound 15

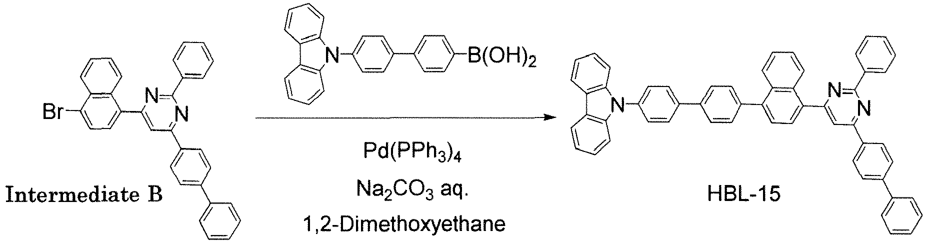

HBL-15 was obtained as a white solid (5.0 g, yield 76%) under the same conditions as those described in Synthesis Example 1 using Intermediate B (4.0 g) and [4'-(carbazole-9-yl) -4-biphenylyl] boronic acid (3.4 g).

The result of mass spectrum analysis revealed m/e = 751 with respect to a molecular weight of 751.93, from which the compound was identified as Compound 15 (HBL-15).

### Synthesis Example 16: Synthesis of Compound 16

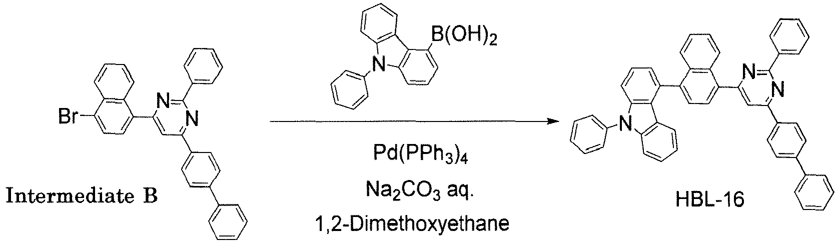

HBL-16 was obtained as a white solid (5.5 g, yield 92%) under the same conditions as those described in Synthesis Example 1 using Intermediate B (4.5 g) and N-phenylcarbazole-4-boronic acid (3.0 g).

The result of mass spectrum analysis revealed m/e = 675 with respect to a molecular weight of 675.84, from which the compound was identified as Compound 16 (HBL-16).

### Synthesis Example 17: Synthesis of Compound 17

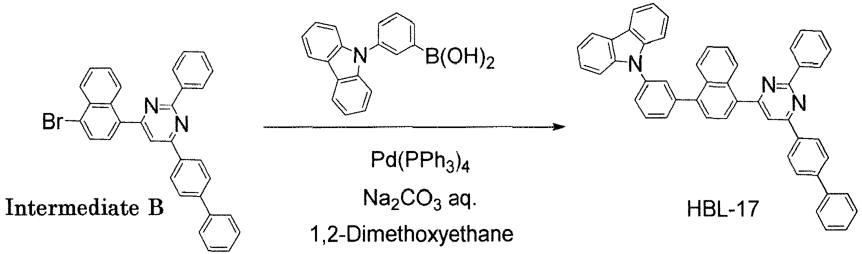

HBL-17 was obtained as a white solid (5.6 g, yield 85%) under the same conditions as those described in Synthesis Example 1 using Intermediate B (5.0 g) and 3-(N-carbazole)phenylboronic acid (3.4 g).

The result of mass spectrum analysis revealed m/e = 675 with respect to a molecular weight of 675.84, from which the compound was identified as Compound 17 (HBL-17).

### Synthesis of Intermediate J

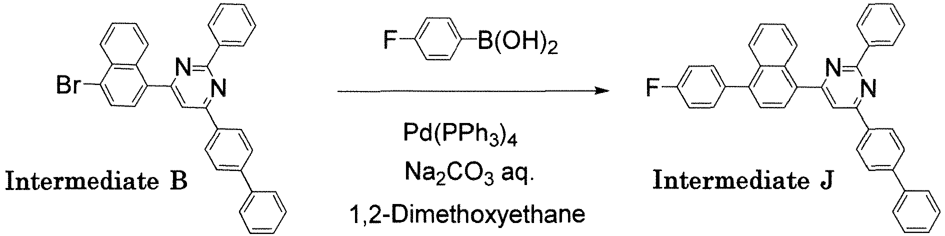

Intermediate J was obtained as a white solid (4.5 g, yield 87%) under the same conditions as those described in Synthesis Example 1 using Intermediate B (5.0 g) and 4-fluorophenylboronic acid (1.6 g).

### Synthesis Example 18: Synthesis of Compound 18

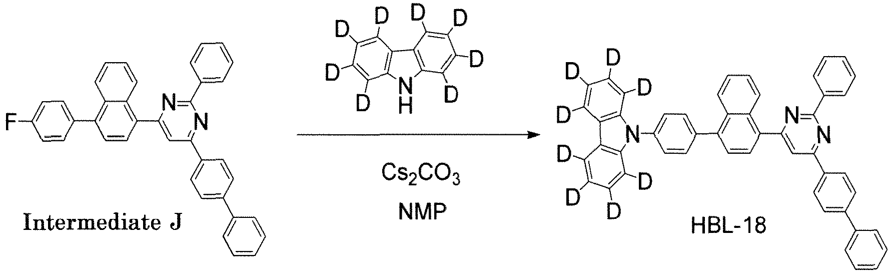

HBL-18 was obtained as a white solid (3.8 g, yield 65%) under the same conditions as those described in Synthesis Example 3 using Intermediate J (4.5 g) and carbazole-ds (2.2 g).

The result of mass spectrum analysis revealed m/e = 683 with respect to a molecular weight of 683.88, from which the compound was identified as Compound 18 (HBL-18).

### Synthesis of Intermediate K

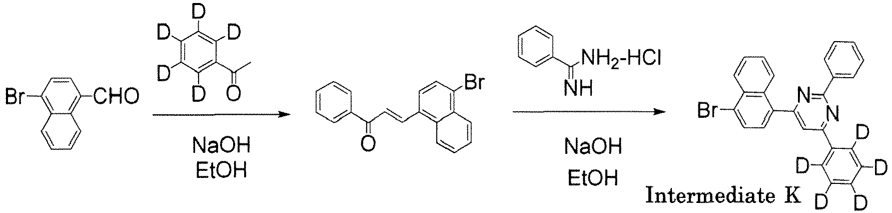

Intermediate K was obtained as a white solid (5.1 g, yield 30%) according to the method of synthesizing Intermediate A using acetophenone (phenyl-d5) (4.8 g) instead of acetophenone.

### Synthesis Example 19: Synthesis of Compound 19

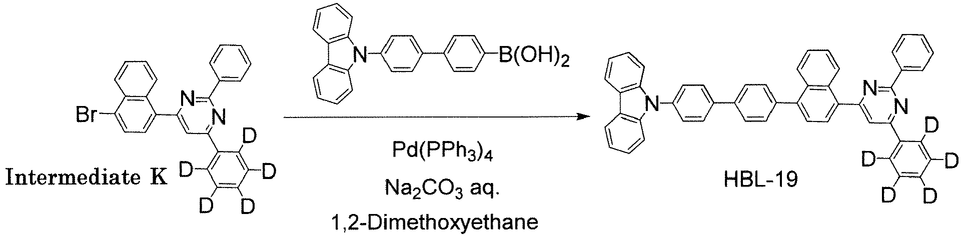

HBL-19 was obtained as a white solid (2.0 g, yield 43%) under the same conditions as those described in Synthesis Example 1 using Intermediate K (3.0 g) and [4'-(carbazole-9-yl) -4-biphenylyl] boronic acid (2.5 g).

The result of mass spectrum analysis revealed m/e = 680 with respect to a molecular weight of 680.87, from which the compound was identified as Compound 19 (HBL-19).

In addition, the compound of the present invention can also be synthesized via the following synthetic intermediates according to the conditions described in the synthesis examples respectively.

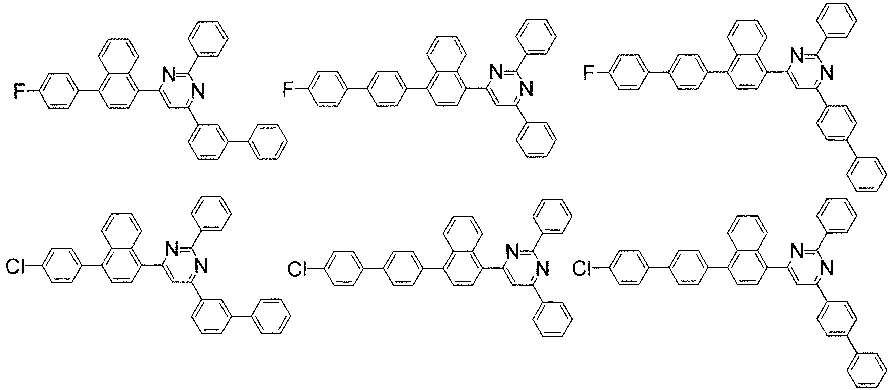

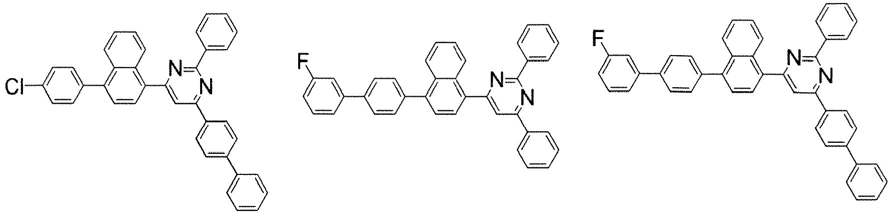

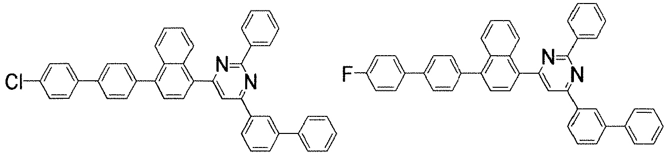

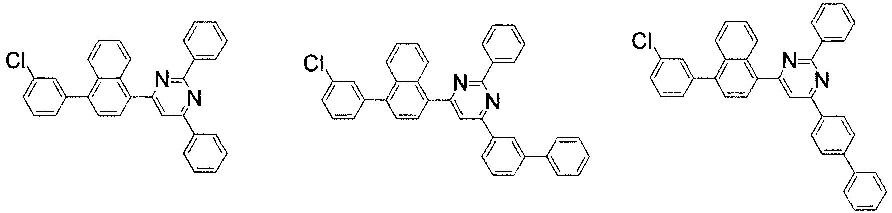

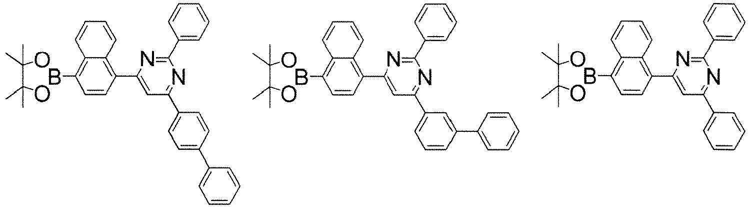

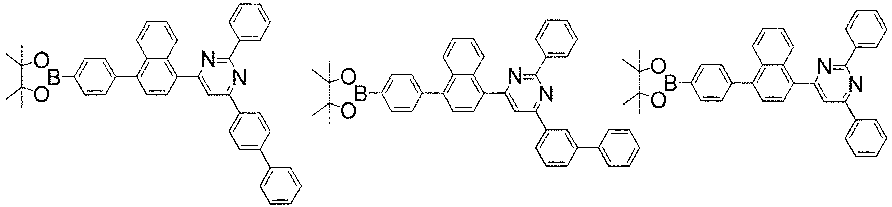

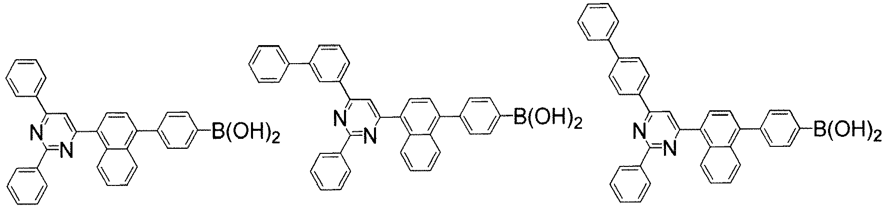

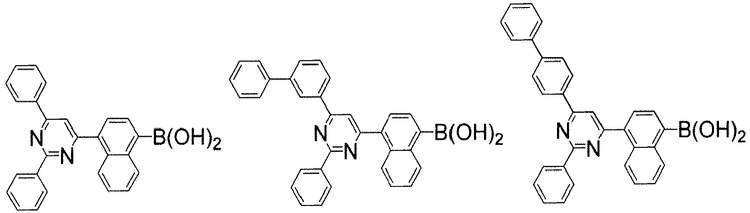

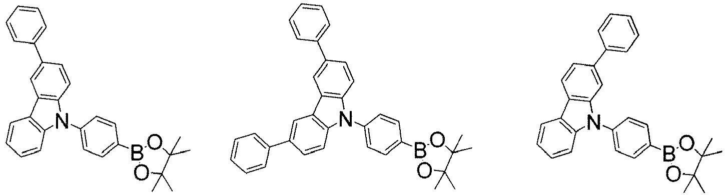

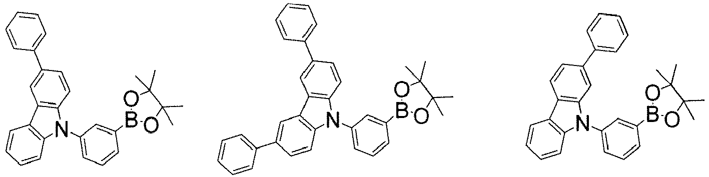

### Reference Signs List

1, 11: Organic EL device
2: Substrate
3: Anode
4: Cathode
5: Light emitting layer
6: Hole transporting zone (hole injecting layer, hole transporting layer, etc.)
7: Electron transporting zone (electron injecting layer, electron transporting layer, etc.)
7a: Hole blocking layer
7b: Electron transporting layer
7c: Electron injecting layer
10, 20: Light emitting unit

## Claims

1. A compound represented by the following formula (1) and having a molecular weight of 650 or more, 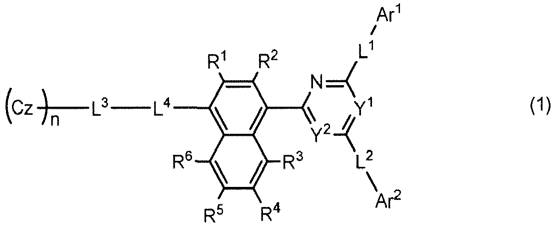 wherein
one of Y¹ and Y² is a nitrogen atom and the other one is CR,
R is selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a fluorine atom, and a cyano group;
Ar¹ and Ar² each are independently selected from a substituted or unsubstituted aryl group having 6 to 50 ring carbon atoms and a substituted or unsubstituted heterocyclic group having 5 to 50 ring atoms;
L¹ and L² each are independently a single bond or a substituted or unsubstituted arylene group having 6 to 50 ring carbon atoms;
R¹ to R⁶ each are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a fluorine atom, and a cyano group, and adjacent two selected from R¹ to R⁶ are not bonded to each other to form a ring;
Cz is represented by the following formula (1-a) or formula (1-b): 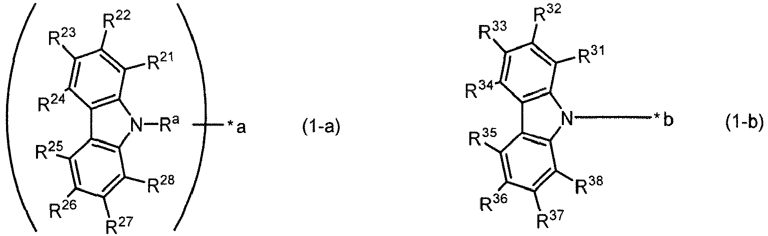 wherein
R²¹ to R²⁸ and R³¹ to R³⁸ each are independently a hydrogen atom or a substituent, and the substituent is selected from a substituted or unsubstituted aryl group having 6 to 50 ring carbon atoms, a substituted or unsubstituted heterocyclic group having 5 to 50 ring atoms but not having a 5-membered nitrogen-containing ring and a 7-membered nitrogen-containing ring, a substituted or unsubstituted alkyl group having 1 to 50 carbon atoms, a substituted or unsubstituted cycloalkyl group having 3 to 50 ring carbon atoms, a fluorine atom, and a cyano group;
provided that adjacent two selected from R²¹ to R²⁸, adjacent two selected from R³¹ to R³⁸, R²⁴ and R²⁵, and R³⁴ and R³⁵ are not bonded to each other to form a ring;
R^{a} is selected from a substituted or unsubstituted aryl group having 6 to 50 ring carbon atoms and a substituted or unsubstituted alkyl group having 1 to 50 carbon atoms;
one selected from R²¹ to R²⁸ is a single bond bonded to L³ via *a; *b represents a position bonded to L³;
n is an integer of 1 to 3, and when n is an integer of 2 or 3, Cz's, which are 2 or 3, are the same or different from each other;
L³ and L⁴ each are independently a single bond or a substituted or unsubstituted arylene group having 6 to 50 ring carbon atoms; provided that when n is an integer of 2 or 3, L⁴ is a substituted or unsubstituted arylene group having 6 to 50 ring carbon atoms.

2. The compound according to claim 1, wherein L³ and L⁴ each are independently a substituted or unsubstituted arylene group having 6 to 50 ring carbon atoms.

3. The compound according to claim 1 or 2, wherein the unsubstituted arylene groups of the substituted or unsubstituted arylene groups having 6 to 50 ring carbon atoms represented by L³ and L⁴ each are independently selected from a phenylene group, a biphenylene group, a naphthylene group, a phenanthrylene group, and an anthrasenylene group.

4. The compound according to any one of claims 1 to 3, wherein at least one selected from R²¹ to R²⁸ that is not a single bond bonded to L³ via *a, or at least one selected from R³¹ to R³⁸ is a substituent.

5. The compound according to any one of claims 1 to 4, wherein at least one of the substituents represented by R²¹ to R²⁸ that is not a single bond bonded to L³ via *a and R³¹ to R³⁸ is selected from a substituted or unsubstituted aryl group having 6 to 50 ring carbon atoms and a substituted or unsubstituted alkyl group having 1 to 50 carbon atoms.

6. The compound according to any one of claims 1 to 5, wherein at least one of the substituents represented by R²¹ to R²⁸ that is not a single bond bonded to L³ via *a and R³¹ to R³⁸ is selected from a methyl group, an ethyl group, an n-propyl group, an isopropyl group, an n-butyl group, an isobutyl group, an s-butyl group, a t-butyl group, a phenyl group, a biphenyl group, a naphthyl group, a phenanthryl group, and a cyano group.

7. The compound according to any one of claims 1 to 6, represented by formula (1-b-1) or (1-b-2): 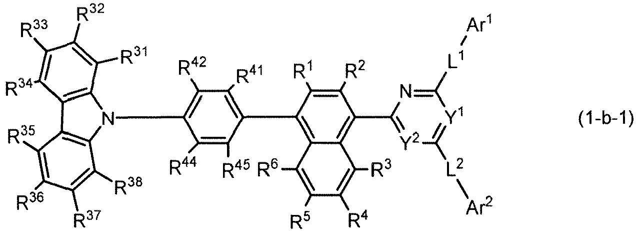 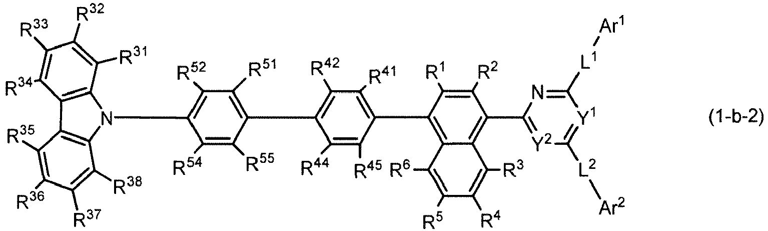 wherein
Y¹, Y², Ar¹, Ar², L¹, L², and R¹ to R⁶ are defined in the formula (1), R³¹ to R38 are defined in the formula (1-b), R⁴¹ to R⁴², R⁴⁴ to R⁴⁵, R⁵¹ to R⁵², and R⁵⁴ to R⁵⁵ each are independently selected from a hydrogen atom, a substituted or unsubstituted aryl group having 6 to 50 ring carbon atoms, and a substituted or unsubstituted alkyl group having 1 to 50 carbon atoms.

8. The compound according to any one of claims 1 to 7, represented by any of formulae (1-b-11) to (1-b-14): 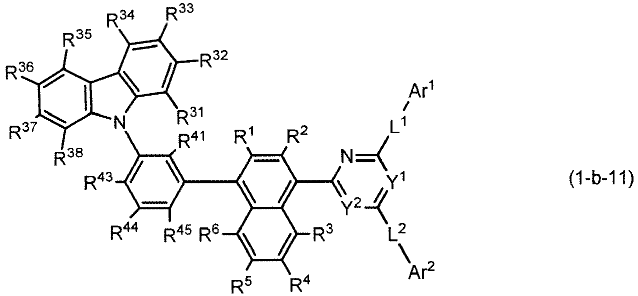 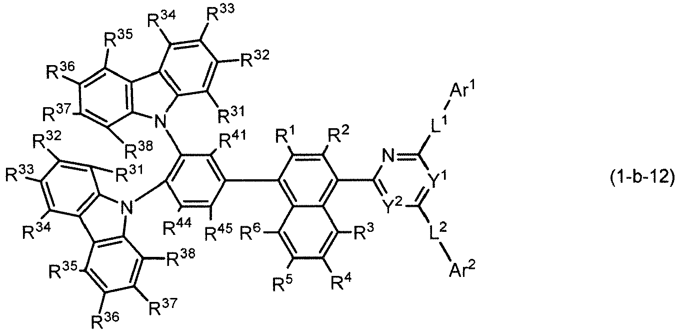 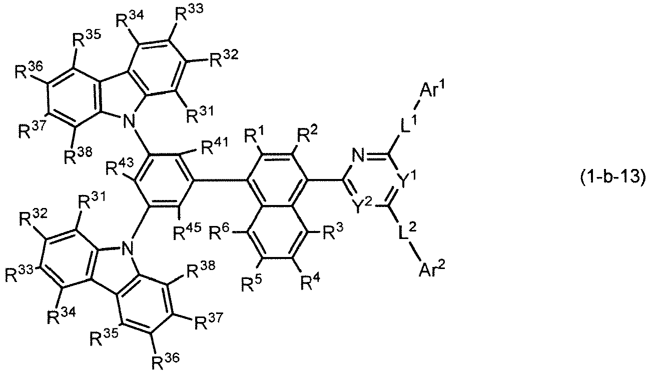 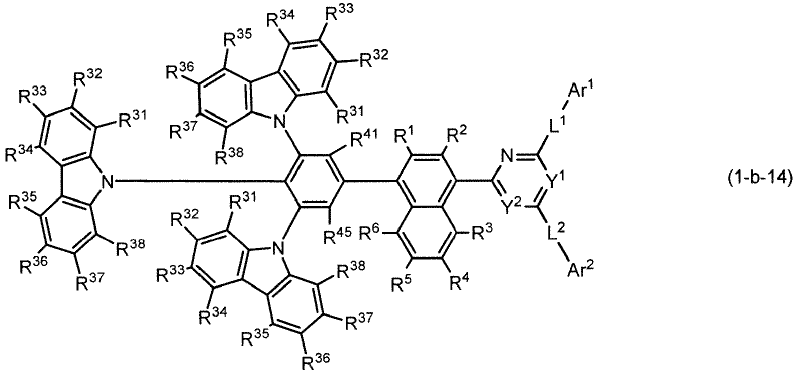 wherein
Y¹, Y², Ar¹, Ar², L¹, L², and R¹ to R⁶ are defined in the formula (1), R³¹ to R³⁸ are defined in the formula (1-b), R⁴¹ and R⁴⁴ to R⁴⁵ are defined in the formula (1-b-1), and R⁴³ is selected from a hydrogen atom, a substituted or unsubstituted aryl group having 6 to 50 ring carbon atoms, and a substituted or unsubstituted alkyl group having 1 to 50 carbon atoms.

9. The compound according to any one of claims 1 to 6, represented by any of formulae (1-a-1) to (1-a-4): 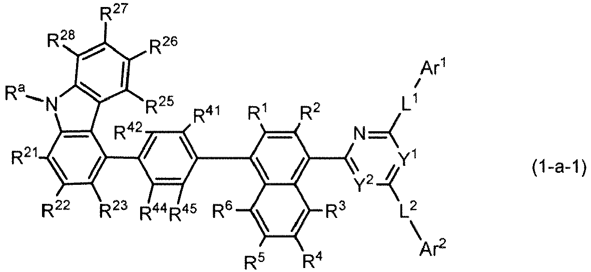 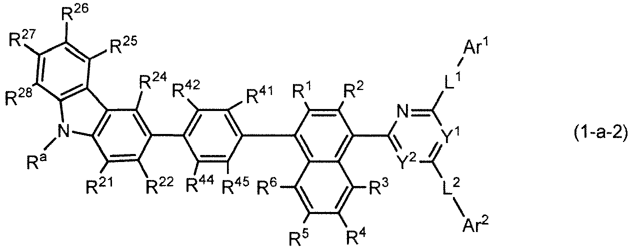 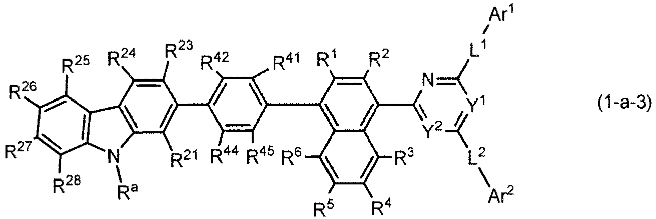 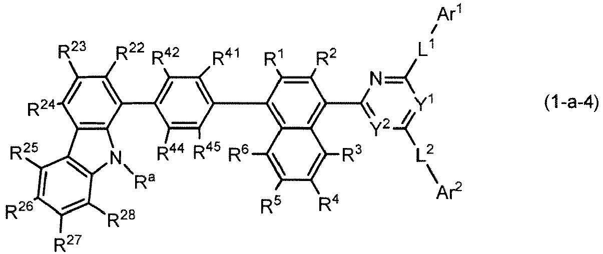 wherein
Y¹, Y², Ar¹, Ar², L¹, L², and R¹ to R⁶ are defined in the formula (1), R²¹ to R²⁸ are defined in the formula (1-a), R⁴¹ to R⁴² and R⁴⁴ to R⁴⁵ are defined in the formula (1-b-1).

10. The compound according to any one of claims 1 and 7 to 9, wherein one of L¹ and L² is a substituted or unsubstituted arylene group having 6 to 50 ring carbon atoms, and the other one is a single bond.

11. The compound according to any one of claims 1 and 7 to 10, wherein L¹ and L² each are independently a substituted or unsubstituted arylene group having 6 to 50 ring carbon atoms.

12. The compound according to any one of claims 1 and 7 to 11, wherein the unsubstituted arylene groups of the substituted or unsubstituted arylene groups having 6 to 50 ring carbon atoms represented by L¹ and L² each are independently selected from a phenylene group, a biphenylene group, a naphthylene group, a phenanthrylene group, a fluorenylene group, and an anthrasenylene group.

13. The compound according to any one of claims 1 and 7 to 9, wherein the unsubstituted aryl groups of the substituted or unsubstituted aryl groups having 6 to 50 ring carbon atoms represented by Ar¹ and Ar² each are independently selected from a phenyl group, a biphenyl group, a naphthyl group, a phenanthryl group, an anthrasenyl group, a chrysenyl group, and a pyrenyl group.

14. The compound according to any one of claims 1 and 7 to 9, wherein the unsubstituted heterocyclic groups of the substituted or unsubstituted heterocyclic groups having 5 to 50 ring atoms represented by Ar¹ and Ar² each are independently selected from a pyridyl group, a dibenzothiophenyl group, a dibenzofuranyl group, an azadibenzofuranyl group, a pyrimidinyl group, a quinolyl group, an isoquinolyl group, a benzothiophenyl group, and a benzofuranyl group.

15. The compound according to claim 1 or 9, wherein the unsubstituted aryl group of the substituted or unsubstituted aryl group having 6 to 50 ring carbon atoms represented by R^{a} is selected from a phenyl group, a biphenyl group, a naphthyl group, and a phenanthryl group.

16. The compound according to any one of claims 1 and 7 to 9, wherein the unsubstituted alkyl groups of the substituted or unsubstituted alkyl groups having 1 to 50 carbon atoms represented by R and R¹ to R⁶ each are independently selected from a methyl group, an ethyl group, an n-propyl group, an isopropyl group, an n-butyl group, an isobutyl group, an s-butyl group, and a t-butyl group.

17. The compound according to any one of claims 1 and 7 to 9, wherein the unsubstituted cycloalkyl groups of the substituted or unsubstituted cycloalkyl groups having 3 to 50 ring carbon atoms represented by R and R¹ to R⁶ each are independently selected from a cyclopropyl group, a cyclobutyl group, a cyclopentyl group, and a cyclohexyl group.

18. The compound according to any one of claims 1 and 7 to 9, wherein the substituted or unsubstituted heterocyclic group having 5 to 50 ring atoms represented by R and R¹ to R⁶ is independently selected from a carbazolyl group, a dibenzofuranyl group, a naphthobenzofuranyl group, a dibenzothiophenyl group, a naphthobenzothiophenyl group, a phenyldibenzofuranyl group, or a phenyldibenzothiophenyl group.

19. The compound according to any one of claims 1 and 7 to 9, wherein the unsubstituted aryl groups of the substituted or unsubstituted aryl groups having 6 to 50 ring carbon atoms represented by R and R¹ to R⁶ each are independently selected from a phenyl group, a p-biphenyl group, an m-biphenyl group, an o-biphenyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-terphenyl-4-yl group, an o-terphenyl-3-yl group, an o-terphenyl-2-yl group, a 1-naphthyl group, a 2-naphthyl group, a fluorenyl group, a 9,9'-spirobifluorenyl group, a 9,9-dimethylfluorenyl group, and a 9,9-diphenylfluorenyl group.

20. The compound according to any one of claims 1 and 7 to 9, wherein R¹ to R⁶ are all hydrogen atoms.

21. The compound according to any one of claims 1 and 7 to 9, wherein R⁴¹ to R⁴⁵ are all hydrogen atoms.

22. The compound according to claim 1 or 7, wherein all of R⁵¹ to R⁵² and R⁵⁴ to R⁵⁵ are hydrogen atoms.

23. The compound according to any one of claims 1, 4 to 6, and 9, wherein all except the substituents selected from R²¹ to R²⁸ that are not single bonds bonded to L³ via *a are hydrogen atoms.

24. The compound according to any one of claims 1 and 4 to 8, wherein all except the substituents selected from R³¹ to R³⁸ are hydrogen atoms.

25. The compound according to any of claims 1 to 24, comprising at least one deuterium atom.

26. A material for organic electroluminescent devices, comprising the compound according to any one of claims 1 to 25.

27. An organic electroluminescent device comprising a cathode, an anode, and organic layers intervening between the cathode and the anode, the organic layers including a light emitting layer, at least one layer of the organic layers containing the compound according to any one of claims 1 to 25.

28. The organic electroluminescent device according to claim 27, wherein the organic layers comprise an electron transporting zone intervening between the cathode and the light emitting layer, and the electron transporting zone comprises the compound.

29. The organic electroluminescent device according to claim 28, wherein the electron transporting zone comprises a first electron transporting layer on a cathode side and a second electron transporting layer on a cathode side, and the first electron transporting layer, the second electron transporting layer, or both of the first electron transporting layer and the second electron transporting layer contain the compound.

30. The organic electroluminescent device according to claim 28 or 29, wherein the electron transporting zone further comprises a hole blocking layer on a cathode side, and the hole blocking layer contains the compound.

31. The organic electroluminescent device according to claim 30, wherein the hole blocking layer is adjacent to the light emitting layer.

32. The organic electroluminescent device according to any one of claims 27 to 31, wherein the light emitting layer comprises a fluorescent dopant material.

33. The organic electroluminescent device according to any one of claims 27 to 31, wherein the light emitting layer comprises a phosphorescent dopant material.

34. An electronic device comprising the organic electroluminescent device according to any one of claims 27 to 33.
